(19) Europäisches Patentamt
European Patent Office
Office européen des brevets

(11) EP 2 473 636 B1

(12) **EUROPEAN PATENT SPECIFICATION**

(45) Date of publication and mention of the grant of the patent:
**26.10.2016 Bulletin 2016/43**

(21) Application number: **10814513.7**

(22) Date of filing: **02.09.2010**

(51) Int Cl.:
***C12Q 1/68*** *(2006.01)*

(86) International application number:
**PCT/US2010/047721**

(87) International publication number:
**WO 2011/028933 (10.03.2011 Gazette 2011/10)**

(54) **TYPE 1 INTERFERON DIAGNOSTIC**

TYP-1-INTERFERON-DIAGNOSEMITTEL

DIAGNOSTIC D'INTERFÉRON DE TYPE 1

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **03.09.2009 US 239630 P**

(43) Date of publication of application:
**11.07.2012 Bulletin 2012/28**

(73) Proprietor: **MedImmune, LLC**
**Gaithersburg, MD 20878 (US)**

(72) Inventors:
- **HIGGS, Brandon**
  **Gaithersburg, MD 20878 (US)**
- **ZHU, Wei**
  **Boyds, MD 20841 (US)**
- **MOREHOUSE, Chris**
  **Middletown, MD 21769 (US)**
- **WHITE, Barbara**
  **Finksburg, MD 21048 (US)**
- **JALLAL, Bahija**
  **Potomac, MD 20854 (US)**
- **YAO, Yihong**
  **Boyds, MD 20854 (US)**

(74) Representative: **Vossius & Partner**
**Patentanwälte Rechtsanwälte mbB**
**Siebertstrasse 3**
**81675 München (DE)**

(56) References cited:
**WO-A2-2008/070137**
**WO-A2-2009/100342**
**US-A1- 2007 092 890**
**US-A1- 2009 221 008**
**US-A1- 2010 143 372**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 473 636 B1

SEQUENCE LISTING

<110> MEDIMMUNE, LLC

<120> INTERFERON DIAGNOSTIC

<130> MED0217.PCT4

<140>
<141>

<150> 61/239,630
<151> 2009-09-03

<160> 35

<170> PatentIn version 3.5

<210> 1
<211> 20
<212> DNA
<213> Artificial Sequence

<220>
<223> Description of Artificial Sequence: Synthetic primer

<400> 1
gctaccacat ccaaggaagg 20

<210> 2
<211> 23
<212> DNA
<213> Artificial Sequence

<220>
<223> Description of Artificial Sequence: Synthetic probe

<400> 2
cgcaaattac ccactcccga ccc 23

<210> 3
<211> 22
<212> DNA
<213> Artificial Sequence

<220>
<223> Description of Artificial Sequence: Synthetic primer

<400> 3
gcctcgaaag agtcctgtat tg 22

<210> 4
<211> 17
<212> DNA
<213> Artificial Sequence

<220>
<223> Description of Artificial Sequence: Synthetic primer

<400> 4
acagagcctc gcctttg 17

<210> 5
<211> 18
<212> DNA
<213> Artificial Sequence

<220>
<223> Description of Artificial Sequence: Synthetic probe

<400> 5
agctggcggc gggtgtgg 18

<210> 6
<211> 17
<212> DNA
<213> Artificial Sequence

<220>
<223> Description of Artificial Sequence: Synthetic primer

<400> 6
ccttgcacat gccggag 17

<210> 7
<211> 19
<212> DNA

<213> Artificial Sequence

<220>
<223> Description of Artificial Sequence: Synthetic primer

<400> 7
acatcgctca gacaccatg 19

<210> 8
<211> 23
<212> DNA
<213> Artificial Sequence

<220>
<223> Description of Artificial Sequence: Synthetic probe

<400> 8
ccgttgactc cgaccttcac ctt 23

<210> 9
<211> 20
<212> DNA
<213> Artificial Sequence

<220>
<223> Description of Artificial Sequence: Synthetic primer

<400> 9
accagagtta aaagcagccc 20

<210> 10
<211> 24
<212> DNA
<213> Artificial Sequence

<220>
<223> Description of Artificial Sequence: Synthetic primer

<400> 10
tgggtgtgaa ccatgagaag tatg 24

<210> 11
<211> 29
<212> DNA
<213> Artificial Sequence

<220>
<223> Description of Artificial Sequence: Synthetic probe

<400> 11
cctcaagatc atcagcaatg cctcctgca 29

<210> 12

<211> 20
<212> DNA
<213> Artificial Sequence

<220>
<223> Description of Artificial Sequence: Synthetic primer

<400> 12
caggggtgct aagcagttgg 20

<210> 13
<211> 20
<212> DNA
<213> Artificial Sequence

<220>
<223> Description of Artificial Sequence: Synthetic primer

<400> 13
ctctcacctc atcagcagtg 20

<210> 14
<211> 20
<212> DNA
<213> Artificial Sequence

<220>
<223> Description of Artificial Sequence: Synthetic probe

<400> 14
ccagaggcca ccctgaccac 20

<210> 15
<211> 21
<212> DNA
<213> Artificial Sequence

<220>
<223> Description of Artificial Sequence: Synthetic primer

<400> 15
tcacaactgt agcaatcctg g 21

<210> 16
<211> 21
<212> DNA
<213> Artificial Sequence

<220>
<223> Description of Artificial Sequence: Synthetic primer

<400> 16
gatgcgaaga ttcactggat g 21

<210> 17
<211> 28
<212> DNA
<213> Artificial Sequence

<220>
<223> Description of Artificial Sequence: Synthetic probe

<400> 17
agttctcaag gcagacagta agctcttc 28

<210> 18
<211> 22
<212> DNA
<213> Artificial Sequence

<220>
<223> Description of Artificial Sequence: Synthetic primer

<400> 18
tgttgaacca gggatccata tg 22

<210> 19
<211> 17
<212> DNA
<213> Artificial Sequence

<220>
<223> Description of Artificial Sequence: Synthetic primer

<400> 19
aagccgtagt ggggtct 17

<210> 20
<211> 23
<212> DNA
<213> Artificial Sequence

<220>
<223> Description of Artificial Sequence: Synthetic probe

<400> 20
tataccgctc ggttatgctg gtg 23

<210> 21
<211> 24
<212> DNA
<213> Artificial Sequence

<220>
<223> Description of Artificial Sequence: Synthetic primer

<400> 21

aacataaatg gcagagattt tcca 24

<210> 22
<211> 25
<212> DNA
<213> Artificial Sequence

<220>
<223> Description of Artificial Sequence: Synthetic primer

<400> 22
aaagactcct accttattct ggatg 25

<210> 23
<211> 27
<212> DNA
<213> Artificial Sequence

<220>
<223> Description of Artificial Sequence: Synthetic probe

<400> 23
ctgaactgta gaaagggacg gaaggac 27

<210> 24
<211> 21
<212> DNA
<213> Artificial Sequence

<220>
<223> Description of Artificial Sequence: Synthetic primer

<400> 24
cttctacacc aacatccagg a 21

<210> 25
<211> 665
<212> DNA
<213> Homo sapiens

<400> 25

gggaacacat ccaagcttaa gacggtgagg tcagcttcac attctcagga actctccttc 60

tttgggtctg gctgaagttg aggatctctt actctctagg ccacggaatt aacccgagca 120

ggcatggagg cctctgctct cacctcatca gcagtgacca gtgtggccaa agtggtcagg 180

gtggcctctg gctctgccgt agttttgccc ctggccagga ttgctacagt tgtgattgga 240

ggagttgtgg ccatggcggc tgtgcccatg gtgctcagtg ccatgggctt cactgcggcg 300

ggaatcgcct cgtcctccat agcagccaag atgatgtccg cggcggccat tgccaatggg 360

ggtggagttg cctcgggcag ccttgtggct actctgcagt cactgggagc aactggactc 420

tccggattga ccaagttcat cctgggctcc attgggtctg ccattgcggc tgtcattgcg 480

aggttctact agctccctgc ccctcgccct gcagagaaga gaaccatgcc aggggagaag 540

gcacccagcc atcctgaccc agcgaggagc caactatccc aaatatacct ggggtgaaat 600

ataccaaatt ctgcatctcc agaggaaaat aagaaataaa gatgaattgt tgcaactctt 660

caaaa 665

<210> 26
<211> 656
<212> DNA
<213> Homo sapiens

<400> 26

gggaacacat ccaagcttaa gacggtgagg tcagcttcac attctcagga actctccttc 60

tttgggtctg gctgaagttg aggatctctt actctctagg ccacggaatt aacccgagca 120

ggcatggagg cctctgctct cacctcatca gcagtgacca gtgtggccaa agtggtcagg 180

gtggcctctg gctctgccgt agttttgccc ctggccagga ttgctacagt tgtgattgga 240

ggagttgtgg ctgtgcccat ggtgctcagt gccatgggct tcactgcggc gggaatcgcc 300

tcgtcctcca tagcagccaa gatgatgtcc gcggcggcca ttgccaatgg gggtggagtt 360

gcctcgggca gccttgtggc tactctgcag tcactgggag caactggact ctccggattg 420

accaagttca tcctgggctc cattgggtct gccattgcgg ctgtcattgc gaggttctac 480

tagctccctg cccctcgccc tgcagagaag agaaccatgc caggggagaa ggcacccagc 540

catcctgacc cagcgaggag ccaactatcc caaatatacc tggggtgaaa tataccaaat 600

tctgcatctc cagaggaaaa taagaaataa agatgaattg ttgcaactct tcaaaa 656

<210> 27
<211> 1742
<212> DNA
<213> Homo sapiens

<400> 27

tctttgaagc ttcaaggctg ctgaataatt tccttctccc atttgtgcc tgcctagcta 60

tccagacaga gcagctaccc tcagctctag ctgatactac agacagtaca acagatcaag 120

aagtatggca gtgacaactc gtttgacatg gttgcacgaa aagatcctgc aaaatcattt 180

tggagggaag cggcttagcc ttctctataa gggtagtgtc catggattcc gtaatggagt 240

tttgcttgac agatgttgta atcaagggcc tactctaaca gtgatttata gtgaagatca 300

tattattgga gcatatgcag aagagagtta ccaggaagga aagtatgctt ccatcatcct 360

ttttgcactt caagatacta aaatttcaga atggaaacta ggactatgta caccagaaac 420

actgttttgt tgtgatgtta caaaatataa ctccccaact aatttccaga tagatggaag 480

aaatagaaaa gtgattatgg acttaaagac aatggaaaat cttggacttg ctcaaaattg 540

tactatctct attcaggatt atgaagtttt tcgatgcgaa gattcactgg atgaaagaaa 600

gataaaaggg gtcattgagc tcaggaagag cttactgtct gccttgagaa cttatgaacc 660

atatggatcc ctggttcaac aaatacgaat tctgctgctg ggtccaattg gagctgggaa 720

gtccagcttt ttcaactcag tgaggtctgt tttccaaggg catgtaacgc atcaggcttt 780

ggtgggcact aatacaactg ggatatctga gaagtatagg acatactcta ttagagacgg 840

gaaagatggc aaatacctgc cgtttattct gtgtgactca ctggggctga gtgagaaaga 900

aggcggcctg tgcagggatg acatattcta tatcttgaac ggtaacattc gtgatagata 960

ccagtttaat cccatggaat caatcaaatt aaatcatcat gactacattg attccccatc 1020

gctgaaggac agaattcatt gtgtggcatt tgtatttgat gccagctcta ttcaatactt 1080

ctcctctcag atgatagtaa agatcaaaag aattcgaagg gagttggtaa acgctggtgt 1140

ggtacatgtg gctttgctca ctcatgtgga tagcatggat ttgattacaa aaggtgacct 1200

tatagaaata gagagatgtg agcctgtgag gtccaagcta gaggaagtcc aaagaaaact 1260

tggatttgct ctttctgaca tctcggtggt tagcaattat tcctctgagt gggagctgga 1320

ccctgtaaag gatgttctaa ttctttctgc tctgagacga atgctatggg ctgcagatga 1380

cttcttagag gatttgcctt ttgagcaaat agggaatcta agggaggaaa ttatcaactg 1440

tgcacaagga aaaaaataga tatgtgaaag gttcacgtaa atttcctcac atcacagaag 1500

attaaaattc agaaaggaga aaacacagac caaagagaag tatctaagac caaagggatg 1560

tgttttatta atgtctagga tgaagaaatg catagaacat tgtagtactt gtaaataact 1620

agaaataaca tgatttagtc ataattgtga aaaataataa taatttttct tggatttatg 1680

ttctgtatct gtgaaaaaat aaatttctta taaaactcgg gtctaaaaaa aaaaaaaaaa 1740

aa 1742

<210> 28
<211> 5889
<212> DNA
<213> Homo sapiens

<400> 28

gctgccagct gagttttttt gctgctttga gtctcagttt tctttctttc ctagagtctc 60

tgaagccaca gatctcttaa gaactttctg tctccaaacc gtggctgctc gataaatcag 120

acagaacagt taatcctcaa tttaagcctg atctaacccc tagaaacaga tatagaacaa 180

tggaagtgac aacaagattg acatggaatg atgaaaatca tctgcgcaag ctgcttggaa 240

atgtttcttt gagtcttctc tataagtcta gtgttcatgg aggtagcatt gaagatatgg 300

ttgaaagatg cagccgtcag ggatgtacta taacaatggc ttacattgat tacaatatga 360

ttgtagcctt tatgcttgga aattatatta atttacatga aagttctaca gagccaaatg 420

attccctatg gttttcactt caaaagaaaa atgacaccac tgaaatagaa actttactct 480

taaatacagc accaaaaatt attgatgagc aactggtgtg tcgtttatcg aaaacggata 540

ttttcattat atgtcgagat aataaaattt atctagataa aatgataaca agaaacttga 600

aactaaggtt ttatggccac cgtcagtatt tggaatgtga agtttttcga gttgaaggaa 660

ttaaggataa cctagacgac ataaagagga taattaaagc cagagagcac agaaataggc 720

ttctagcaga catcagagac tataggccct atgcagactt ggtttcagaa attcgtattc 780

ttttggtggg tccagttggg tctggaaagt ccagtttttt caattcagtc aagtctattt 840

ttcatggcca tgtgactggc caagccgtag tggggtctga tatcaccagc ataaccgagc 900

ggtataggat atattctgtt aaagatggaa aaaatggaaa atctctgcca tttatgttgt 960

gtgacactat ggggctagat ggggcagaag gagcaggact gtgcatggat gacattcccc 1020

acatcttaaa aggttgtatg ccagacagat atcagtttaa ttcccgtaaa ccaattacac 1080

ctgagcattc tacttttatc acctctccat ctctgaagga caggattcac tgtgtggctt 1140

atgtcttaga catcaactct attgacaatc tctactctaa aatgttggca aaagtgaagc 1200

aagttcacaa agaagtatta aactgtggta tagcatatgt ggccttgctt actaaagtgg 1260

atgattgcag tgaggttctt caagacaact ttttaaacat gagtagatct atgacttctc 1320

aaagccgggt catgaatgtc cataaaatgc taggcattcc tatttccaat attttgatgg 1380

ttggaaacta tgcttcagat ttggaactgg accccatgaa ggatattctc atcctctctg 1440

cactgaggca gatgctgcgg gctgcagatg attttttaga agatttgcct cttgaggaaa 1500

ctggtgcaat tgagagagcg ttacagccct gcatttgaga taagttgcct tgattctgac 1560

atttggccca gcctgtactg gtgtgccgca atgagagtca atctctattg acagcctgct 1620

tcagattttg cttttgttcg ttttgccttc tgtccttgga acagtcatat ctcaagttca 1680

aaggccaaaa cctgagaagc ggtgggctaa gataggtcct actgcaaacc acccctccat 1740

atttccgtac catttacaat tcagtttctg tgacatcttt ttaaaccact ggaggaaaaa 1800

tgagatattc tctaatttat tcttctataa cactctatat agagctatgt gagtactaat 1860

cacattgaat aatagttata aaattattgt atagacatct gcttcttaaa cagattgtga 1920

gttctttgag aaacagcgtg gattttactt atctgtgtat tcacagagct tagcacagtg 1980

cctggtaatg agcaagcata cttgccatta cttttccttc ccactctctc caacatcaca 2040

ttcactttaa atttttctgt atatagaaag gaaaactagc ctgggcaaca tgatgaaacc 2100

ccatctccac tgcaaaaaaa aaaaaaaaaa ataagaaaga acaaaacaaa ccccacaaaa 2160

attagctggg tatgatggca cgtgcctgta gtcccagtta ctcaggatga ttgattgagc 2220

cttggaggtg gaggctacag tgagctgaga ttgtgccact gtactctagc cagggagaaa 2280

gagtgagatc ctggctcaaa aaaaccaaat aaaacaaaac aaacaaacga aaaacagaaa 2340

ggaagactga aagagaatga aaagctgggg agaggaaata aaaataaaga aggaagagtg 2400

tttcatttat atctgaatga aaatatgaat gactctaagt aattgaatta attaaaatga 2460

gccaactttt ttttaacaat ttacatttta tttctatggg aaaaaataaa tattcctctt 2520

ctaacaaacc catgcttgat tttcattaat tgaattccaa atcatcctag ccatgtgtcc 2580

ttccatttag gttactgggg caaatcagta agaaagttct tatatttatg ctccaaataa 2640

ttctgaagtc ctcttactag ctgtgaaagc tagtactatt aagaaagaaa acaaaattcc 2700

caaaagatag ctttcacttt ttttttcct taaagacttc ctaattctct tctccaaatt 2760

cttagtcttc ttcaaaataa tatgctttgg ttcaatagtt atccacattc tgacagtcta 2820

atttagtttt aatcagaatt atactcatct tttgggtagt catagatatt aagaaagcaa 2880

gagtttctta tgtccagtta tggaatattt cctaaagcaa ggctgcaggt gaagttgtgc 2940

tcaagtgaat gttcaggaga cacaattcag tggaagaaat taagtcttta aaaaagacct 3000

aggaatagga gaaccatgga aattgaggag gtaggcctac aagtagatat tgggaacaaa 3060

attagagagg caaccagaaa aagttatttt aggctcacca gagttgttct tattgcacag 3120

taacacacca atataccaaa acagcaggta ttgcagtaga gaaagagttt aataattgaa 3180

tggcagaaaa atgaggaagg ttgaggaaac ctcaaatcta cctccctgct gagtctaagt 3240

ttaggatttt taagagaaag gcaggtaagg tgctgaaggt ctggagctgc tgatttgttg 3300

gggtataggg aatgaaatga aacatacaga gatgaaaact ggaagttttt ttttgtttgt 3360

tttgtttttt ttttgttgtt gttttttttt tttttgtttt ttttgctgag tcaattcctt 3420

ggagggggtc ttcagactga ctggtgtcag cagacccatg ggattccaag atctggaaaa 3480

ctttttagat agaaacttga tgtttcttaa cgttacatat attatcttat agaaataact 3540

aagggaagtt agtgccttgt gaccacatct atgtgacttt taggcagtaa gaaactataa 3600

ggaaaggagc taacagtcat gctgtaagta gctacaggga attggcttaa agggcaagtt 3660

ggttagtact tagctgtgtt tttattcaaa gtctacattt tatgtagtgg ttaatgtttg 3720

ctgttcatta ggatggtttc acagttacca tacaaatgta gaagcaacag gtccaaaaag 3780

tagggcatga ttttctccat gtaatccagg gagaaaacaa gccatgacca ttgttggttg 3840

ggagactgaa ggtgattgaa ggttcaccat catcctcacc aacttttggg ccataattca 3900

cccaaccctt tggtggagcc tgaaaaaaat ctgggcagaa tgtaggactt ctttattttg 3960

tttaaagggg taacacagag tgcccttatg aaggagttgg agatcctgca aggaagagaa 4020

ggagtgaagg agagatcaag agagagaaac aatgaggaac atttcatttg acccaacatc 4080

ctttaggagc ataaatgttg acactaagtt atccctttg tgctaaaatg gacagtattg 4140

gcaaaatgat accacaactt cttattctct ggctctatat tgctttggaa acacttaaac 4200

atcaaatgga gttaaataca tatttgaaat ttaggttagg aaatattggt gaggaggcct 4260

caaaaagggg gaaacatctt ttgtctggga ggatattttc cattttgtgg atttccctga 4320

tctttttcta ccaccctgag gggtggtggg aattatcatt ttgctacatt ttagaggtca 4380

tccaggattt ttgaaacttt acattcttta cggttaagca agatgtacag ctcagtcaaa 4440

gacactaaat tcttcttaga aaaatagtgc taaggagtat agcagatgac ctatatgtgt 4500

gttggctggg agaatatcat cttaaagtga gagtgatgtt gtggagacag ttgaaatgtc 4560

aatgctagag cctctgtggt gtgaatgggc acgttaggtt gttgcattag aaagtgactg 4620

tttctgacag aaatttgtag ctttgtgcaa actcacccac catctacctc aataaaatat 4680

agagaaaaga aaaatagagc agtttgagtt ctatgaggta tgcaggccca gagagacata 4740

agtatgttcc tttagtcttg cttcctgtgt gccacactgc ccctccacaa ccatagctgg 4800

gggcaattgt ttaaagtcat tttgttcccg actagctgcc ttgcacatta tcttcatttt 4860

cctggaattt gatacagaga gcaatttata gccaattgat agcttatgct gtttcaatgt 4920

aaattcgtgg taaataactt aggaactgcc tcttcttttt ctttgaaaac ctacttataa 4980

ctgttgctaa taagaatgtg tattgttcag gacaacttgt ctccatacag ttgggttgta 5040

accctcatgc ttggcccaaa taaactctct acttatatca gtttttccta cacttcttcc 5100

ttttaggtca acaataccaa gaggggttac tgtgctgggt aatgtgtaaa cttgtgtctt 5160

gtttagaaag ataaatttaa agactatcac attgcttttt cataaaacaa gacaggtcta 5220

caattaattt attttgacgc aaattgatag gggggccaag taagccccat atgcttaatg 5280

atcagctgat gaataatcat ctcctagcaa cataactcaa tctaatgcta aggtacccac 5340

aagatggcaa ggctgatcaa agtcgtcatg gaatcctgca accaaaagcc atgggaattt 5400

ggaagccctc aaatcccatt cctaatctga tgagtctatg gaccaatttg tggaggacag 5460

tagattaaat agatctgatt tttgccatca atgtaaggag gataaaaact tgcataccaa 5520

ttgtacaccc ttgcaaaatc tttctctgat gttggagaaa atgggccagt gagatcatgg 5580

atatagaagt acagtcaatg ttcagctgta ccctcccaca atcccacttc cttcctcaac 5640

acaattcaaa caaatagact cagactgttt caggctccag gacaggaagt gcagtgtagg 5700

caaaattgca aaaattgagg gcacaggggt ggaggtgggg gggttgaata acaagctgtg 5760

ctaaataatt acgtgtaaat atattttttc atttttaaaa attgatttct tttgcacatt 5820

ccatgacaat atatgtcaca tttttaaaat aaatgcaaag aagcatacat ccaaaaaaaa 5880

aaaaaaaaa 5889

<210> 29
<211> 836
<212> DNA
<213> Homo sapiens

<400> 29

ccagccttca gccggagaac cgtttactcg ctgctgtgcc catctatcag caggctccgg 60

gctgaagatt gcttctcttc tctcctccaa ggtctagtga cggagcccgc gcgcggcgcc 120

accatgcggc agaaggcggt atcgcttttc ttgtgctacc tgctgctctt cacttgcagt 180

ggggtggagg caggtaagaa aaagtgctcg gagagctcgg acagcggctc cgggttctgg 240

aaggccctga ccttcatggc cgtcggagga ggactcgcag tcgccgggct gcccgcgctg 300

ggcttcaccg gcgccggcat cgcggccaac tcggtggctg cctcgctgat gagctggtct 360

gcgatcctga atgggggcgg cgtgcccgcc ggggggctag tggccacgct gcagagcctc 420

ggggctggtg gcagcagcgt cgtcataggt aatattggtg ccctgatggg ctacgccacc 480

cacaagtatc tcgatagtga ggaggatgag gagtagccag cagctcccag aacctcttct 540

tccttcttgg cctaactctt ccagttagga tctagaactt tgcctttttt ttttttttt 600

tttttttgag atgggttctc actatattgt ccaggctaga gtgcagtggc tattcacaga 660

tgcgaacata gtacactgca gcctccaact cctagcctca agtgatcctc ctgtctcaac 720

ctcccaagta ggattacaag catgcgccga cgatgcccag aatccagaac tttgtctatc 780

actctcccca acaacctaga tgtgaaaaca gaataaactt cacccagaaa acactt 836

<210> 30

<211> 848
<212> DNA
<213> Homo sapiens

<400> 30

ccagccttca gccggagaac cgtttactcg ctgctgtgcc catctatcag caggctccgg 60

gctgaagatt gcttctcttc tctcctccaa ggtctagtga cggagcccgc gcgcggcgcc 120

accatgcggc agaaggcggt atcgcttttc ttgtgctacc tgctgctctt cacttgcagt 180

ggggtggagg caggtgagaa tgcgggtaag aaaaagtgct cggagagctc ggacagcggc 240

tccgggttct ggaaggccct gaccttcatg gccgtcggag gaggactcgc agtcgccggg 300

ctgcccgcgc tgggcttcac cggcgccggc atcgcggcca actcggtggc tgcctcgctg 360

atgagctggt ctgcgatcct gaatgggggc ggcgtgcccg ccggggggct agtggccacg 420

ctgcagagcc tcggggctgg tggcagcagc gtcgtcatag gtaatattgg tgccctgatg 480

ggctacgcca cccacaagta tctcgatagt gaggaggatg aggagtagcc agcagctccc 540

agaacctctt cttccttctt ggcctaactc ttccagttag gatctagaac tttgcctttt 600

tttttttttt tttttttttg agatgggttc tcactatatt gtccaggcta gagtgcagtg 660

gctattcaca gatgcgaaca tagtacactg cagcctccaa ctcctagcct caagtgatcc 720

tcctgtctca acctcccaag taggattaca agcatgcgcc gacgatgccc agaatccaga 780

actttgtcta tcactctccc caacaaccta gatgtgaaaa cagaataaac ttcacccaga 840

aaacactt 848

<210> 31
<211> 860
<212> DNA
<213> Homo sapiens

<400> 31

ccagccttca gccggagaac cgtttactcg ctgctgtgcc catctatcag caggctccgg 60

gctgaagatt gcttctcttc tctcctccaa ggtctagtga cggagcccgc gcgcggcgcc 120

accatgcggc agaaggcggt atcgcttttc ttgtgctacc tgctgctctt cacttgcagt 180

ggggtggagg caggtgagaa tgcgggtaag gatgcaggta agaaaaagtg ctcggagagc 240

tcggacagcg gctccgggtt ctggaaggcc ctgaccttca tggccgtcgg aggaggactc 300

gcagtcgccg ggctgcccgc gctgggcttc accggcgccg gcatcgcggc caactcggtg 360

gctgcctcgc tgatgagctg gtctgcgatc ctgaatgggg gcggcgtgcc cgccgggggg 420

ctagtggcca cgctgcagag cctcggggct ggtggcagca gcgtcgtcat aggtaatatt 480

ggtgccctga tgggctacgc cacccacaag tatctcgata gtgaggagga tgaggagtag 540

ccagcagctc ccagaacctc ttcttccttc ttggcctaac tcttccagtt aggatctaga 600

actttgcctt ttttttttt ttttttttt tgagatgggt tctcactata ttgtccaggc 660

tagagtgcag tggctattca cagatgcgaa catagtacac tgcagcctcc aactcctagc 720

ctcaagtgat cctcctgtct caacctccca agtaggatta caagcatgcg ccgacgatgc 780

ccagaatcca gaactttgtc tatcactctc cccaacaacc tagatgtgaa aacagaataa 840

acttcaccca gaaaacactt 860

<210> 32
<211> 3512
<212> DNA
<213> Homo sapiens

<400> 32

aactcagctg agtgttagtc aaagaaggtg tgtcctgctc cccaatgaca ggttgctcag 60

agactgctga tttccatccc tatataaaga gagtccctgg catacagaga ctgctctgct 120

ccaggcatct gccacaatgt gggtgcttac acctgctgct tttgctggga agctcttgag 180

tgtgttcagg caacctctga gctctctgtg gaggagcctg gtcccgctgt tctgctggct 240

gagggcaacc ttctggctgc tagctaccaa gaggagaaag cagcagctgg tcctgagagg 300

gccagatgag accaaagagg aggaagagga ccctcctctg cccaccaccc caaccagcgt 360

caactatcac ttcactcgcc agtgcaacta caaatgcggc ttctgtttcc acacagccaa 420

aacatccttt gtgctgcccc ttgaggaagc aaagagagga ttgcttttgc ttaaggaagc 480

tggtatggag aagatcaact tttcaggtgg agagccattt cttcaagacc ggggagaata 540

cctgggcaag ttggtgaggt tctgcaaagt agagttgcgg ctgcccagcg tgagcatcgt 600

gagcaatgga agcctgatcc gggagaggtg gttccagaat tatggtgagt atttggacat 660

tctcgctatc tcctgtgaca gctttgacga ggaagtcaat gtccttattg gccgtggcca 720

aggaaagaag aaccatgtgg aaaaccttca aaagctgagg aggtggtgta gggattatag 780

agtcgctttc aagataaatt ctgtcattaa tcgtttcaac gtggaagagg acatgacgga 840

acagatcaaa gcactaaacc ctgtccgctg gaaagtgttc cagtgcctct taattgaggg 900

tgagaattgt ggagaagatg ctctaagaga agcagaaaga tttgttattg gtgatgaaga 960

atttgaaaga ttcttggagc gccacaaaga agtgtcctgc ttggtgcctg aatctaacca 1020

gaagatgaaa gactcctacc ttattctgga tgaatatatg cgctttctga actgtagaaa 1080

gggacggaag gacccttcca agtccatcct ggatgttggt gtagaagaag ctataaaatt 1140

cagtggattt gatgaaaaga tgtttctgaa gcgaggagga aaatacatat ggagtaaggc 1200

tgatctgaag ctggattggt agagcggaaa gtggaacgag acttcaacac accagtggga 1260

aaactcctag agtaactgcc attgtctgca atactatccc gttggtattt cccagtggct 1320

gaaaacctga ttttctgctg cacgtggcat ctgattacct gtggtcactg aacacacgaa 1380

taacttggat agcaaatcct gagacaatgg aaaaccatta actttacttc attggcttat 1440

aaccttgttg ttattgaaac agcacttctg tttttgagtt tgttttagct aaaaagaagg 1500

aatacacaca ggaataatga ccccaaaaat gcttagataa ggccccctata cacaggacct 1560

gacatttagc tcaatgatgc gtttgtaaga aataagctct agtgatatct gtgggggcaa 1620

aatttaattt ggatttgatt ttttaaaaca atgtttactg cgatttctat atttccattt 1680

tgaaactatt tcttgttcca ggtttgttca tttgacagag tcagtatttt ttgccaaata 1740

tccagataac cagttttcac atctgagaca ttacaaagta tctgcctcaa ttatttctgc 1800

tggttataat gctttttttt ttttgccttt atgccattgc agtcttgtac tttttactgt 1860

gatgtacaga aatagtcaac agatgtttcc aagaacatat gatatgataa tcctaccaat 1920

tttcaagaag tctctagaaa gagataacac atggaaagac ggtgtggtgc agcccagccc 1980

acggtggctg ttccatgaat gctggctacc tatgtgtgtg gtacctgttg tgtccctttc 2040

tcttcaaaga tcctgagcaa aacaaagata cgctttccat ttgatgatgg agttgacatg 2100

gaggcagtgc ttgcattgct ttgttcgcct atcatctggc cacatgaggc tgtcaagcaa 2160

aagaatagga gtgtagttga gtagctggtt ggccctacat ctctgagaag tgacggcaca 2220

ctgggttggc ataagatatc ctaaaatcac gctggaacct tgggcaagga agaatgtgag 2280

caagagtaga gagagtgcct ggatttcatg tcagtgaagc caagtcacca tatcatattt 2340

ttgaatgaac tctgagtcag ttgaaatagg gtaccatcta ggtcagttta agaagagtca 2400

gctcagagaa agcaagcata agggaaaatg tcacgtaaac tagatcaggg aacaaaatcc 2460

tctccttgtg gaaatatccc atgcagtttg ttgatacaac ttagtatctt attgcctaaa 2520

aaaaaatttc ttatcattgt ttcaaaaaag caaaatcatg gaaaattttt gttgtccagg 2580

caaataaaag gtcattttaa tttagctgca atttcagtgt tcctcactag gtggcattta 2640

aatgtcgcct gatgtcatta agcaccatcc aaaaagtctg cttcataatc tattttcaag 2700

acttggtgat tctgaaagtt ttggtttttg tgactttgtt tctcaggaaa aaaaatattc 2760

ctacttaaat tttaagtcta taattcaatt taaatatgtg tgtgtctcat ccaggatagg 2820

ataggttgtc ttctattttc cattttacct atttactttt tttgtaagaa aagagaaaaa 2880

tgaattctaa agatgttccc catgggtttt gattgtgtct aagctatgat gaccttcata 2940

taatcagcat aaacataaaa caaatttttt acttaacatg agtgcacttt actaatcctc 3000

atggcacagt ggctcacgcc tgtaatccca gcacttggga ggacaatgtg ggtggatcac 3060

gaggtcagga gttcgagaac agcctggcca acatggtgaa accccgtctc cactaaaaat 3120

acaaaaatta gccaggcatg gtggcgtaca cttgtaattc cagctactca agaggctgag 3180

gcaggaggat tgcttgaacc ctgaaggcag aggttacaga gccaagatag cgccactgca 3240

ctccagcctg gatgacagag caagactccg tctcaaaaaa aaaaaaaaaa aaaagcaaga 3300

gagttcaact aagaaaggtc acatatgtga aagcccaagg acactgtttg atatacagca 3360

ggtattcaat cagtgttatt tgaaaccaaa tctgaatttg aagtttgaat cttctgagtt 3420

ggaatgaatt tttttctagc tgagggaaac tgtatttttc tttccccaaa gaggaatgta 3480

atgtaaagtg aaataaaact ataagctatg tt 3512

<210> 33

<211> 526
<212> DNA
<213> Homo sapiens

<400> 33

gaattcggct ttggtgactc tagataacct cgggccgatc gcacgccccc cgtggcggcg 60
acgacccatt cgaacgtctg ccctatcaac tttcgatggt agtcgccgtg cctaccatgg 120
tgaccacggt gacggggaat cagggttcga ttccggagag ggagcctgag aaacggctcc 180
acatccaagg aaggcagcag gcgcgcaaat tacccactcc cgacccgggg aggtagtgac 240
gaaaaataac aatcaggact ctttcgaggc cctgtaattg gaatgagtcc actttaaatc 300
ctttacgagg atccattgga gggcaagtct ggtgccagca gccgcggtaa ttccagctcc 360
aatagcgtat attaaagttc tgcagttaaa aagctcgtag ttggatcttg ggagcgggcg 420
ggcggtccgc cggaggcgag ccaccgcccg tccccgcccc ttgcctctcg gcgccccctc 480
gatgctctta gctgagtgtc aaacagagga ttacccatgt aagctt 526

<210> 34
<211> 551
<212> DNA
<213> Homo sapiens

<400> 34

gaattcaccg ccgagaccgc gtccgccccg cgagcacaga gcctcgcctt tgccgatccg 60
ccgcccgtcc acacccgccg ccagctcacc atggatgatg atatcgccgc gctcgtcgtc 120
gacaacgctc cggcatgtgc aaggccggct tcgcgggcga cgatgccccc cgggccgttt 180
cccctccatc gtggggcgcc ccaggcacca gggcgtgatg gtgggcatgg gtcagaagga 240
ttcctatgtg ggcacgaggc ccagagcaag agaggcatcc tcaccctgaa gtaccccatc 300
gagcaggcat cgtcaccaac tgggacgaca tggagaaaat ctggcaccac accttctaca 360
atgagctgcg tgtggctccg aggagcaccc cgtgctgctg accgaggccc ccctgaaccc 420
caaggccaac cggagaagat gacccagatc atgtttgaga ccttcaacac cccagccatg 480
tacgttgcta tccaggctgt gctatcctgt acgcctctgg ccgtaaacat gaggattacc 540
catgtaagct t 551

<210> 35
<211> 684
<212> DNA
<213> Homo sapiens

<400> 35

gaattcaaat tgagcccgca gcctcccgct tcgctctctg ctcctcctgt tcgacagtca 60

gccgcatctt cttttgcgtc gccagccgag ccacatcgct cagacaccat ggggaaggtg 120

aaggtcgagt caacggattt ggtcgtattg ggcgcctggt caccagggct gcttttaacc 180

tggtaaagtg gatattgttg ccatcaatga ccccttcatt gacctcaact acatggttta 240

catgttccaa tatattccac ccatggcaaa ttccatggca ccgtcaaggc tgagaacggg 300

aagcttgtca tcaatggaaa tcccatcacc atcttccagg agcgagatcc ctccaaaatc 360

aagtggggcg atgctggcgc agtacgtcgt ggagtccact ggcgtcttca ccaccatgga 420

gaaggctggg gctcatttgc agggggggagc caaaagggtc atcatctctg ccccctctgc 480

tgatgccccc atgttcgtca tgggtggacc atgagaagta tgacaacagc ctcaagatca 540

tcagcaatgc ctcctgcacc accaactgct tagcacccct ggccaaggtc atccatgaca 600

actttggtat cgtggaagga ctcatgacca catcatgcca tcactgccac ccagaagaaa 660

catgaggatt acccatgtgg atcc 684

## Description

### SEQUENCE LISTING

The instant application contains a Sequence Listing which has been submitted in ASCII format via EFS-Web. The ASCII copy, created on September 1, 2010, is named MED217T4.txt and is 28,665 bytes in size.

### FIELD OF THE DISCLOSURE

The present disclosure relates to pharmacodynamic (PD) markers inducible by type 1 interferons, such as interferon (IFN) alpha, probes and kits that detect the PD markers, and methods employing the same. The present disclosure further relates to PD markers induced by autoimmune disease. The present disclosure further relates to genes whose expression can be used as a diagnostic for patients suffering from autoimmune diseases, such as SLE, DM, PM, SSc, RA, Sjogrens, and lupus nephritis. The disclosure further relates to genes whose expression can be used for identifying patients suffering from an autoimmune disease who will respond to a therapeutic agent that modulates type 1 interferon activity, such as an anti-interferon alpha antibody.

### BACKGROUND OF THE DISCLOSURE

The present disclosure encompasses PD markers that are induced by IFN$\alpha$. The PD markers can be used in methods of treating patients with a therapeutic agent that a therapeutic agent that modulates type 1 interferon activity, such as an agent that binds to and modulates IFN$\alpha$ activity, methods that identify patients as candidates for a therapeutic agent that modulates type 1 interferon activity, methods of diagnosing a patient as having a disorder associated with increased type 1 interferon or IFN$\alpha$ levels, methods of monitoring disease progression of a patient receiving treatment with a therapeutic agent that modulates type 1 interferon activity, such as a therapeutic agent that binds to and modulates IFN$\alpha$ activity, and methods of identifying a candidate therapeutic for treating IFN$\alpha$-mediated disorders. The present disclosure also encompasses PD markers otherwise involved in autoimmune diseases such as SLE, DM, PM, SSc, RA, Sjogrens, and lupus nephritis.

WO2008070137 discloses the use of a combination of 25 genes including IF127, IF144, IF144L, and RSAD2 for identifying a subject suitable for treatment of lupus with an anti-interferon alpha antibody or an anti-interferon alpha receptor antibody that modulates type 1 interferon activity.

### SUMMARY OF THE DISCLOSURE

The present invention is defined in the appended claims. One embodiment of the disclosure encompasses a method of identifying a patient as a candidate for a therapeutic agent that modulates type 1 interferon activity, such as a therapeutic agent that binds to and modulates IFN$\alpha$ activity and an agent that binds to a receptor of a type I interferon or IFN$\alpha$. Presence or absence of an IFN$\alpha$-inducible PD marker expression profile is detected in a sample from the patient. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

Another embodiment of the disclosure encompasses a method of treating a patient having a type I IFN or IFN$\alpha$-mediated disease or disorder. An agent that modulates type I IFN or IFN$\alpha$ activity is administered to the patient. In one embodiment, the agent binds to and neutralizes IFN or IFN$\alpha$ activity. In another embodiment, the agent binds to a receptor of a type 1 interferon or IFN$\alpha$. The agent neutralizes a type I IFN or IFN$\alpha$-inducible PD marker expression profile of the patient. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

Yet another embodiment of the disclosure encompasses a method of treating an autoimmune disease patient comprising a moderate or strong type I IFN or an IFN$\alpha$ PD marker profile. An agent that modulates type I IFN or IFN$\alpha$ activity is administered to the patient. In one embodiment, the agent binds to and neutralizes IFN or IFN$\alpha$ activity. In another embodiment, the agent binds to a receptor of a type 1 interferon or IFN$\alpha$. The agent neutralizes a type I IFN or IFN$\alpha$-inducible PD marker expression profile of the patient. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

A further embodiment of the disclosure encompasses a method of neutralizing a type I IFN or IFNα-inducible PD marker expression profile in a patient in need thereof.. An agent that modulates type I IFN or IFNα activity is administered to the patient. In one embodiment, the agent binds to and neutralizes IFN or IFNα activity. In another embodiment, the agent binds to a receptor of a type 1 interferon or IFNα. The agent neutralizes a type I IFN or IFNα-inducible PD marker expression profile of the patient. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

Another embodiment of the disclosure encompasses a method of diagnosing a patient as having a disorder associated with increased IFNα levels. Presence or absence of an IFNα-inducible PD marker expression profile is detected in a sample from the patient. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

A further embodiment of the disclosure encompasses a method of monitoring disease progression of a patient receiving treatment with a therapeutic agent that binds to and modulates IFNα activity. A first IFNα-inducible PD marker expression profile is obtained in a first sample from the patient. An agent that modulates type I IFN or IFNα activity is administered to the patient. In one embodiment, the agent binds to and neutralizes IFN or IFNα activity. In another embodiment, the agent binds to a receptor of a type 1 interferon or IFNα. A second IFNα-inducible PD marker expression profile is obtained from a second sample from the patient. The first and the second IFNα-inducible PD marker expression profiles are compared. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

Yet another embodiment of the disclosure encompasses a method of identifying a candidate therapeutic for treating IFNα-mediated disorders. Cells comprising an IFNα-inducible PD marker expression profile are contacted with an agent. Presence or absence of a change in the IFNα-induced PD marker expression profile of the cells is detected. The PD marker expression profile comprises up-regulation of expression or activity of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

A further embodiment of the disclosure encompasses a set of oligonucleotides. The set of oligonucleotides may comprise oligonucleotides that specifically detect expression of a set of genes. In a specific embodiment, the set of genes may be: (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

Yet a further embodiment of the disclosure encompasses oligonucleotides that specifically detect 18S, ACTB, and GAPDH.

Another embodiment of the disclosure is a kit comprising oligonucleotides for specifically detecting at least four of IFI27, IFI44, IFI44L, IFI6 and RSAD2, and 18S, ACTB, and GAPDH, as well as reagents suitable for the detection.

Another embodiment of the disclosure encompasses a method of detecting IFN activity in a sample. Cells comprising a polynucleotide sequence comprising a reporter gene under the control of an IFN-stimulated response element are incubated with a sample. Expression of the reporter gene is detected.

Yet a further embodiment of the disclosure encompasses a method of monitoring autoimmune disorder progression or regression of a patient. A first PD marker expression profile is obtained from a first sample from the patient. A second PD marker expression profile is obtained from a second sample from the patient. The first and the second PD marker expression profiles are compared. A variance in the first and the second PD marker expression profiles indicates disease progression or regression.

## BRIEF DESCRIPTION OF THE FIGURES

Figure 1. Receiver operator characteristic (ROC) curve for the four gene (IFI27, IFI44, IFI44L, and RSAD2) signature used for a diagnostic. This plot demonstrates the trade off between sensitivity (true positive rate) and 1-specificity (false positive rate) for treated SLE patients using the primary clinical endpoint at days 182 and 196.

Figures 2A and B. Clear boundary between diagnostic test positive and negative patients in SLE using the four gene (IFI27, IFI44, IFI44L, and RSAD2) signature based test. (A) Average $log_2$ fold change - using Applied Biosystem's qRT-PCR TaqMan low density array (TLDA) platform for test negative and test positive patients is shown. (B) Average $log_2$ fold change density plot of the gene signature values for drug-treated SLE patients.

Figures 3A and B. Time adjusted area under the curve minus baseline SLEDAI score in four gene (IFI27, IFI44, IFI44L, and RSAD2) signature (A) positive or (B) negative SLE patients in placebo or 0.3/1/3/10 mg/kg of MEDI-545 cohorts. All data are from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening.

Figures 4A and B. SELDAI responses, reduction (improvement) ≥ 4 points. A. Diagnostic positive. B. Diagnostic negative. All data are from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening.

Figures 5A and B. SLEDAI responses, reduction (improvement) > 4 points, in Dx+ subjects with > 50% reduction vs. < 50% reduction in Dx post baseline. All data are from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening.

Figures 6A and B. Composite Responses. A) Composite response for patients positive for four gene signature. B) Composite response for patients negative for four gene signature. All data are from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening.

Figures 7A and B. SLEDAI area under the curve minus baseline. A) subjects positive for four gene signature. B) subjects negative for four gene signature. All data are from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening.

Figures 8A and B. SLEDAI change from baseline. A) subjects positive for four gene signature. B) subjects negative for four gene signature. All data are from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening.

Figures 9 A-C. Subjects with > 50% inhibition of Type I IFN Signature have higher SLEDAI responses (reduction > 4 points). A) 1 mg/kg IV q2wks B) 3 mg/kg IV q2wks C) 10 mg/kg IV q2wks

Figures 10 A-C. Subjects with < 50% inhibition of Type I IFN Signature have lower SLEDAI responses (reduction > 4 points). A) 1 mg/kg IV q2wks B) 3 mg/kg IV q2wks C) 10 mg/kg IV q2wks.

Figures 11A and B. Five gene signature in various diseases. A) Gene signature in whole blood from normal, SLE, SM, PM, RA, and SSc. B) Gene signature in normal skin, SLE skin, SSc skin, normal muscle, DM muscle, PM muscle, normal synovium tissue.

Figures 12A and B. Four gene signature in various diseases. A) Gene signature in whole blood from normal, SLE, SM, PM, RA, and SSc. B) Gene signature in normal skin, SLE skin, SSc skin, normal muscle, DM muscle, PM muscle, normal synovium tissue.

## DETAILED DESCRIPTION

The disclosure encompasses methods of identifying, diagnosing, treating, and monitoring disease progression in patients. Patients include any animal having a type I IFN or an IFNα-inducible disease, disorder, or condition. Patients include any animal having an autoimmune disease or disorder or condition. Autoimmune diseases/disorders/conditions include systemic lupus erythematosus (SLE), insulin dependent diabetes mellitus, inflammatory bowel disease (including Crohn's disease, ulcerative colitis, and Celiac's disease), multiple sclerosis, psoriasis, autoimmune thyroiditis, scleroderma, rheumatoid arthritis, glomerulonephritis, idiopathic inflammatory myositis, Sjogren's syndrome, vasculitis, inclusion body myositis (IBM), dermatomyositis (DM), polymyositis (PM), sarcoidosis, scleroderma and lupus nephritis. The patient may have the disease, disorder, or condition as a result of experimental research, e.g., it may be an experimental model developed for the disease, disorder, or condition. Alternatively, the patient may have the disease, disorder, or condition in the absence of experimental manipulation. Patients include humans, mice, rats, horses, pigs, cats, dogs, and any animal used for research.

The patient may comprise a type I IFN or IFNα-inducible PD marker expression profile. The type I IFN or IFNα-inducible PD marker expression profile may be a strong profile, a moderate profile, or a weak profile. The type I IFN or IFNα-inducible PD marker expression profile can readily be designated as strong, moderate, or weak by determining the fold dysregulation of the type I IFN or IFNα-inducible PD marker expression profile of the patient, (*e.g.*, the fold increase in expression of upregulated type I IFN or IFNα-inducible PD markers in the patient), relative to a control sample(s) or control patient(s) and comparing the patient's fold dysregulation to that of other patients having a type I IFN or IFNα-inducible PD marker expression profile. Fold dysregulation can be calculated by well known methods in the art as can the comparing. See, *e.g.*, Example 8 of International Application No. PCT/US2007/024947. In one embodiment, the fold dysregulation is calculated as fold change in mRNA expression levels. Strong, moderate, or weak profiles may likewise be generated for genes that are not specifically type I IFN or IFNα-inducible.

Up or down regulation of a group of genes comprised by a type I IFN or IFNα-inducible PD marker expression profile can be calculated by well known methods in the art. In one embodiment, up or down regulation is calculated as average fold change in the mRNA expression levels of the group of at least four genes chosen from IFI27, IFI44, IFI44L, IFI6 and RSAD2. In another embodiment, the up or down regulation is calculated as the difference between the mean Ct (cycle threshold) for at least four target genes (IFI27, IFI44, IFI44L, IFI6 and RSAD2) and the mean Ct of three control genes.

### I. Type I IFN or IFNα-inducible PD marker expression profile

### A. Diagnostic Genes

The group of genes included in the type I IFN or IFNα-inducible PD marker expression profile of the patient are (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

In a specific embodiment, the group of genes included in the type I IFN or IFNα-inducible PD marker expression profile of the patient comprises IFI27, IFI44, IFI44L, IFI6 and RSAD2. In another specific embodiment, the group of genes included in the type I IFN or IFNα-inducible PD marker expression profile of the patient consists of IFI27, IFI44, IFI44L, IFI6 and RSAD2. In a further specific embodiment, the group of genes included in the type I IFN or IFNα-inducible PD marker expression profile of the patient comprises IFI27, IFI44, IFI44L, and RSAD2. In another specific embodiment, the group of genes included in the type I IFN or IFNα-inducible PD marker expression profile of the patient consists of IFI27, IFI44, IFI44L, and RSAD2.

The IFNα-inducible PD markers in an expression profile may include (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

The IFNα-inducible PD markers in an expression profile may consist of (a) IFI27, IFI44, IFI44L, IFI6 and RSAD2; or (b) IFI44, IFI44L, IFI6 and RSAD2; or (c) IFI27, IFI44L, IFI6 and RSAD2; or (d) IFI27, IFI44, IFI6 and RSAD2; or (e) IFI27, IFI44, IFI44L, and RSAD2; or (f) IFI27, IFI44, IFI44L, and IFI6.

### B. Serum Proteins

The IFNα-inducible PD markers in an expression profile may include alterations in any one or more of serum protein levels of adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin.

The IFNα-inducible PD markers in an expression profile may include alterations in any one or more of serum protein levels of adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, orvWF.

The IFNα-inducible PD markers in an expression profile may include alterations in any one or more of serum protein levels of BDNK, complement 3, CD40 ligand, EGF, ENA-78, EN-RAGE, IGF-1, MDC, myeloperoxidase, RANTES, or thrombopoietin.

IFNα-inducible PD marker expression profiles may include up-regulated expression or activity of genes in cells exposed to elevated IFNα levels relative to baseline. Up-regulated expression or activity of genes includes an increase in expression of mRNA from a gene, an increase in expression of a protein encoded by a gene, or an increase in activity of a protein encoded by a gene. The expression or activity of the genes may be up-regulated as a direct or indirect response to IFNα.

**C. Interferon subtypes**

The patient comprising the type I IFN or IFNα-inducible PD marker expression profile may further comprise upregulation of expression of any number of IFNα or type-I IFN subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. The patient may comprise upregulation of expression of IFN subtypes IFNα1, IFNα2, IFNα8, and IFNα14.

Alternatively, a patient treated in the methods encompassed by the disclosure may simply be one identified as comprising a gene expression profile with upregulation of expression of any number of IFNα or type-I IFN subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. These subtypes may include IFNα1, IFNα2, IFNα8, and IFNα14.

**D. IFN-α receptors**

The patient comprising the type I IFN or IFNα-inducible PD marker expression profile may further comprise upregulation of expression of IFNα receptors, either IFNAR1 or IFNAR2, or both, or TNFα, or IFNγ, or IFNγ receptors (either IFNGR1, IFNGR2, or both IFNGR1 and IFNGR2). The patient may simply be identified as one who comprises upregulation of expression of IFNα receptors, either IFNAR1 or IFNAR2, or both, or TNFα, or IFNγ, or IFNγ receptors (either IFNGR1, IFNGR2, or both IFNGR1 and IFNGR2).

**II. Upregulation**

The upregulation or downregulation of the type I IFN or IFNα-inducible PD markers in the patient's expression profile may be by any degree relative to that of a sample from a control (which may be from a sample that is not disease tissue of the patient (e.g., non-lesional skin of a psoriasis patient) or from a healthy person not afflicted with the disease or disorder) or may be relative to that of genes from the patient whose expression is not changed by the disease (so called "house keeping" genes.)

The degree upregulation or downregulation may be at least 10%, at least 15%, at least 20%, at least 25%, at least 30%, at least 40%, at least 50%, at least 60%, at least 70%, at least 75%, at least 80%, at least 85, at least 90%, at least 95%, at least 100%, at least 125%, at least 150%, or at least 200%, or at least 300%, or at least 400%, or at least 500% or more that of the control or control sample.

Type I IFN or IFNα-inducible PD marker expression profile may be calculated as the average fold increase in the expression or activity of the set of genes comprised by the PD marker. The Type I IFN or IFNα-inducible PD marker expression profile may also be calculated as the difference between the mean Ct (cycle threshold) for the four target genes and the mean Ct of three control genes.

The average fold increase in the expression or activity of the set of genes may be between at least about 2 and at least about 15, between at least about 2 and at least about 10, or between at least about 2 and at least about 5. The average fold increase in the expression or activity of the set of genes may be at least about 2, at least about 2.5, at least about 3, at least about 3.5, at least about 4, at least about 4.5, at least about 5, at least about 5.5, at least about 6, at least about 6.5, at least about 7, at least about 8, at least about 9 or at least about 10.

The degree of increased expression permits the identification of a fold change cutoff for identifying signature positive and signature negative patients suffering from autoimmune diseases. In one embodiment, the cutoff is at least about 2. In another embodiment, the cutoff is at least about 2.5. In another embodiment, the cutoff is at least about 3. In another embodiment, the cutoff is at least about 3.5. In another embodiment, the cutoff is at least about 4. In another embodiment, the cutoff is at least about 4.5. In another embodiment, the cutoff is chosen from at least 3.5, 3.6, 3.7, 3.8, 3.9, 4.0, 4.1, 4.2, 4.3, 4.4, and 4.5. In another embodiment the cutoff is between about 2 and about 8. In one embodiment, the cutoff is the mean of the increased expression levels of at least four of IFI27, IFI44, IFI44L, IFI6 and RSAD2. In another embodiment, the cutoff is the median of the increased expression levels of at least four of IFI27, IFI44, IFI44L, IFI6 and RSAD2.

The degree of increased expression also permits the identification of a delta Ct cutoff for identifying signature positive and signature negative patients suffering from autoimmune diseases. In one embodiment, the cutoff is at least about 7.6. In another embodiment, the cutoff is 7.56. The fold change cutoff may be used to determine an appropriate delta Ct cutoff (e.g., 1 < log2 of the fold change < 3 corresponds to delta Ct range of 8.65 to 6.56.). Thus, in another embodiment, the delta Ct cutoff is between about 6.56 to about 8.56.

Furthermore, the patient may overexpress or have a tissue that overexpresses a type I IFN subtype at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control. The type I IFN subtype may be any one of IFNα1, IFNα2, IFNα4, IFNα5, IFNα6, IFNα7, IFNα8, IFNα10, IFNα14, IFNα17, IFNα21, IFNβ, or IFNω. The type I IFN subtypes may include all of IFNα1, IFNα2, IFNα8, and IFNα14.

The up-regulated expression or activity of any gene detected in a sample, by probes, or by probes in kits in an IFNα-inducible PD marker expression profile may be at least 1.2-fold, at least 1.25-fold, at least 1.3-fold, at least 1.4-fold, at least 1.5-fold, at least 2.0-fold, at least 2.25-fold, at least 2.5-fold, at least 2.75-fold, at least 3.0-fold, at least 3.5-fold, at least 4.0-fold, at least 4.5-fold, at least 5.0-fold, at least 6.0-fold, at least 7.0-fold, at least 8.0-fold, at least 9.0-fold, at least 10.0-fold, at least 15.0-fold, at least 20.0-fold, at least 25.0-fold, or at least 50.0-fold relative to baseline levels of control cells, *e.g.,* cells of healthy volunteers or cells of control animals or cells not exposed to IFNα in culture. All of the genes in the IFNα-inducible PD marker expression profile may have up-regulated expression or activity at the same fold increase. Alternatively, the genes in the PD marker expression profile may have varying levels of up-regulated expression or activity.

### A. Measuring Upregulation

Up- or down-regulation of gene expression or activity of IFNα-inducible PD markers may be determined by any means known in the art. For example, up- or down-regulation of gene expression may be detected by determining mRNA levels. mRNA expression may be determined by northern blotting, slot blotting, quantitative reverse transcriptase polymerase chain reaction, or gene chip hybridization techniques. See U.S. Pat. Nos. 5,744,305 and 5,143,854 for examples of making nucleic acid arrays for gene chip hybridization techniques. *See* Establishing and functional characterization of an HEK-293 cell line expressing autofluorescently tagged β-actin (pEYFP-ACTIN) and the neurokinin type 1 receptor (NK1-R) Hrovat, A; Zavec, AB; Pogacnik, A; Frangez, R; Vrecl, M 2010 Cellular & Molecular Biology Letters 1, 55-69, Expression profiles of proliferative and antiapoptotic genes in sporadic and colitis-related mouse colon cancer models Svec, J; Ergang, P; Mandys, V; Kment, M; Pacha, J 2010 International Journal of Experimental Pathology 1, 44-53, and Protein kinase inhibitors emodin and dichloro-ribofuranosylbenzimidazole modulate the cellular accumulation and cytotoxicity of cisplatin in a schedule-dependent manner Kurokawa, T; He, GA; Siddik, ZH 2010 Cancer Chemotherapy and Pharmacology 3, 427-436, for examples of how to use the TAQMAN® method for measuring gene expression.

Primers that selectively bind to targets in polymerase chain reactions (PCR) can be chosen based on empirically determining primers that hybridize in a PCR reaction and produce sufficient signal to detect the target over background, or can be predicted using the melting temperature of the primer:target duplex as described in Maniatis et al. Molecular Cloning, Second Edition, Section 11.46. 1989. Similarly, probes for detecting PCR products in a TAQMAN® or related method can be empirically chosen or predicted. Such primers and probes (collectively "oligonucleotides") may be between 10 and 30 nucleotides or greater in length.

Up- or down-regulation of gene expression or activity of IFNα-inducible PD markers may be determined by detecting protein levels. Methods for detecting protein expression levels include immuno-based assays such as enzyme-linked immunosorbant assays, western blotting, protein arrays, and silver staining.

An IFNα-inducible PD marker expression profile may comprise a profile of protein activity. Up- or down-regulation of gene expression or activity of IFNα-inducible PD markers may be determined by detecting activity of proteins including, but not limited to, detectable phosphorylation activity, de-phosphorylation activity, or cleavage activity. Furthermore, up- or down-regulation of gene expression or activity of IFNα-inducible PD markers may be determined by detecting any combination of these gene expression levels or activities.

## III. Type I IFN or an IFNα-inducible disease, disorder, or conditions

A type I IFN or an IFNα-inducible disease, disorder, or condition is any that exhibits a type I IFN or an IFNα PD marker expression profile or gene signature. A PD marker expression profile and a gene signature will be understood to be equivalent. These diseases, disorders, or conditions include those with an autoimmune component such as systemic lupus erythematosus (SLE), insulin dependent diabetes mellitus, inflammatory bowel disease (including Crohn's disease, ulcerative colitis, and Celiac's disease), multiple sclerosis, psoriasis, autoimmune thyroiditis, schleroderma, rheumatoid arthritis, glomerulonephritis, idiopathic inflammatory myositis, Sjogren's syndrome, vasculitis, inclusion body myositis (IBM), dermatomyositis, polymyositis, lupus nephritis, and sarcoidosis. Other diseases, disorders, or conditions include graft versus host disease and transplant rejection.

**A.** Patient Symptoms

The patients may also exhibit any of a number of symptoms as discussed in, e.g., International Application No. PCT/US2007/024941, or may have a clinical SLEDAI score or BILAG score as discussed in the same. These symptoms may include fatigue, organ damage, malar rash, and alopecia. The patient may be scored using a known clinical scoring system, e.g., SLEDAI which is an index of SLE disease activity as measured and evaluated within the last 10 days (Bombardier C, Gladman D D, Urowitz M B, Caron D, Chang C H and the Committee on Prognosis Studies in SLE: Derivation of the SLEDAI for Lupus Patients. Arthritis Rheum 35:630-640, 1992.). Disease activity under the SLEDAI scoring system can range from 0 to 105. The following categories of SLEDAI activity have been defined: no activity (SLEDAI = 0); mild activity (SLEDAI = 1-5); moderate activity (SLEDAI = 6-10); high activity (SLEDAI = 11-19); very high activity (SLEDAI = 20 or higher). (Griffiths, et al., Assessment of Patients with Systemic Lupus Erythematosus and the use of Lupus Disease Activity Indices). Another disease scoring index is the BILAG index which is an activity index of SLE that is based on specific clinical manifestations in eight organ systems: general, mucocutaneous, neurological, musculoskeletal, cardiovascular, respiratory, renal, and hematology results. Scoring is based on a letter system, but weighted numerical scores can also be assigned to each letter, making it possible to calculate a BILAG score in the range of 0-72. (Griffiths, et al., Assessment of Patients with Systemic Lupus Erythematosus and the use of Lupus Disease Activity Indices). Other scoring indices include the PGA score, the composite responder index (CRI), and the ANAM4™ test. The methods described herein, *e.g.,* of treating an autoimmune disorder, may be used for any subject identified as having any activity level of disease activity as measured by any classification methodology known in the art, e.g., mild, moderate, high, or very high. The methods described herein, e.g., of treating an autoimmune disorder, may result in a decrease in a patient's symptoms or may result in an improvement in a score of disease for the patient's type I IFN or an IFN$\alpha$-inducible disease, disorder, or condition.

**IV. Therapeutic Agents**

A therapeutic agent may be administered to a patient or a patient may be identified as a candidate for administration of an agent or a therapeutic agent. A therapeutic agent may modulate type 1 interferon or IFN$\alpha$ activity. Suitable therapeutic agents include molecules that bind to and modulate type I IFN or IFN$\alpha$ activity. Suitable therapeutic agents include molecules that bind to and modulate activity of receptors of type I interferons or IFN$\alpha$. The therapeutic agent may be a small molecule or a biological agent. If the therapeutic agent is a small molecule it may be synthesized or identified and isolated from a natural source.

If the therapeutic agent is a biological agent, it may be an antibody specific for any subtype(s) of type I IFN or IFN$\alpha$. For instance, the antibody may be specific for any one of IFN$\alpha$1, IFN$\alpha$2, IFN$\alpha$4, IFN$\alpha$5, IFN$\alpha$6, IFN$\alpha$7, IFN$\alpha$8, IFN$\alpha$10, IFN$\alpha$14, IFN$\alpha$17, IFN$\alpha$21, IFN$\beta$, or IFN$\omega$. Alternatively, the antibody may be specific for any two, any three, any four, any five, any six, any seven, any eight, any nine, any ten, any eleven, any twelve type I IFN of IFN$\alpha$ subtypes. If the antibody is specific for more than one type I IFN subtype, the antibody may be specific for IFN$\alpha$1, IFN$\alpha$2, IFN$\alpha$4, IFN$\alpha$5, IFN$\alpha$8, IFN$\alpha$10, and IFN$\alpha$21; or it may be specific for IFN$\alpha$1, IFN$\alpha$2, IFN$\alpha$4, IFN$\alpha$5, IFN$\alpha$8, and IFN$\alpha$10; or it may be specific for IFN$\alpha$1, IFN$\alpha$2, IFN$\alpha$4, IFN$\alpha$5, IFN$\alpha$8, and IFN$\alpha$21; or it may be specific for IFN$\alpha$1, IFN$\alpha$2, IFN$\alpha$4, IFN$\alpha$5, IFN$\alpha$10, and IFN$\alpha$21. Antibodies specific for type I IFN or IFN$\alpha$ include MEDI-545, any biologic or antibody other than MEDI-545, antibodies described in U.S. patent applications 11/009,410 filed December 10, 2004 and 11/157,494 filed June 20, 2005, 9F3 and other antibodies described in U.S. Patent No. 7,087,726 (Example 1 and Example 2, those disclosed in Table 3 and Table 4, and/or those disclosed in the table entitled "Deposit of Material" on lines 25-54, column 56), NK-2 and YOK5/19 (WO 84/03105), LO-22 (U.S. Patent 4,902,618), 144 BS (U.S. Patent 4,885,166), and EBI-1, EBI-2, and EBI-3 (EP 119476). A therapeutic agent that modulates IFN$\alpha$ activity may neutralize IFN$\alpha$ activity. One of skill in the art is well aware of preparation and formulation of such biological agents and methods of their administration.

MEDI-545 is a fully human, 147,000 Dalton IgGlk monoclonal antibody (Mab) that binds to a majority of interferon-alpha (IFN-$\alpha$) subtypes. MEDI-545 is made from 100% human protein sequences, thereby making it a fully human monoclonal antibody. Fully human monoclonal antibodies may have advantages over other forms of monoclonal antibodies, such as chimeric and humanized antibodies, as they may have a more favorable safety profile and may be eliminated less rapidly from the human body, thereby possibly reducing the frequency of dosing. MEDI-545 was derived from an IgG4$\kappa$ antibody, 13H5, which was selected based on functional assays as having the most desirable properties for a potential therapeutic agent. 13H5 was subsequently converted to an IgG1 antibody isotype, produced in CHO cells, and selected for further characterization and preclinical development with an initial designation of MDX-1103, now referred to as MEDI-545. See also U.S. Patent Application Publication No. 2007/0014724; PCT Application PCT/US2008/058133 filed March 25, 2008 entitled "Antibodies with Decreased Deamidation Profiles," and PCT Application PCT/US2008/058132 filed March 25, 2008.

The therapeutic agent may be an antibody against an interferon receptor, including those disclosed in U.S. Patent Nos. 7,619,070 and 7,662,381 and International Application No. PCT/US2009/033358.

### A. Antibodies

The antibody may be a synthetic antibody, a monoclonal antibody, polyclonal antibodies, a recombinantly produced antibody, an intrabody, a multispecific antibody (including bi-specific antibodies), a human antibody, a humanized antibody, a chimeric antibody, a single-chain Fv (scFv) (including bi-specific scFv), a BiTE molecule, a single chain antibody, a Fab fragments, a F(ab') fragment, a disulfide-linked Fv (sdFv), or an epitope-binding fragment of any of the above. The antibody may be any of an immunoglobulin molecule or immunologically active portion of an immunoglobulin molecule. Furthermore, the antibody may be of any isotype. For example, it may be any of isotypes IgG1, IgG2, IgG3 or IgG4. The antibody may be a full-length antibody comprising variable and constant regions, or an antigen-binding fragment thereof, such as a single chain antibody, or a Fab or Fab'2 fragment. The antibody may also be conjugated or linked to a therapeutic agent, such as a cytotoxin or a radioactive isotope.

In the methods of treatment a second agent other than an agent that binds to modulates IFNα activity, or an agent that binds to and modulates the activity of a receptor of a type I interferon or IFNα may be administered to the patient. Second agents include, but are not limited to, non-steroidal anti-inflammatory drugs such as ibuprofen, naproxen, sulindac, diclofenac, piroxicam, ketoprofen, diflunisal, nabumetone, etodolac, and oxaprozin, indomethacin; anti-malarial drugs such as hydroxychloroquine; corticosteroid hormones, such as prednisone, hydrocortisone, methylprednisolone, and dexamethasone; methotrexate; immunosuppressive agents, such as azathioprine and cyclophosphamide; and biologic agents that, e.g., target T cells such as Alefacept and Efalizumab, or target TNFα, such as, Enbrel, Remicade, and Humira.

### B. Identifying Candidate Therapeutic Agents

A candidate therapeutic for treating IFNα-mediated disorders may be identified by the methods encompassed by the disclosure. Candidate therapeutics may be any type of molecule including a small molecule or a biological agent. A candidate therapeutic identified by the methods encompassed by the disclosure may immediately be identified as useful as a therapeutic for a disease, disorder, or condition. Alternatively, a candidate therapeutic identified by the methods encompassed by the disclosure may need to be further tested and/or modified before selection for treating patients. Alternatively, a candidate therapeutic identified by the methods encompassed by the disclosure may, after further testing, be de-selected as a molecule for treating patients.

In methods that identify candidate therapeutics, cells comprising an IFNα-inducible PD marker expression profile are contacted with an agent. The cells may be any type of cells, such as commercially available immortalized cell lines that comprise an IFNα-inducible PD marker expression profile, commercially available immortalized cell lines that have been treated with IFNα to induce an IFNα-inducible PD marker expression profile, cells isolated from a patient having an IFNα-inducible PD marker expression profile, or cells isolated from a healthy patient and treated with IFNα to induce an IFNα-inducible PD marker expression profile.

Presence or absence of a change in the IFNα-inducible PD marker expression profile of the cells is detected following contacting the cells with the agent. Presence of change may be any change in IFNα-inducible PD marker expression profile including at least a 10% decrease in up-regulated expression or activity of at least 1 gene in the IFNα-inducible PD marker expression profile, at least a 20% decrease of the at least 1 up-regulated gene, at least a 30% decrease of the at least up-regulated 1 gene, at least a 40% decrease of the at least 1 up-regulated gene, at least a 50% decrease of the at least 1 up-regulated gene, at least a 60% decrease of the at least 1 up-regulated gene, at least a 70% decrease of the at least 1 up-regulated gene, at least a 75% decrease of the at least 1 up-regulated gene, at least an 80% decrease of the at least 1 up-regulated gene, at least an 85% decrease of the at least 1 up-regulated gene, at least a 90% decrease of the at least 1 up-regulated gene, at least a 95% decrease of the at least 1 up-regulated gene, at least a 96% decrease of the at least 1 up-regulated gene, at least a 97% decrease of the at least 1 up-regulated gene, at least a 98% decrease of the at least 1 up-regulated gene, at least a 99% decrease of the at least 1 up-regulated gene, or a 100% decrease of the at least 1 up-regulated gene.

### V. Neutralization of the type I IFN or IFNα-inducible profile in patients

Treatment with the agent may result in neutralization of the type I IFN or IFNα-inducible profile. Treatment with the agent may result in a decrease in one or more symptoms of the type I IFN or an IFNα-mediated disease or disorder. Treatment with the agent may result in fewer flare-ups related to the type I IFN or an IFNα-mediated disease or disorder. Treatment with the agent may result in improved prognosis for the patient having the type I IFN or an IFNα-mediated disease or disorder. Treatment with the agent may result in a higher quality of life for the patient. Treatment with the agent may alleviate the need to co-administer second agents or may lessen the dosage of administration of the second agent to the patient. Treatment with the agent may reduce the number of hospitalizations of the patient that are related to the type I IFN or an IFNα-mediated disease or disorder.

The agent that binds to and modulates type I IFN or IFNα activity may neutralize a type I IFN or IFNα-inducible profile. Neutralization of the type I IFN or IFNα-inducible profile may be a reduction in at least one, at least two, at least three, at least four genes. Neutralization of the type I IFN or IFNα-inducible profile is a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least four genes up-regulated in the type I IFN or IFNα-inducible profile. Alternatively, neutralization of the type I IFN or IFNα-inducible profile refers to a reduction of expression of up-regulated type I IFN or IFNα-inducible genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those type I IFN or IFNα-inducible genes in a control sample. If the agent that binds to and modulates type I IFN or IFNα activity is a biologic agent, such as an antibody, the agent may neutralize the type I IFN or IFNα profile at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively neutralize expression of one or more type I IFN or IFNα subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. These subtypes may include all of IFNα1, IFNα2, IFNα8, and IFNα14. Alternatively, these subtypes may include IFNα1, IFNα2, IFNα4, IFNα5, IFNα8, IFNα10, IFNα21. Neutralization of the IFNα or type-I IFN subtypes may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, or at least ten of the subtypes. Neutralization of the IFNα or type-I IFN subtypes may be a reduction in expression of IFNα or type-I IFN subtype genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those IFNα or type I IFN subtypes in a control sample. If the agent that binds to and modulates IFNα activity or type I IFN activity is a biologic agent, such as an antibody, the agent may neutralize the IFNα or type I IFN subtypes at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively neutralize expression of IFNα receptors, either IFNAR1 or IFNAR2, or both, or TNFα, or IFNγ, or IFNγ receptors (either IFNGR1, IFNGR2, or both IFNGR1 and IFNGR2). Neutralization of expression of IFNα receptors, either IFNAR1 or IFNAR2, or both, or TNFα, or IFNγ, or IFNγ receptors (either IFNGR1, IFNGR2, or both IFNGR1 and IFNGR2) may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, or at least six of these genes. Neutralization of expression of IFNα receptors, either IFNAR1 or IFNAR2, or TNFα, or IFNγ, or IFNγ receptors (either IFNGR1, IFNGR2, or both IFNGR1 and IFNGR2) is a reduction of expression of at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of these genes in a control sample. If the agent that binds to and modulates type I IFN or IFNα activity is a biologic agent, such as an antibody, the agent may neutralize expression of IFNα receptors IFNAR1 or IFNAR2, or TNFα, or IFNγ, or IFNγ receptors IFNGR1 or IFNGR2 at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

### C. Patient Samples

Samples may also be obtained from patients in the methods of the disclosure. Samples include any biological fluid or tissue, such as whole blood, saliva, urine, synovial fluid, bone marrow, cerebrospinal fluid, nasal secretions, sputum, amniotic fluid, bronchoalveolar lavage fluid, peripheral blood mononuclear cells, total white blood cells, lymph node cells, spleen cells, tonsil cells, or skin. The samples may be obtained by any means known in the art.

## VI. Methods of monitoring disease progression

In methods of monitoring disease progression of a patient samples from the patient may be obtained before and after administration of an agent, e.g., an agent that binds to and modulates type I IFN or IFNα activity, or an agent that binds to and does not modulate type I IFN or IFNα activity, or a combination of agents that may or may not include an agent that binds to and modulates type I IFN or IFNα activity. Type I IFN or IFNα inducible PD marker expression profiles are obtained in the (before and after agent administration) samples. The type I IFN or IFNα inducible PD marker expression profiles in the samples are compared. Comparison may be of the number of type I IFN or IFNα inducible PD markers present in the samples or may be of the quantity of type I IFN or IFNα inducible PD markers present in the samples, or any combination thereof. Variance indicating efficacy of the therapeutic agent may be indicated if the number or level (or any combination thereof) of up-regulated type I IFN or IFNα inducible PD markers decreases in the sample obtained after administration of the therapeutic agent relative to the sample obtained before administration of the therapeutic agent. The number of up-regulated type I IFN or IFNα inducible PD markers may decrease by at least 1, at least 2, at least 3, at least 4, at least 5, at least 6, at least 7, at least 8, at least 9, or at least 10 fold. The level of any given up-regulated type I IFN or IFNα inducible PD marker may decrease by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with decreased levels may be at least 1, at least 2, at least 3, or at least 4. Any combination of decreased number and decreased level of up-regulated type I IFN or IFNα inducible PD markers may indicate efficacy. Variance indicating efficacy of the therapeutic agent may be indicated if the number or level (or any combination thereof) of down-regulated type I IFN or IFNα inducible PD markers decreases in the sample obtained after administration of the therapeutic agent relative to the sample obtained before administration of the therapeutic agent.

The sample obtained from the patient may be obtained prior to a first administration of the agent, *i.e.,* the patient is naïve to the agent. Alternatively, the sample obtained from the patient may occur after administration of the agent in the course of treatment. For example, the agent may have been administered prior to the initiation of the monitoring protocol. Following administration of the agent an additional sample may be obtained from the patient and type I IFN or IFNα inducible PD markers in the samples are compared. The samples may be of the same or different type, e.g., each sample obtained may be a blood sample, or each sample obtained may be a serum sample. The type I IFN or IFNα inducible PD markers detected in each sample may be the same, may overlap substantially, or may be similar.

The samples may be obtained at any time before and after the administration of the therapeutic agent. The sample obtained after administration of the therapeutic agent may be obtained at least 2, at least 3, at least 4, at least 5, at least 6, at least 7, at least 8, at least 9, at least 10, at least 12, or at least 14 days after administration of the therapeutic agent. The sample obtained after administration of the therapeutic agent may be obtained at least 2, at least 3, at least 4, at least 5, at least 6, at least 7, or at least 8 weeks after administration of the therapeutic agent. The sample obtained after administration of the therapeutic agent may be obtained at least 2, at least 3, at least 4, at least 5, or at least 6 months following administration of the therapeutic agent.

Additional samples may be obtained from the patient following administration of the therapeutic agent. At least 2, at least 3, at least 4, at least 5, at least 6, at least 7, at least 8, at least 9, at least 10, at least 12, at least 15, at least 20, at least 25 samples may be obtained from the patient to monitor progression or regression of the disease or disorder over time. Disease progression may be monitored over a time period of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least 1 year, at least 2 years, at least 3 years, at least 4 years, at least 5 years, at least 10 years, or over the lifetime of the patient. Additional samples may be obtained from the patient at regular intervals such as at monthly, bi-monthly, once a quarter year, twice a year, or yearly intervals. The samples may be obtained from the patient following administration of the agent at regular intervals. For instance, the samples may be obtained from the patient at one week following each administration of the agent, or at two weeks following each administration of the agent, or at three weeks following each administration of the agent, or at one month following each administration of the agent, or at two months following each administration of the agent. Alternatively, multiple samples may be obtained from the patient following each administration of the agent.

Disease progression in a patient may similarly be monitored in the absence of administration of an agent. Samples may periodically be obtained from the patient having the disease or disorder. Disease progression may be identified if the number of type I IFN or IFNα inducible PD markers increases in a later-obtained sample relative to an earlier obtained sample. The number of type I IFN or IFNα inducible PD markers may increase by at least 1, at least 2, at least 3, at least 4, at least 5, at least 6, at least 7, at least 8, at least 9, or at least 10. Disease progression may be identified if level of any given up-regulated type I IFN or IFNα inducible PD marker increases by at least 10%, at least 20%, at least 25%, at least 30%, at least 35%, at least 40%, at least 50%, at least 60%, at least 70%, at least 80%, at least 90%, or at least 95%. Disease progression may be identified if level of any given down-regulated type I IFN or IFNα inducible PD marker decreases by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. The number of down-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. Any combination of increased number and increased level of up-regulated type I IFN or IFNα inducible PD marker may indicate disease progression. Alternatively, or in combination, any combination of decreased number and decreased level of down-regulated type I IFN or IFNα inducible PD marker may indicate disease progression. Disease regression may also be identified in a patient having a disease or disorder, not treated by an agent. In this instance, regression may be identified if the number of type I IFN or IFNα inducible PD markers decreases in a later-obtained sample relative to an earlier obtained sample. The number of type I IFN or IFNα inducible PD markers may decrease by at least 1, at least 2, at least 3, at least 4, at least 5, at least 6, at least 7, at least 8, at least 9, or at least 10. Disease regression may be identified if level of any given up-regulated type I IFN or IFNα inducible PD marker decreases by at least 10%, at least 20%, at least 25%, at least 30%, at least 35%, at least 40%, at least 50%, at least 60%, at least 70%, at least 80%, at least 90%, or at least 95%. Disease regression may be identified if level of any given down-regulated type I IFN or IFNα inducible PD marker increases by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. The number of down-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. Disease progression or disease regression may be monitored by obtaining samples over any period of time and at any interval. Disease progression or disease regression may be monitored by obtaining samples over the course of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least 1 year, at least 2 years, at least 3 years, at least 4 years, at least 5 years, at least 10 years, or over the lifetime of the patient. Disease progression or disease regression may be monitored by obtaining samples at least monthly, bi-monthly, once a quarter year, twice a year, or yearly. The samples need not be obtained at strict intervals.

**VII. Kits and Probes**

The disclosure also encompasses kits and probes. The probes may be any molecule that detects any expression or activity of any gene that may be included in an IFNα-inducible PD marker expression profile.

**VIII. Methods of Detecting IFN Activity**

The disclosure also encompasses methods of detecting IFN activity. These methods may employ cells comprising a polynucleotide sequence comprising a reporter gene under the control of an interferon-stimulated response element. The cells comprising the polynucleotide sequence may be any cells amenable to transfection or transformation with a polynucleotide sequence and that can be maintained in culture. These cells include animal cells, bacterial cells, yeast cells, insect cells, or plant cells. These cells may be adherent or may grow in suspension. If the cells are animal cells, they may be from a known cell line such as HeLa, COS, NIH3T3, AGS, 293, CHO, Huh-7, HUVEC, MCF-7, C6, BHK-21, BNL CL 2, C2C12, HepG2, and ATDC5. Countless other cell lines are known and can be obtained by those of skill in the art. The cells may alternatively be primary cells that have or have not been immortalized.

The cells may comprise a polynucleotide sequence comprising a reporter gene under the control of an interferon-stimulated response element. The polynucleotide sequence may be stably integrated in the DNA of the cell or may be an extrachomosomal element that is stably or transiently in the cell. The polynucleotide may have been introduced to the cell as a naked polynucleotide molecule, a polynucleotide molecule complexed with lipids or other molecules, or a polynucleotide in a virus particle.

If the polynucleotide was introduced as a naked polynucleotide molecule, the polynucleotide may have been a linear or a circular molecule. Non-limiting examples of circular polynucleotide molecules include plasmids, and artificial chromosomes. These vectors may be cleaved with enzymes, for example, to generate linear polynucleotide molecules.

Furthermore, if the polynucleotide was introduced as a naked polynucleotide it may have been introduced into the cells by any of many well known techniques in the art. These techniques include, but are not limited to, electroporation, microinjection, and biolistic particle delivery. See, also, e.g., Loeffler and Behr, 1993, Meth. Enzymol. 217:599-618; Cohen et al., 1993, Meth. Enzymol. 217:618-644; Clin. Pharma. Ther. 29:69-92 (1985), Sambrook, et al. Molecular Cloning: A Laboratory Manual. 2nd, ed., Cold Spring Harbor Laboratory, Cold Spring Harbor Laboratory Press, Cold Spring Harbor, N.Y., 1989 and Ausubel et al., ed. Current Protocols in Molecular Biology, John Wiley & Sons, Inc., N.Y., N.Y. (1987-2001).

If the polynucleotide was introduced as a complex with lipids or liposomes, it too may have been introduced by one of many known techniques to the skilled artisan. Lipids or liposomes comprise a mixture of fat particles or lipids which bind to DNA or RNA to provide a hydrophobic coated delivery vehicle. Suitable liposomes may comprise any of the conventional synthetic or natural phospholipid liposome materials including phospholipids from natural sources such as egg, plant or animal sources such as phosphatidylcholine, phosphatidylethanolamine, phosphatidylglycerol, sphingomyelin, phosphatidylserine or phosphatidylinositol. Synthetic phospholipids also may be used, e.g., dimyristoylphosphatidylcholine, dioleoylphosphatidylcholine, dioleoylphosphatidycholine and corresponding synthetic phosphatidylethanolamines and phosphatidylglycerols. Lipids or liposomes that may be conjugated with the vector are also commercially available to the skilled artisan. Examples of commercially available lipid or liposome transfection reagents known to those of skill in the art include LIPOFECTAMINE™ (Invitrogen), GENEJUICE® (Novagen), GENEJAMMER® (Stratagene), FUGENE®HD (Roche), MEGAFECTIN™ (Qbiogene), SUPERFECT® (Qiagen), and EFFECTENE® (Qiagen).

If the polynucleotide was introduced as a complex with other molecules it may have been compacted or in a nanosphere. Compacted polynucleotide complexes are described in U.S. Patents 5,972,901, 6,008,336, and 6,077,835. Nanospheres are described in U.S. Patent Nos. 5,718,905 and 6,207,195. These compacted polynucleotide complexes and nanospheres that complex nucleic acids utilize polymeric cations. Typical polymeric cations include gelatin, poly-L-lysine, and chitosan. Alternatively, the polynucleotide may have been complexed with DEAE-dextran, or transfected using techniques such as calcium phosphate coprecipitation, or calcium chloride coprecipitation.

If the polynucleotide was introduced associated with a virus, the virus may have been any well known suitable virus for polynucleotide delivery. Example viruses that may be used as vectors include adenovirus, adeno-associated virus, lentivirus, retrovirus, herpes virus (e.g. herpes simplex virus), vaccina virus, papovirus, Sendai virus, SV40 virus, respiratory syncytial virus, etc.

The polynucleotide sequence may include a reporter gene and an interferon-stimulated response element. The reporter gene may be any one of luciferase, chloramphenicol acetyl transferase, β-galactosidase, green fluorescent protein, β-glucuronidase, or secreted placental alkaline phosphatase. Variations of many of these reporter genes, *e.g.,* green fluorescent protein and luceriferase, are known and can be readily identified and/or produced by those of skill in the art. Other reporter genes in addition to those listed will also be known to those of skill in the art and are readily available. Interferon-stimulated response elements are also known to those of skill in the art. They may be obtained from commercial vendors such as Stratagene, Clonetech, and Biomyx. They have also been reported in, for instance, Alcantara et al. (Nuc. Acid. Res. 30 (2002):2068-2075 and Kirchhoff et al. (Oncogene 18 (1999):3725-3736).

The cells employed in the assay may be incubated with a sample. The sample may be obtained from a patient, from a vendor with patient samples, or a control sample used for calibration or as a control. If the sample is obtained from a patient it may be any biological fluid or tissue, such as whole blood, saliva, urine, synovial fluid, bone marrow, cerebrospinal fluid, nasal secretions, sputum, amniotic fluid, bronchoalveolar lavage fluid, peripheral blood mononuclear cells, total white blood cells, lymph node cells, spleen cells, tonsil cells, or skin.

Expression of the reporter gene is detected by any well known means in the art. The expression, even if "0" indicates IFN activity in the sample. One of skill in the art may further quantitate any level of expression of the reporter gene which may then correlate to level of IFN activity in the sample.

Applicants provide a set of non-limiting embodiments to describe some of the aspects of the disclosure.

**EMBODIMENTS**

Embodiment 1. A method of treating a patient having a type I IFN or an IFNα-mediated disease or disorder comprising:

administering an agent that binds to and modulates type I IFN or IFNα activity;

wherein the patient comprises a type I IFN or IFNα-inducible PD marker expression profile;

and wherein the agent neutralizes the type I IFN or IFNα-inducible PD marker expression profile of the patient.

Embodiment 2. A method of treating an autoimmune disease patient comprising a moderate or strong type I IFN or an IFNα PD marker profile comprising:

administering an agent that binds to and modulates type I IFN or IFNα activity;

wherein the agent neutralizes the type I IFN or IFNα-inducible PD marker expression profile of the patient.

Embodiment 3. A method of neutralizing a type I IFN or IFNα-inducible PD marker expression profile in a patient having a disease or disorder, comprising:

administering an agent that binds to and modulates type I IFN or IFNα activity to the patient;

wherein the agent neutralizes the type I IFN or IFNα-inducible PD marker expression profile of the patient.

Embodiment 4. The method of any one of embodiments 1 to 3 further comprising detecting neutralization of the type I IFN or IFNα-inducible PD marker expression profile of the patient.

Embodiment 5. The method of any one of embodiments 1 to 4 wherein the type I IFN or IFNα-inducible PD marker expression profile comprises transcripts of PD marker genes.

Embodiment 6. The method of any one of embodiments 1 to 4 wherein the type I IFN or IFNα-inducible PD marker expression profile comprises polypeptides expressed from PD marker genes.

Embodiment 7. The method of any one of embodiments 1 to 6 wherein the type I IFN or IFNα-inducible PD marker expression profile comprises up-regulated expression or activity of a set of genes chosen from of:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 8. The method of any one of embodiments 1 to 6 wherein the type I IFN or IFNα-inducible PD marker expression profile consists of up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 9. The method embodiment 7 or 8 wherein the up-regulated expression or activity of a set of genes is calculated as an average fold increase in the expression or activity of the set of genes.

Embodiment 10. The method embodiment 9 wherein the average fold increase in the expression or activity of the set of genes is between at least about 3 and at least about 15, between at least about 3 and at least about 10, or between at least about 3 and at least about 5.

Embodiment 11. The method embodiment 9 wherein the average fold increase in the expression or activity of the set of genes is at least about 2, at least about 2.5, at least about 3, at least about 3.5, at least about 4, at least about 4.5, at least about 5, at least about 5.5, at least about 6, at least about 6.5, at least about 7, at least about 8, at least about 9 or at least about 10.

Embodiment 12. The method of any one of embodiments 1 to 11 wherein the agent is a biologic agent.

Embodiment 13. The method of embodiment 12 wherein the agent is an antibody.

Embodiment 14. The method of embodiment 13 wherein the antibody is MEDI-545.

Embodiment 15. The method of embodiment 13 wherein the antibody is specific for one or more type I IFN or IFNα subtype but is not MEDI-545.

Embodiment 16. The method of any one of embodiments 1 to 15 wherein the administering the agent alleviates one or more symptoms of the disease or disorder.

Embodiment 17. The method of any one of embodiments 13 to 16 wherein the antibody is administered at a dose between approximately 0.03 and 30 mg/kg.

Embodiment 18. The method of embodiment 17 wherein the antibody is administered at a dose between 0.3 and 3 mg/kg or between .03 and 1 mg/kg.

Embodiment 19. The method of any one of embodiments 1 to 18 wherein the agent neutralizes the type I IFN or IFNα-inducible PD marker expression profile of the patient by at least 10%, by at least 20%, by at least 30%, by at least 40% or by at least 50%.

Embodiment 20. The method of any one of embodiments 1 to 19 wherein the disease or disorder is one of lupus, lupus nephritis, dermatomyositis, polymyositis, psoriasis, SSc, vasculitis, sarcoidosis, Sjogren's syndrome, or idiopathic inflammatory myositis.

Embodiment 21. The method of embodiment 20 wherein the disease or disorder is lupus.

Embodiment 22. The method of embodiment 20 wherein the disease or disorder is psoriasis.

Embodiment 23. The method of any one of embodiments 1 to 22 wherein the type I IFN or IFNα-inducible PD marker expression profile comprises up-regulated expression or activity of at least IFNα subtypes 1, 2, 8, and 14.

Embodiment 24. A method of monitoring or prognosing autoimmune disease progression of a patient comprising:

obtaining a first IFNα-inducible PD marker expression profile in a first sample from a patient.

Embodiment 25. The method of embodiment 24 wherein the first IFNα-inducible PD marker expression profile is a strong profile and the patient prognosis is disease progression.

Embodiment 26. The method of embodiment 25 wherein the autoimmune disease is SLE and the progression is an SLE flare.

Embodiment 27. The method of embodiment 26 wherein the first IFNα-inducible PD marker expression profile is a weak profile and the patient prognosis is disease regression.

Embodiment 28. The method of embodiment 24 further comprising:

obtaining a second IFNα-inducible PD marker expression profile in a second sample from a patient;

wherein an increase in number or level of type I IFN or IFNα inducible PD markers in the second relative to the first expression profile prognoses disease progression; or

wherein a decrease in number or level of type I IFN or IFNα inducible PD markers in the second relative to the first expression profile prognoses disease regression.

Embodiment 29. The method of any one of embodiments 26 to 28 wherein the type I IFN or IFNα-inducible PD marker expression profile comprises transcripts of PD marker genes.

Embodiment 30. The method of any one of embodiments 24 to 28 wherein the type I IFN or IFNα-inducible PD marker expression profile comprises polypeptides expressed from PD marker genes.

Embodiment 31. The method of any one of embodiments 24 to 28 wherein the type I IFN or IFNα-inducible PD marker expression profile comprises expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 32. The method of any one of embodiments 24 to 28 wherein the type I IFN or IFNα-inducible PD marker expression profile consists of expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 33. A method of monitoring disease progression of a patient receiving treatment with a therapeutic agent that binds to and modulates IFNα activity comprising:

obtaining a first IFNα-inducible PD marker expression profile in a first sample from the patient;

administering a therapeutic agent that binds to and modulates IFNα activity;

obtaining a second IFNα-inducible PD marker expression profile in a second sample from the patient; and

comparing the first and the second IFNα-inducible PD marker expression profiles,

wherein a variance in the first and the second IFNα-inducible PD marker expression profiles indicates a level of efficacy of the therapeutic agent that binds to and modulates IFNα activity.

Embodiment 34. The method of embodiment 33 wherein the first type I IFN or IFNα-inducible PD marker expression profile comprises up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 35. The method of embodiment 33 wherein the first type I IFN or IFNα-inducible PD marker expression profile consists of up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 36. The method of embodiment 34 or 35 wherein the variance is a decrease in up-regulated expression of activity levels of the genes.

Embodiment 37. The method of any one of embodiments 33 to 35 wherein the disease or disorder is one of lupus, lupus nephritis, dermatomyositis, polymyositis, psoriasis, SSc, vasculitis, sarcoidosis, Sjogren's syndrome, or idiopathic inflammatory myositis.

Embodiment 38. The method of embodiment 37 wherein the disease is lupus.

Embodiment 39. The method of any one of embodiments 33 to 38 wherein the therapeutic agent is a small molecule or a biologic agent.

Embodiment 40. The method of embodiment 39 wherein the biologic agent is an antibody.

Embodiment 41. The method of embodiment 40 wherein the antibody is MEDI-545 and/or an antibody specific for one or more type I IFN or IFNα subtype but is not MEDI-545.

Embodiment 42. The method of any one of embodiments 33 to 41 wherein the first IFNα-inducible PD marker expression profile is obtained prior to administration of the therapeutic agent.

Embodiment 43. The method of any one of embodiments 33 to 41 wherein the first IFNα-inducible PD marker expression profile is obtained at the time of administration of the therapeutic agent.

Embodiment 44. The method of any one of embodiments 33 to 43 wherein the first and the second sample are whole blood or serum.

Embodiment 45. The method of any one of embodiments 33 to 44 further comprising obtaining a third IFNα-inducible PD marker expression profile in a third sample from the patient.

Embodiment 46. The method of embodiment 45 further comprising obtaining a fourth IFNα-inducible PD marker expression profile in a fourth sample from the patient.

Embodiment 47. The method of embodiment 46 further comprising obtaining a fifth IFNα-inducible PD marker expression profile in a fifth sample from the patient.

Embodiment 48. The method of embodiment 47 further comprising obtaining a sixth IFNα-inducible PD marker expression profile in a sixth sample from the patient.

Embodiment 49. The method of any one of embodiments 33 to 48 wherein the second sample is obtained at least one week, at least two weeks, at least three weeks, at least one month or at least two months following administration of the therapeutic agent.

Embodiment 50. The method of embodiment 45 wherein the third sample is obtained at least 2 days, at least 5 days, at least one week, at least 2 weeks, at least three weeks, at least one month or at least two months following obtaining the second sample.

Embodiment 51. The method of embodiment 46 wherein the fourth sample is obtained at least two days, at least five days, at least one week, at least two weeks, at least three weeks, at least one month or at least two months following obtaining the third sample.

Embodiment 52. The method of embodiment 47 wherein the fifth sample is obtained at least two days, at least five days, at least one week, at least two weeks, at least three weeks, at least one month or at least two months following obtaining the fourth sample.

Embodiment 53. The method of any one of embodiments 33 to 52 wherein variance is a decrease in up-regulated expression or activity of the gene.

Embodiment 54. The method of embodiment 53 wherein the decrease is at least 10%, at least 20%, at least 25%, at least 30%, at least 40%, at least 45%, at least 50%, at least 60%, at least 70%, at least 75%, at least 80%, at least 85%, at least 90%, at least 95%, at least 96%, at least 97%, at least 98%, at least 99%, or 100%.

Embodiment 55. A method of identifying a patient as a candidate for a therapeutic agent that binds to and modulates IFN$\alpha$ activity comprising:

detecting presence or absence of an IFN$\alpha$-inducible PD marker expression profile in a sample from the patient,

wherein detecting presence of the IFN$\alpha$-induced PD marker expression profile identifies the patient as a candidate for the therapeutic agent that binds to and modulates IFN$\alpha$ activity.

Embodiment 56. The method of embodiment 55 wherein the IFN$\alpha$-inducible PD marker expression profile comprises up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 57. The method of embodiment 55 wherein the IFN$\alpha$-inducible PD marker expression profile consists of up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 58. The method embodiment 56 or 57 wherein the up-regulated expression or activity of a set of genes is calculated as an average fold increase in the expression or activity of the set of genes.

Embodiment 59. The method embodiment 58 wherein the average fold increase in the expression or activity of the set of genes is at least about 2, at least about 3, and at least about 4.

Embodiment 60. The method embodiment 59 wherein the average fold increase in the expression or activity of the set of genes is at least about 2, at least about 2.5, at least about 3, at least about 3.5, at least about 4, at least about 4.5, at least about 5, at least about 5.5, at least about 6, at least about 6.5, at least about 7, at least about 8, at least about 9 or at least about 10.

Embodiment 61. The method of any one of embodiments 55 to 60 wherein the patient has been diagnosed as having a disorder chosen from lupus, lupus nephritis, dermatomyositis, polymyositis, psoriasis, SSc, vasculitis, sarcoidosis, Sjogren's syndrome, or idiopathic inflammatory myositis.

Embodiment 62. The method of embodiment 61 wherein the disorder is lupus.

Embodiment 63. The method of any one of embodiments 55 to 62 wherein the therapeutic agent is a small molecule or a biologic agent.

Embodiment 64. The method of embodiment 63 wherein the biologic agent is an antibody.

Embodiment 65. The method of embodiment 64 wherein the antibody is MEDI-545.

Embodiment 66. The method of embodiment 64 wherein the antibody is specific for one or more type I IFN or IFN$\alpha$ subtype but is not MEDI-545.

Embodiment 67. The method of any one of embodiments 55 to 66 wherein the up-regulated expression or activity comprises an increase in mRNA levels of one or more of the genes.

Embodiment 68. The method of any one of embodiments 55 to 66 wherein the up-regulated expression or activity comprises an increase in protein levels of one or more of the genes.

Embodiment 69. The method of any one of embodiments 55 to 66 wherein the up-regulated expression or activity comprises an increase in enzymatic activity of a protein expressed from one or more of the genes.

Embodiment 70. The method of any one of embodiments 55 to 69 wherein the sample is whole blood.

Embodiment 71. A method of diagnosing a patient as a having a disorder associated with increased IFN$\alpha$ levels comprising:

detecting presence or absence of an IFN$\alpha$-inducible PD marker expression profile in a sample from the patient,

wherein detecting presence of the IFN$\alpha$-induced PD marker expression profile identifies the patient as having a disorder associated with increased IFN$\alpha$ levels.

Embodiment 72. The method of embodiment 71 wherein the IFN$\alpha$-inducible PD marker expression profile comprises up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 73. The method of embodiment 71 wherein the IFNα-inducible PD marker expression profile consists of up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 74. The method embodiment 72 or 73 wherein the up-regulated expression or activity of a set of genes is calculated as an average fold increase in the expression or activity of the set of genes.

Embodiment 75. The method embodiment 74 wherein the average fold increase in the expression or activity of the set of genes is between at least about at least about 2, at least about 3 and at least about 4.

Embodiment 76. The method embodiment 74 wherein the average fold increase in the expression or activity of the set of genes is at least about 2, at least about 2.5, at least about 3, at least about 3.5, at least about 4, at least about 4.5, at least about 5, at least about 5.5, at least about 6, at least about 6.5, at least about 7, at least about 8, at least about 9 or at least about 10.

Embodiment 77. The method of any one of embodiments 72 to 76 wherein the disease or disorder is one of lupus, lupus nephritis, dermatomyositis, polymyositis, psoriasis, SSc, vasculitis, sarcoidosis, Sjogren's syndrome, or idiopathic inflammatory myositis.

Embodiment 78. The method of embodiment 77 wherein the disorder is lupus.

Embodiment 79. The method of any one of embodiments 72 to 78 wherein the up-regulated expression or activity comprises an increase in mRNA levels of one or more of the genes.

Embodiment 80. The method of any one of embodiments 72 to 78 wherein the up-regulated expression or activity comprises an increase in protein levels of one or more of the genes.

Embodiment 81. The method of any one of embodiments 72 to 78 wherein the up-regulated expression or activity comprises an increase in enzymatic activity of a protein expressed from one or more of the genes.

Embodiment 82. A method of identifying a candidate therapeutic for treating IFNα-mediated disorders comprising:

contacting cells comprising an IFNα-inducible PD marker expression profile with an agent; and

detecting presence or absence of a change in the IFNα-induced PD marker expression profile of the cells,

wherein the presence of a change comprising a reduction in the up-regulation of the genes of the IFNα-inducible PD marker expression profile indicates the agent is a candidate therapeutic agent.

Embodiment 83. The method of embodiment 82 wherein the IFNα-inducible PD marker expression profile comprises up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 84. The method of embodiment 82 wherein the IFNα-inducible PD marker expression profile consists of up-regulated expression or activity of a set of genes chosen from:

(a) IFI27, IFI44, IFI44L, IFI6, and RSAD2;

(b) IFI44, IFI44L, IFI6, and RSAD2;

(c) IFI27, IFI44L, IFI6, and RSAD2;

(d) IFI27, IFI44, IFI6, and RSAD2;

(e) IFI27, IFI44, IFI44L, and RSAD2; and

(f) IFI27, IFI44, IFI44L, and IFI6.

Embodiment 85. The method embodiment 83 or 84 wherein the up-regulated expression or activity of a set of genes is calculated as an average fold increase in the expression or activity of the set of genes.

Embodiment 86. The method embodiment 85 wherein the average fold increase in the expression or activity of the set of genes is between at least about 3 and at least about 15, between at least about 3 and at least about 10, or between at least about 3 and at least about 5.

Embodiment 87. The method embodiment 85 wherein the average fold increase in the expression or activity of the set of genes is at least about 2, at least about 2.5, at least about 3, at least about 3.5, at least about 4, at least about 4.5, at least about 5, at least about 5.5, at least about 6, at least about 6.5, at least about 7, at least about 8, at least about 9 or at least about 10.

Embodiment 88. The method of any one of embodiments 83 to 87 wherein the cells obtained from a patient comprising a disorder associated with increased IFNα levels.

Embodiment 89. The method of any one of embodiments 83 to 87 wherein the cells are cells treated with IFNα to induce the IFNα-inducible PD marker expression profile.

Embodiment 90. The method of any one of embodiments 83 to 89 wherein the up-regulation of the genes of the IFNα-inducible PD marker expression profile comprises an increase in mRNA levels of one or more of the genes.

Embodiment 91. The method of any one of embodiments 83 to 89 wherein the up-regulation of the genes of the IFNα-inducible PD marker expression profile comprises an increase in protein levels of one or more of the genes.

Embodiment 92. The method of any one of embodiments 83 to 89 wherein the up-regulation of the genes of the IFNα-inducible PD marker expression profile comprises an increase in enzymatic activity of a protein expressed from one or more of the genes.

Embodiment 93. A set of primers comprising polynucleotides that specifically amplify and detect expression of any one of the following sets of genes:

(a) IFI27, IFI44, IFI44L, IFI6, and RSAD2; or

(b) IFI44, IFI44L, IFI6, and RSAD2; or

(c) IFI27, IFI44L, IFI6, and RSAD2; or

(d) IFI27, IFI44, IFI6, and RSAD2; or

(e) IFI27, IFI44, IFI44L, and RSAD2; or

(f) IFI27, IFI44, IFI44L, and IFI6.

Embodiment 94. The set of primers of embodiment 93 further comprising primers for amplifying and detecting 18S, ACTB, and GAPDH.

Embodiment 95. A set of primers consisting of polynucleotides that specifically detect expression of any one of the sets of genes:

(a) IFI27, IFI44, IFI44L, IFI6, and RSAD2; or

(b) IFI44, IFI44L, IFI6, and RSAD2; or

(c) IFI27, IFI44L, IFI6, and RSAD2; or

(d) IFI27, IFI44, IFI6, and RSAD2; or

(e) IFI27, IFI44, IFI44L, and RSAD2; or

(f) IFI27, IFI44, IFI44L, and IFI6.

Embodiment 96. The embodiments 93 and 94, wherein the set of primers have sequences of SEQ ID NOs 1-24.

Embodiment 96. Any of embodiments 1-95, wherein IFI27, IFI44, IFI44L, IFI6, and RSAD2 have the sequences of SEQ ID NOs: 25-32.

Embodiment 97. A kit comprising the primers of embodiments 93-96.

Embodiment 98. Any of embodiments 1-96, wherein the increased expression is the mean or median increased expression in the mRNA levels of IFI27, IFI44, IFI44L, IFI6, and RSAD2.

Embodiment 99. A method of identifying a subject suitable for treatment with a therapeutic agent that modulates type 1 interferon activity comprising detecting increased mRNA of at least four of IFI27, IFI44, IFI44L, IFI6, and RSAD2 in a sample of the subject, wherein an increase in mRNA of at least about four fold indicates a subject suitable for treatment with the agent.

Embodiment 100. The method of embodiment 99, wherein the mRNA is increased relative to the mRNA of at least four of IFI27, IFI44, IFI44L, IFI6, and RSAD2 in pooled samples from healthy patients.

Embodiment 101. The method of any of embodiments 99 or 100, wherein the increased mRNA is relative to the mRNA of one or more control genes present in the sample.

Embodiment 102. The method of any of embodiments 99-101, wherein the one or more control genes are chosen from ACTB, GAPDH, and 18S rRNA.

Embodiment 103. The method of any of embodiment 99-102, wherein increased mRNA of IFI27, IFI44, IFI44L, and RSAD2 is detected.

Embodiment 104. The method of any of embodiment 99-103, wherein the agent is chosen from an anti-interferon alpha antibody and an anti-interferon alpha receptor antibody.

Embodiment 105. The method of embodiment of any of 99-104, wherein the anti-interferon antibody is sifalimumab.

Embodiment 106. The method of embodiment of any of 99-105, wherein the anti-interferon antibody is not sifalimumab.

Embodiment 107. The method of embodiment of any of 99-106 wherein detecting mRNA of at least IFI27, IFI44, IFI44L, IFI6, and RSAD2 comprises

1) isolating RNA from a sample obtained from the subject;

2) synthesizing cDNA from the RNA;

3) hybridizing the cDNA with oligonucleotides that hybridize to nucleic acid sequences of SEQ ID NOs: 25-32; and

4) amplifying the cDNA and detecting the amplified products.

Embodiment 108. The method of embodiment of any of 99-107, wherein the oligonucleotides are chosen from oligonucleotides having the sequences of SEQ ID NOs: 13-24.

Embodiment 109. A method of identifying a subject suitable for treatment with a therapeutic agent that modulates type 1 interferon activity comprising detecting increased mRNA of at least four of IFI27, IFI44, IFI44L, IFI6, and RSAD2 in a sample of the subject, wherein the increased mRNA is calculated according to the following algorithm:

$$\Delta Ct_{IFN} = \frac{(Ct_{IFI44} - Ct_{REF}) + (Ct_{IFI44L} - Ct_{REF}) + (Ct_{IFI27} - Ct_{REF}) + (Ct_{RSAD2} - Ct_{REF})}{4};$$

wherein

$$\Delta Ct_{REF} = \frac{Ct_{ACTB} + Ct_{GAPDH} + Ct_{18S}}{3}$$

and wherein a $\Delta Ct_{IFN}$ of about 7.6 indicates a subject suitable for treatment with the agent.

Embodiment 110. The method of embodiment of any of 99-109, wherein the agent is chosen from an anti-interferon alpha antibody and anti-interferon alpha receptor antibody.

Embodiment 111. The method of embodiment of any of 99-110, wherein the anti-interferon antibody is sifalimumab.

Embodiment 112. The method of embodiment of any of 99-111, wherein the anti-interferon antibody is not sifalimumab.

Embodiment 113. The method of embodiment of any of 99-112, wherein detecting the mRNA of at least IFI27, IFI44, IFI44L, IFI6, and RSAD2 comprises:

1) isolating RNA from a sample obtained from the subject;
2) synthesizing cDNA from the RNA
3) hybridizing the cDNA with oligonucleotides that hybridize to nucleic acid sequences of SEQ ID NOs: 25-35, and
4) amplifying the cDNA and detecting the amplified products.

Embodiment 114. The method of embodiment of any of 99-113, wherein the oligonucleotides are chosen from oligonucleotides having the sequences of SEQ ID NOs: 1-24.

Embodiment 115. A method for treating a subject with a therapeutic agent that modulates type 1 interferon activity comprising:

a) identifying a subject suitable for treatment by detecting increased mRNA of at least four of IFI27, IFI44, IFI44L, IFI6, and RSAD2 in a sample of the subject, wherein an increase in mRNA of at least about 4 fold indicates a subject suitable for treatment; and
b) administering the therapeutic agent.

Embodiment 116. The method of embodiment of any of 99-115, wherein the increased mRNA is relative to the mRNA of at least four of IFI27, IFI44, IFI44L, IFI6, and RSAD2 in pooled samples from healthy patients.

Embodiment 117. The method of embodiment of any of 99-116, wherein the increased mRNA is relative to the mRNA of one or more control genes present in the sample.

Embodiment 118. The method of embodiment of any of 99-117, wherein the one or more control genes are chosen from ACTB, GAPDH, and 18S rRNA.

Embodiment 119. The method of embodiment of any of 99-118, wherein increased mRNA of IFI27, IFI44, IFI44L, and RSAD2 is detected.

Embodiment 120. The method of embodiment of any of 99-119, wherein the agent is chosen from an anti-interferon alpha antibody and anti-interferon alpha receptor antibody.

Embodiment 121. The method of embodiment of any of 99-120, wherein the anti-interferon antibody is sifalimumab.

Embodiment 122. The method of embodiment of any of 99-121, wherein the anti-interferon antibody is not sifalimumab.

Embodiment 123. The method of embodiment of any of 99-122 wherein detecting the mRNA of at least IFI27, IFI44, IFI44L, IFI6, and RSAD2 comprises

1) isolating RNA from a sample obtained from the subject;

2) synthesizing cDNA from the RNA;

3) hybridizing the cDNA with oligonucleotides that hybridize to nucleic acid sequences of SEQ ID NOs: 25-32, and

4) amplifying the cDNA and detecting the amplified products.

Embodiment 124. The method of embodiment of any of 99-123, wherein the oligonucleotides are chosen from oligonucleotides having the sequences of SEQ ID NOs: 13-24.

Embodiment 125. A method of identifying a subject suitable for treatment with a therapeutic agent that modulates type 1 interferon activity comprising

a) detecting increased mRNA of at least four of IFI27, IFI44, IFI44L, IFI6, and RSAD2 in a sample of the subject, wherein the increased mRNA is calculated according to the following algorithm:

$$\Delta Ct_{IFN} = \frac{(Ct_{IFI44} - Ct_{REF}) + (Ct_{IFI44L} - Ct_{REF}) + (Ct_{IFI27} - Ct_{REF}) + (Ct_{RSAD2} - Ct_{REF})}{4};$$

wherein

$$\Delta Ct_{REF} = \frac{Ct_{ACTB} + Ct_{GAPDH} + Ct_{18S}}{3}$$

and wherein a $\Delta Ct_{IFN}$ of about 7.6 indicates a subject suitable for treatment with a therapeutic agent that modulates IFN$\alpha$ activity; and
b) administering the therapeutic agent.

Embodiment 126. The method of embodiment of any of 99-125, wherein the agent is chosen from an anti-interferon alpha antibody and anti-interferon alpha receptor antibody.

Embodiment 127. The method of embodiment of any of 99-126, wherein the anti-interferon antibody is sifalimumab.

Embodiment 128. The method of embodiment of any of 99-127, wherein the anti-interferon antibody is not sifalimumab.

Embodiment 129. The method of embodiment of any of 99-128, wherein detecting the mRNA of at least IFI27, IFI44, IFI44L, IFI6, and RSAD2 comprises:

1) isolating RNA from a sample obtained from the subject;
2) synthesizing cDNA from the RNA;
3) hybridizing the cDNA with oligonucleotides that hybridize to nucleic acid sequences of SEQ ID NOs: 25-35, and
4) amplifying the cDNA and detecting the amplified products.

Embodiment 130. The method of embodiment of any of 99-129, wherein the oligonucleotides are chosen from oligonucleotides having the sequences of SEQ ID NOs: 1-24.

The set of examples that follow are provided for the purpose of illustration only and the disclosure should in no way be construed as being limited to these examples.

## EXAMPLES

## IX. Example 1: Development of a diagnostic assay for anti-IFN alpha therapeutics

### A. Background

Gene expression profiling was used to identify pharmacodynamic (PD) genes whose transcripts satisfy three selection criteria. Specifically, they are 1) inducible by type 1 IFN subtypes, 2) repressed in SLE patient sera by MEDI-545, and 3) over-expressed in SLE patients compared to normal healthy donors. Such genes include those in International Application No. PCT/US2007/024947, filed December 6, 2007, International Application No. PCT/US2008/062646, filed May 5, 2008, International Application No. PCT/US2009/033407, filed February 6, 2009 (which relates to miRNA marker profiles and type 1 IFN or IFN$\alpha$-induced PD marker profiles in inflammatory or autoimmune diseases) and International Application No. PCT/US2009/048028, filed June 19, 2009.

Prior studies allowed the identification of groups of genes than can be used as "signatures" of diseases, such as SLE. For example, we previously identified a 21-gene signature for SLE and other autoimmune diseases. *See* International Application No. PCT/US2007/024947, filed December 6, 2007. We wanted to determine whether a subset of the 21 genes could be reliably used to identify patients suffering from autoimmune diseases, such as SLE. Moreover, we wanted to determine whether a subset of the 21 genes could be used as a signature for whether a patient would respond to a therapetutic agent that modulates type 1 interferon, such as, for example, anti-interferon alpha antibodies or anti intereron receptor antibodies.

To determine whether a subset of the 21 genes could be used to identify patients, we analyzed gene expression data from patients involved in on-going clinical trials of MEDI-545. MEDI-545 was administered to SLE patients following standard clinical protocols. Clinical outcome was assessed using standard SLE evaluation methods such as the Safety of Estrogens in Lupus Erythematosus-Systemic Lupus Erythematosus Disease Activity Index (SELENA-SLEDAI), the British Isles Lupus Activity Group index, and the Physician Global Assessment (MDGA). Gene expression profiling of patient samples was conducted using Affymetrix human genome U133 plus 2.0 GeneChips® to identify candidate genes.

As discussed below, this analysis led to the identification of a set of diagnostic genes (e.g., 4 or 5 gene based assay) for the identification of patients (e.g., SLE or myositis patients) for treatment with an anti-IFN alpha or type I interferon therapeutic. Specifically, the diagnostic gene set was selected for its ability to predict response to sifalimumab (MEDI-545) in SLE patients. As a result, measurement of the expression of these four genes can be used to predict SLE patients who will or will not benefit from treatment with sifalimumab. As detailed below, expression of the four genes may be used in a variety of conditions and to identify patients suitable for treatment with therapies directed against those diseases. A group of five genes suitable for a diagnostic assay consists of IFI44, IFI44L, IFI27, RSAD2 and IFI6. Any 4 genes selected from this group may be suitable for a diagnostic assay. The group of genes for which analytical data is provided in Table 1 and Figures 1 and 2 consists of IFI27, IFI44, IFI44L, and RSAD2.

### B. Assay Principle

The diagnostic assay is based on quantitative Reverse Transcription-Polymerase Chain Reaction (qRT-PCR). The assay amplifies and detects transcripts originating from four target genes: IFI44, IFI44L, IFI27 and RSAD2. The diagnostic assay also amplifies and detects a set of endogenous RNA molecules as controls ("housekeeping genes"):

ACTB, GAPDH, and 18S rRNA. The transcript targets are amplified from total RNA samples obtained from whole blood samples collected from patients using the cleared PAXgene™ Blood RNA System (Qiagen, kit Cat # 762164; Becton Dickinson, collection tubes Cat # 762165; K042613). RNA is isolated using the procedures specified in the PAXgene™ Blood RNA kit. The target transcripts are amplified using two sequence-specific forward and reverse primers (unlabeled). Each amplified target is detected using a sequence-specific probe that is labeled with a fluorescent reporter moiety, FAM, and a fluorescence quenching moiety, BHQ1. Each target is amplified and detected in individual wells of a 96 well plate. A quantitative determination of the amount of each transcript in a sample is made based on detection of fluorescence on the Applied Biosystems 7500 Fast Dx Real-Time PCR Instrument, a Clinical Multiplex Test System (Applied Biosystems, Foster City, CA, Cat # 4406985; K082562) with SDS software version 1.4.

The quantitative measurement results in the determination of a Cycle Threshold (Ct) value for each target that corresponds to the relative abundance of the transcript in the RNA sample. The Ct is determined by measuring the geometric increase in the fluorescence signal that results from the release of reporter-quencher proximity by the 5'-3' exonuclease activity of the polymerase during the elongation phase of each amplification cycle. The quantitative Ct values for each gene and control are used to calculate the level of overexpression of target genes in subject suffering from autoimmune diseases relative to healthy subjects. The level of overexpression may be expressed as a mean fold change in mRNA level of the target genes in a subject suffering from an autoimmune disease relative to the mean level of the genes in a healthy subject of or it may be expressed as a score of overexpression of the Type I Interferon-inducible genes.

Specifically, the score ($\Delta Ct_{IFN}$) ("delta Ct") is calculated as the difference between the mean Ct for the four target genes and the mean Ct of the three control genes according to the following algorithm:

$$\Delta Ct_{IFN} = \frac{(Ct_{IFI44} - Ct_{REF}) + (Ct_{IFI44L} - Ct_{REF}) + (Ct_{IFI27} - Ct_{REF}) + (Ct_{RSAD2} - Ct_{REF})}{4};$$

; where

$$\Delta Ct_{REF} = \frac{Ct_{ACTB} + Ct_{GAPDH} + Ct_{18S}}{3}$$

This score is then used to determine a qualitative test result in which a score greater than or equal to a cutoff is a positive test result and a score less than a cutoff is a negative test result. A negative result indicates that a patient is not likely to respond to sifalimumab treatment (Non-Responder) and a positive result indicates that a patient is likely to respond to sifalimumab treatment (Responder).

**C. Assay Steps**

**1. RNA Preparation**

Total RNA is prepared from whole blood, using the cleared PAXgene™ Blood RNA System (Qiagen, kit Cat # 762164; Becton Dickinson, collection tubes Cat # 762165; K042613) and provides the template for cDNA synthesis. RNA yield and quality is tested by spectrophotometry by measuring the absorbance at wavelengths of 260nm and 280nm. RNA yield is calculated using the formula C (mg/ml) = A280/0.025. In addition, the purity of RNA is evaluated using the ratio of the absorbance at 260 nm and 280 nm. The acceptable range of A260/A280 ratio is ≥ 1.6.

**2. cDNA Synthesis and Target Amplification**

cDNA is synthesized from purified total RNA using the liquid RT Enzyme Mix and the RT Buffer containing the RT primers and nucleotides. Synthesis of cDNA uses a random priming approach using RT primers that are a mixture of random hexadecamers that hybridize to the RNA molecules and serve as a substrate for the RT Enzymes. The resulting cDNA contains a proportional mixture of DNA representing the sequences present in the RNA sample.

The target transcripts are simultaneously amplified and detected using the qPCR buffer, the gene primer and probes and clinical grade AmpliTaq Gold polymerase. During each amplification cycle, two sequence-specific forward and reverse primers (unlabeled) hybridize to complementary cDNA templates during the annealing phase and serve as a substrate for the AmpliTaq Gold polymerase during the elongation phase, resulting in the production of a new DNA strand complementary to the target. The forward and reverse primers each hybridize to a different sense or antisense strand. Detection of each amplified target is accomplished using a sequence-specific probe that is labeled with a fluorescent reporter moiety, FAM, and a fluorescence quenching moiety, BHQ 1, that hybridizes to the complementary target sequence during the annealing phase. Each target is amplified and detected in individual wells of a 96 well plate.

| Thermal Cycling Protocol | |
|---|---|
| 37C for 15 min | |
| 95C for 10 min | |
| 95C for 15 sec | 50 cycles |
| 60C for 1 min | |
| 7500 thermal cycler run mode: Standard | |

### 3. Signal Detection

A quantitative determination of the amount of each transcript in a sample is made based on detection of fluorescence on the Applied Biosystems 7500 Fast Dx Real-Time PCR Instrument (Applied Biosystems, Foster City, CA, Cat # 4406985; K082562) with SDS software version 1.4, which results in the determination of a Cycle Threshold (Ct) value for each target that corresponds to the relative abundance of the transcript in the RNA sample. The instrument determines the Ct by measuring the geometric increase in the fluorescence signal that results from the release of reporter-quencher proximity by the 5'-3' exonuclease activity of the polymerase during the elongation phase of each amplification cycle. The quantitative Ct values for each gene and control are used to calculate a score of overexpression of the Type I Interferon-inducible genes.

### 4. Assay Controls

In vitro Transcript (IVT) RNA controls (each of the four target genes and the housekeeping gene controls) optionally included with each run are designed to detect potential user error, contamination, cross-reactions and/or assay failure during the Reverse Transcription, cDNA synthesis, PCR, hybridization, and/or detection steps. The control results are used as validity controls to validate or invalidate a given run. They are not used to validate the RNA isolation, which is performed with the cleared PAXgene™ Blood RNA System and controlled by a RNA quality check. A Negative and a low Positive control, close to the cutoff, may be included provided.

### 5. Software

Software is used to calculate the delta Ct using the equation described above. The delta Ct is then used to determine a qualitative test result in which a score greater than or equal to a cutoff is a positive test result and a score less than the cutoff is a negative test result. A negative result indicates that a patient is not likely to respond to sifalimumab treatment (Non-Responder) and a positive result indicates that a patient is likely to respond to sifalimumab treatment (Responder).

### 6. Assay Kit Components

| Usage | Components |
|---|---|
| RT Reagents | RT Buffer |
| | RT Enzyme mix |
| | Diluent |

(continued)

| Usage | Components |
|---|---|
| DNA Amplification Reagents | qPCR buffer |
| | Oligonucleotide primers and probes for IFI44, IFI44L, IFI27 RSAD2 and Oligonucleotide primers and probes for Three Control Genes (18S, ACTB, and GAPDH) |
| | AmpliTaq Gold Polymerase |
| Controls | Positive Control |
| | Negative Control |
| | NTC |

**7. Oligonucleotides for simultaneously amplifying and detecting target genes**

| Oligo Name | Target | SEQ ID NO: | Sequence |
|---|---|---|---|
| MEDI_0001-F | 18S | SEQ ID NO: 1 | GCTACCACATCCAAGGAAGG |
| MEDI_0001-P | | SEQ ID NO: 2 | CGCAAATTACCCACTCCCGACCC |
| MEDI_0001-R | | SEQ ID NO: 3 | GCCTCGAAAGAGTCCTGTATTG |
| MEDI_0002-F | ACTB | SEQ ID NO: 4 | ACAGAGCCTCGCCTTTG |
| MEDI_0002-P | | SEQ ID NO: 5 | AGCTGGCGGCGGGTGTGG |
| MEDI_0002-R | | SEQ ID NO: 6 | CCTTGCACATGCCGGAG |
| MEDI_0003_A-F | GAPDH | SEQ ID NO: 7 | ACATCGCTCAGACACCATG |
| MEDI_0003_A-P | | SEQ ID NO: 8 | CCGTTGACTCCGACCTTCACCTT |
| MEDI_0003_A-R | | SEQ ID NO: 9 | ACCAGAGTTAAAAGCAGCCC |
| MEDI_0003_B-F | GAPDH | SEQ ID NO: 10 | TGGGTGTGAACCATGAGAAGTATG |
| MEDI 0003 B-P | | SEQ ID NO: 11 | CCTCAAGATCATCAGCAATGCCTCCTGCA |
| MEDI 0003 B-R | | SEQ ID NO: 12 | CAGGGGTGCTAAGCAGTTGG |
| MEDI_0004-F | IFI27 | SEQ ID NO: 13 | CTCTCACCTCATCAGCAGTG |
| MEDI_0004-P | | SEQ ID NO: 14 | CCAGAGGCCACCCTGACCAC |
| MEDI_0004-R | | SEQ ID NO: 15 | TCACAACTGTAGCAATCCTGG |
| MEDI_0005-F | IFI44 | SEQ ID NO: 16 | GATGCGAAGATTCACTGGATG |
| MEDI_0005-P | | SEQ ID NO: 17 | AGTTCTCAAGGCAGACAGTAAGCTCTTC |

(continued)

| Oligo Name | Target | SEQ ID NO: | Sequence |
|---|---|---|---|
| MEDI_0005-R | | SEQ ID NO: 18 | TGTTGAACCAGGGATCCATATG |
| MEDI_0006-F | IFI44L | SEQ ID NO: 19 | AAGCCGTAGTGGGGTCT |
| MEDI_0006-P | | SEQ ID NO: 20 | TATACCGCTCGGTTATGCTGGTG |
| MEDI-0006-R | | SEQ ID NO: 21 | AACATAAATGGCAGAGATTTTCCA |
| MEDI_0007-F | RSAD2 | SEQ ID NO: 22 | AAAGACTCCTACCTTATTCTGGATG |
| MEDI_0007-P | | SEQ ID NO: 23 | CTGAACTGTAGAAAGGGACGGAAGGAC |
| MEDI_0007-R | | SEQ ID NO: 24 | CTTCTACACCAACATCCAGGA |

8. **Sequence of Target genes**

| Target Name | SEQ ID NO |
|---|---|
| IFI27 variant 1 | SEQ ID NO: 25 |
| IFI27 variant 2 | SEQ ID NO: 26 |
| IFI44 | SEQ ID NO: 27 |
| IFI44L | SEQ ID NO: 28 |
| IFI6 variant 1 | SEQ ID NO: 29 |
| IFI6 variant 2 | SEQ ID NO: 30 |
| IFI6 variant 3 | SEQ ID NO: 31 |
| RSAD2 | SEQ ID NO: 32 |
| 18S | SEQ ID NO: 33 |
| ACTB | SEQ ID NO: 34 |
| GAPDH | SEQ ID NO: 35 |

**X. Example 2: A variety of analyses demonstrate that response to MEDI-545 correlates with the four gene signature**

The threshold value used to designate a diagnostic positive versus negative subject was determined using two primary methods. First, the distribution of the four gene diagnostic fold change score from 202 SLE subjects was evaluated for the presence of modes beyond a single mode. Such a multi-mode distribution would imply the presence of more than one score population. From this distribution, two distinct modes were identified and the centroid of the region that discriminated these two modes was found at a value of approximately 4. The distribution shows that a range about to 2 to about 8 may be used to discriminate the two modes and thus a suitable mean fold change cutoff may be between about 2 and about 8.

Second, the four gene diagnostic fold change score distribution of 24 normal healthy donors was evaluated for upper limits in comparison to the SLE score distribution. The average diagnostic fold change score from the 24 normal healthy donors is 1.34 with the upper bound (mean+2*SD) of 2.91. Since the subject count in the normal healthy donor population was much smaller than the subject counts in our disease population, we used a conservative estimate of the upper bound diagnostic score in the normal healthy donors that agreed with the results from the bimodal SLE score distribution. As such, a cut point of ≥ 4 was selected for stratifying SLE patients in diagnostic positive and diagnostic negative groups.

For the diagnostic positive population, 147 SLE subjects were stratified into this group and the mean, median, and standard deviation for the scores are 36.13, 63.76, and 2.71, respectively. For the diagnostic negative population, 55 SLE subjects were stratified in this group and mean, median, and standard deviation for the scores are 0.95, 0.89, and 2.00, respectively. A two-sample two-tailed Welch's modified t-test indicates a significant difference between the two groups at a p-value of $1.62 \times 10^{-66}$.

Receiver Operator Characteristic (ROC) curves using SLEDAI clinical endpoint were generated using the four gene signature (i.e. IFI27, IFI44, IFI44L, and RSAD2 signature) obtained from the clinical trial data based on the fold-change calculation. The curve is shown in Figure 1 using a SLEDAI drop of at least 4 points from baseline as the endpoint, evaluated at days 182, 196, and 210 post-treatment. Based on the ROC curve, a cutoff point of mean fold change of ≥ 4 in expression for IFI27, IFI44, IFI44L, and RSAD2 was selected.

With a cutoff point of mean fold change ≥ 4 the sensitivity and specificity of the test is 87.0% and 32.9%, respectively, based on data obtained from blood samples collected on day 182 post treatment. The AUC for day 182 data is 0.59. With a cut point of mean fold change ≥ 4 the sensitivity and specificity of the test is 85.7% and 32.8%,, respectively, based on data obtained from blood samples collected on day 196 post treatment. The AUC for day 196 data is 0.57. Finally, when using the same cutoff point, the sensitivity, specificity, and AUC values obtained at day 210 post treatment are 86.0%, 33.8%, and 0.56, respectively.

When using the mean of overexpression shown as a fold-change for each of the four genes as a classifier to partition SLE patients into Diagnostic Positive (Responder) versus Diagnostic Negative (Non-Responder) status, there is a clear separation between groups. The distribution of the four gene mean scores on a log2 scale clearly exhibits two apparent modes, with few values falling between them. Figures 2A and B show the distribution of patients into diagnostic test positive and negative groups using the four gene (IFI27, IFI44, IFI44L, and RSAD2) diagnostic with a cut off of mean fold change of≥4.

### A. SLENA-SLEDAI ≥ 4

We pooled all sifalimumab dose groups across MI-CP 152, and used the clinical endpoint of reduction in SELENA-SLEDAI ≥ 4 points from baseline (SELENA-SLEDAI Responder), to calculate at Day 182 a predictive value positive of 46.6% (PPV; percentage of subject Responders among Diagnostic-Positive subjects [Dx+]). Similarly, we calculated a predictive value negative at Day 182 of 82.1% (NPV; percentage of subject Non-Responders among Diagnostic-Negative subjects [Dx-]). For this same clinical endpoint, the sensitivity and specificity values were 87.0% and 32.9%.

Because we observed a placebo response rate of approximately 20% to 40%, we found it appropriate to calculate the "delta PPV" and "delta NPV," to factor in the placebo effect on the accuracy rates. The delta PPV value is the difference between the PPV for sifalimumab-treated subjects (based on mostly 0.3 and 1.0 mg/kg data available at the time of the interim analysis) and the PPV for placebo-treated subjects. Similarly, for the delta NPV, this calculation consisted of the difference between the NPV for sifalimumab-treated subjects (again mostly 0.3 and 1.0 mg/kg group data) and the NPV for placebo-treated subjects. These delta PPV/NPV statistics are conservative estimates of the PPV and NPV and demonstrate the percentage of responding patients given drug, in the context of those patients that respond given placebo. As such, these statistics show the inherent 'noise' level in responder status. Even with these adjusted PPV/NPV values, however, we still see a strong response rate for Dx positive patients treated with Sifalimumab as compared to Dx negative patients treated with Sifalimumab.

A summary of results is shown in Table 1.

**Table 1. Response rates stratified by diagnostic status, reported at days 182, 196, and 210 post treatment**

| Treatment Group | SELENA-SLEDAI | | | | | | |
|---|---|---|---|---|---|---|---|
| | DX Status | Responders at day 182 | Day 182 95% CI | Responders at day 196 | Day 196 95% CI | Responders at day 210 | Day 210 95% CI |
| Sifalimumab | Positive | 46.6% (88) | 36.5%-56.9% | 48.9% (88) | 38.7%-59.1 % | 50.6% (87) | 40.3%-60.8% |
| | Negative | 17.9% (28) | 7.9%-35.6% | 21.4% (28) | 10.2%-39.5% | 21.4% (28) | 10.2%-39.5% |
| Placebo | Positive | 32.1% (28) | 17.9%-50.7% | 39.3% (28) | 23.6%-57.6% | 37.0% (27) | 21.5%-55.8% |
| | Negative | 11.1% (9) | 2.0%-43.5% | 22.2%(19) | 6.3%-54.7% | 11.1% (9) | 2.0%-43.5% |
| Sifalimumab - Placebo | Positive | 14.4% | -5.7%-34.6% | 9.6% | 11.3%-30.5% | 13.5% | -7.5%-34.6% |
| | Negative | 6.7% | -36%-34.8% | -0.8% | 31.9%-30.3% | 10.3% | 15.2%-35.9% |
| CI = confidence interval; DX = diagnostic | | | | | | | |

Figures 3A and B show data from a phase 1b, multicenter, randomized, double-blinded, placebo-controlled, dose-escalation study to evaluate multiple intravenous doses of MEDI-545 in patients with moderately to severely active SLE. All SLE subjects have SLEDAI score ≥ 6 at prescreening. Figures 3A and B shows the time adjusted area under the curve minus baseline SLEDAI score in four gene (IFI27, IFI44, IFI44L, and RSAD2) signature positive or negative SLE patients in placebo, or 0.3/1/3/10 mg/kg of MEDI-545 cohorts. Figure 3A signature positive, Figure 3B signature negative.

There are no major correlation patterns between the four gene diagnostic (i.e. IFI27, IFI44, IFI44L, and RSAD2 signature) and the primary demographic/clinical variables of the subjects in the clinical trials. Pearson's correlation coefficients are provided in Table 2 for these primary variables.

**Table 2. Pearson's correlation coefficients for the four gene signature (i.e. IFI27, IFI44, IFI44L, and RSAD2 signature) and the primary demographic/clinical variables. Day 182 and day 196 represents gene expression patterns obtained from blood collected on day 182 and 196, respectively, following MEDI545 administration.**

| Demographic/Clinical variables | Day 182 | Day 196 |
|---|---|---|
| Age | -0.04 | -0.04 |
| Weight | 0.11 | 0.07 |
| Sex | 0.11 | 0.10 |
| Race | -0.09 | -0.09 |
| Ethnicity | -0.14 | -0.14 |
| Country | -0.11 | -0.11 |
| Baseline steroid use | 0.17 | 0.13 |

**B. Positive Correlation in SELDAI responses, reduction (improvement)≥ 4 points.**

Data are from trial CP152. Subjects with moderately to severely active SLE were stratified by screening type I IFN signature and then exposed to placebo or sifalimumab from days 0 - 182. A SLEDAI response is shown, which is SLEDAI reduction (improvement in disease activity) ≥ 4 points. Solid squares/line represent sifalimumab exposed subjects and open squares/dotted lines represent placebo patients. Short lines on days 182-210 only are average values for days 182-210, with no symbols, solid line representing average value for sifalimumab subjects and no symbols, dotted lines representing average values for placebo subjects. Subjects who received at least one dose of placebo or sifalimumab and who had a baseline SLEDAI score ≥ 6 are included, with data shown for subjects receiving 1, 3, or 10 mg/kg sifalimumab or placebo. Subjects who required rescue corticosteroids at levels greater than that allowed in the protocol for an increase in disease activity on or before day 196 are considered non-responders in this analysis.

Figure 4A includes subjects with a positive diagnostic test at screening, with average SLEDAI response (%) on days 182-210 52.5% for sifalimumab subjects (n = 88) and 33.9% for placebo subjects (n = 22). Figure 4B includes subjects with a negative diagnostic test at screening, with average composite response on days 182-210 25.7% for sifalimumab subjects (n = 22) and 22.2% for placebo subjects (n = 6).

**C. Positive correlation in reduction of signature and SLEDAI response. SLEDAI responses, reduction (improvement) ≥ 4 points, in Dx+ subjects with ≥ 50% reduction vs. < 50% reduction in Dx post baseline**

Figures 5A and B. Data are from trial CP152. Subjects with moderately to severely active SLE were stratified by screening type I IFN signature and then exposed to placebo or sifalimumab from days 0 - 182. A SLEDAI response is shown, which is SLEDAI reduction (improvement in disease activity) > 4 points. Solid squares/line represent sifalimumab exposed subjects and open squares/dotted lines represent placebo patients. Short lines on days 182-210 only are average values for days 182-210, with no symbols, solid line representing average value for sifalimumab subjects and no symbols, dotted lines representing average values for placebo subjects. Subjects who received at least one dose of placebo or sifalimumab and a baseline SLEDAI score > 6 are included, with data shown for subjects receiving 1, 3, or 10 mg/kg sifalimumab or placebo. Subjects who required rescue corticosteroids at levels greater than that allowed in the protocol for an increase in disease activity on or before day 196 are considered non-responders in this analysis.

Figure 5A includes subjects with a positive diagnostic test at screening and, if sifalimumab treated, had a > 50% median reduction in baseline diagnostic signature score post dosing days 28-210, with average SLEDAI response (%) on days 182-210 66.3% for sifalimumab subjects (n = 23) and 33.9% for placebo subjects (n = 22). Figure B includes subjects with a positive diagnostic test at screening, and, if sifalimumab treated, had a < 50% median reduction in baseline diagnostic signature score post dosing days 28-210, with average SLEDAI response on days 182-210 48.0% for sifalimumab subjects (n = 23) and 33.9% for placebo subjects (n = 22).

**D. Positive Correlation in Composite Response and Signature**

As shown in Figures 6A and B, the composite response of subjects correlates with a positive four gene signature score. Data are from trial CP 152. Subjects with moderately to severely active SLE were stratified by screening type I IFN signature and then exposed to placebo or sifalimumab at doses of 0.3, 1, 3, or 10 mg/kg IV every 2 weeks, from days 0 - 182. A composite response is shown, which is SLEDAI reduction (improvement) ≥ 4 points + no more than 1 new BILAG B score (a measure of at least moderate flare) + no worsening in physician global assessment > 0.3 inches on 3 inch visual analogue scale. Solid squares/line represent sifalimumab exposed subjects and open squares/dotted lines represent placebo patients. Short lines on days 182-210 only are average values for days 182-210, with no symbols, solid line representing average value for sifalimumab subjects and no symbols, dotted lines representing average values for placebo subjects. Subjects who received at least one dose of placebo or sifalimumab and who had a baseline SLEDAI score ≥ 6 are included. Subjects who required rescue corticosteroids at levels greater than that allowed in the protocol for an increase in disease activity on or before day 196 are considered non-responders in this analysis. Figure 6A includes subjects with a positive diagnostic test at screening, with average composite response (%) on days 182-210 46.4% for sifalimumab subjects (n = 88) and 36.1% for placebo subjects (n = 29). Figure 6B includes subjects with a negative diagnostic test at screening, with average composite response on days 182-210 19.1% for sifalimumab subjects (n = 28) and 14.8% for placebo subjects (n = 9).

**E. Positive Correlation in SLEDAI area under the curve minus baseline**

As shown in Figures 7A and B, reduction in SLEDAI area under the curve minus baseline correlates with a positive four gene signature score. Data are from trial CP152. Subjects with moderately to severely active SLE were stratified by baseline type I IFN signature and then exposed to placebo or sifalimumab at doses of 0.3, 1, 3, or 10 mg/kg IV every 2 weeks, from days 0 - 182. The area under the curve minus baseline for SLEDAI score (a measure of disease activity) is shown, with greater negative values indicating greater reduction in disease activity. Solid squares/line represent sifalimumab exposed subjects and open squares/dotted lines represent placebo patients. Short lines on days 182-210 only are average values for days 182-210, with no symbols, solid line representing average value for sifalimumab subjects and no symbols, dotted lines representing average values for placebo subjects. Subjects who received at least one dose of placebo or sifalimumab are included. Figure 7A includes subjects with a positive diagnostic test at screening, with average SLEDAI area under the curve minus baseline on days 182-210 of -2.34 for sifalimumab subjects (n = 92) and -1.14 for placebo subjects (n = 30). Figure 7B includes subjects with a negative diagnostic test at screening, with average SLEDAI area under the curve minus baseline on days 182-210 of -0.46 for sifalimumab subjects (n = 29) and -0.88 for placebo subjects (n = 10).

**F. Positive Correlation in SLEDAI change from baseline and signature**

As shown in Figures 8A and B reduction in SLEDAI from baseline correlated with a positive four gene signature score. Data are from trial CP152. Subjects with moderately to severely active SLE were stratified by screening type I IFN signature and then exposed to placebo or sifalimumab at doses of 0.3, 1, 3, or 10 mg/kg IV every 2 weeks, from days 0 - 182. A SLEDAI response is shown, which is SLEDAI reduction (improvement in disease activity) ≥ 4 points, in subjects with baseline SLEDAI ≥ 6. Solid squares/line represent sifalimumab exposed subjects and open squares/dotted lines represent placebo patients. Short lines on days 168-210 only are average values for days 168-210, with no symbols, solid line representing average value for sifalimumab subjects and no symbols, dotted lines representing average values for placebo subjects. Subjects who received at least one dose of placebo or sifalimumab. Subjects who required rescue corticosteroids at levels greater than that allowed in the protocol for an increase in disease activity on or before day 196 are considered non-responders in this analysis. Subjects with a positive diagnostic test at baseline and with median reduction in type I IFN signature of ≥ 50% post dosing from days 28-210 are shown. Figure 8A includes subjects who received sifalimumab at 1 mg/kg IV with average SLEDAI response days 168-210 of 53% for sifalimumab subjects (n = 10) and 21% for placebo subjects from the same cohort (n = 7). Figure 8B includes subjects who received sifalimumab at 3 mg/kg IV with average SLEDAI response days 168-210 of 63% for sifalimumab subjects (n = 6) and 33% for placebo subjects from the same cohort (n = 6).

**G. Inhibition of signature, SLEDAI response, and dose**

Figures 9 A-C. Data are from trial CP152, Subjects with moderately to severely active SLE were stratified by screening type I IFN signature and then exposed to placebo or sifalimumab at doses of 0.3, 1, 3, or 10 mg/kg IV every 2 weeks, from days 0 - 182. A SLEDAI response is shown, which is SLEDAI reduction (improvement in disease activity) ≥ 4 points, in subjects with baseline SLEDAI ≥ 6. Solid squares/line represent sifalimumab exposed subjects and open squares/dotted lines represent placebo patients. Short lines on days 168-210 only are average values for days 168-210, with no symbols, solid line representing average value for sifalimumab subjects and no symbols, dotted lines representing average values for placebo subjects. Subjects who received at least one dose of placebo or sifalimumab. Subjects who required rescue corticosteroids at levels greater than that allowed in the protocol for an increase in disease activity on or before day 196 are considered non-responders in this analysis. Subjects with a positive diagnostic test at baseline and with median reduction in type I IFN signature of ≥ 50% post dosing from days 28-210 are shown. Figure 9A includes subjects who received sifalimumab at 1 mg/kg IV with average SLEDAI response days 168-210 of 53% for sifalimumab subjects (n = 10) and 21% for placebo subjects from the same cohort (n = 7). Figure 9B includes subjects who received sifalimumab at 3 mg/kg IV with average SLEDAI response days 168-210 of 63% for sifalimumab subjects (n = 6) and 33% for placebo subjects from the same cohort (n = 6). Figure 9C includes subjects who received sifalimumab at 10 mg/kg IV with average SLEDAI response days 168-210 of 75% for sifalimumab subjects (n = 9) and 45% for placebo subjects from the same cohort (n = 9).

Data are from trial CP152. Subjects with moderately to severely active SLE were stratified by screening type I IFN signature and then exposed to placebo or sifalimumab at doses of 0.3, 1, 3, or 10 mg/kg IV every 2 weeks, from days 0 - 182. A SLEDAI response is shown, which is SLEDAI reduction (improvement in disease activity) ≥ 4 points, in subjects with baseline SLEDAI ≥ 6. Solid squares/line represent sifalimumab exposed subjects and open squares/dotted lines represent placebo patients. Short lines on days 168-210 only are average values for days 168-210, with no symbols, solid line representing average value for sifalimumab subjects and no symbols, dotted lines representing average values for placebo subjects. Subjects who received at least one dose of placebo or sifalimumab. Subjects who required rescue corticosteroids at levels greater than that allowed in the protocol for an increase in disease activity on or before day 196 are considered non-responders in this analysis. Subjects with a positive diagnostic test at baseline and with median reduction in type I IFN signature of < 50% post dosing from days 28-210 are shown. Figure 10A includes subjects who received sifalimumab at 1 mg/kg IV with average SLEDAI response days 168-210 of 37% for sifalimumab subjects (n = 9) and 21% for placebo subjects from the same cohort (n = 7). Figure 10B includes subjects who received sifalimumab at 3 mg/kg IV with average SLEDAI response days 168-210 of 42% for sifalimumab subjects (n = 12) and 33% for placebo subjects from the same cohort (n = 6). Figure 10C includes subjects who received sifalimumab at 10 mg/kg IV with average SLEDAI response days 168-210 of 45% for sifalimumab subjects (n = 15) and 45% for placebo subjects from the same cohort (n = 9).

### H. Delta Ct relative to Fold-change relative to healthy donors

In the initial studies, we calculated fold changes using a pool of 24 normal healthy donors as a reference for each SLE patient. We have found, however, that this reference may have some confounding issues, specifically: 1) it can potentially introduce variance into the assay, and 2) manufacturing an artificial reference with the same dynamic range consistently is burdensome.

We conducted analyses to evaluate the need for this normal reference and found that when comparing delta Ct values to fold change values, there is greater than 99% correlation between the two (both $\log_2$ transformed). Therefore, it is possible to use the delta Ct value with the house keeping genes from the SLE patient samples as the reference, and there is no need to measure or compare to healthy donors. This approach can be enhanced where the primary output of the diagnostic test is a qualitative result of positive or negative, as opposed to a quantitative score. Our results show that a cutoff of ≥4 on the fold change scale translates to a cutoff of ≥7.6 on the delta Ct scale.

## XI. Example 3. Relationship between Disease Activity and Type 1 Interferon- and Other Cytokine-Inducible Gene Expression in Blood in Dermatomyositis and

## Polymyositis

### A. Background

Peripheral blood of 42 patients with DM or PM was subjected to gene expression profiling using Affymetrix human genome U133 plus 2.0 GeneChips® in an initial study to identify the prevalence of patients exhibiting periphery overexpression of type 1 IFN-inducible genes. To gain further scientific insight on type 1 IFN as a potential therapeutic target for DM and PM, 24 patients with DM or PM were then prospectively enrolled and followed for up to 6 years (mean of 1.9 years) while receiving standard clinical care.

**B. Methodology**

Clinical courses including MITAX (Myositis Intention to Treat Activity Index) scoring of disease activity were assessed across 150 patient visits. This index is based on 6 organs or systems that the physician uses, for each organ or system, which he/she would treat with large doses of steroids and/or immunosuppressive drugs. Peripheral blood samples collected at 80 patient visits were used for microarray analysis of cytokine-induced gene expression for type 1 IFN, TNF-α, IL-1β, GM-CSF, IL-10, and IL-13 signaling pathways.

**C. Results**

35 of 42 (87%) DM and PM patients had moderate/strong overexpression of type 1 IFN-inducible genes in the periphery blood. In the longitudinal study during the course of treatment, 21 of 24 patients showed overexpression of a type 1 IFN-inducible gene signature in peripheral blood. Specifically, overexpression of IFI27, IFI44, IFI44L, and RSAD2 and a type 1 IFN-inducible 13-gene composite signature (IFI27, RSAD2, IFI44L, ISG15, OAS3, HERC5, MX1, ESPTI1, IFIT3, IFI44, OAS1, IFIT1, and IFI6) correlated highly with disease activity during treatment. For 3 patients, type 1 IFN-inducible gene overexpression during treatment preceded disease relapse within approximately 1 month TNF-α, IL-1β, GM-CSF, IL-10, and IL-13 inducible gene signatures were also overexpressed in DM and PM patients but were not correlated with disease activity.

Targeting type 1 IFN may provide clinical benefit in DM and PM patient populations with overexpression of type 1 IFN-inducible genes in the periphery. Type 1 IFN-inducible gene overexpression in the periphery blood merits further study for use as a pharmacodynamic and predictive biomarker for developing anti-type 1 IFN therapy for these patients. Specifically, the results described above with SLE indicate that the four gene diagnostic will permit identification of DM and PM patients who will respond to anti-interferon alpha therapy.

**XII. Example 4: Use of four or five gene signature to evaluate the presence and magnitude of overexpression of type 1 IFN-inducible genes in whole blood from patients suffering from SLE, DM, PM, SSc, and RA**

**A. Background**

We undertook studies to determine if there is a commonality of activation of the type I interferon (IFN) pathway in subjects with systemic lupus erythematosus (SLE), dermatomyositis (DM), polymyositis (PM), rheumatoid arthritis (RA), and systemic scleroderma (SSc).

We identified over-expressed transcripts in the whole blood (WB) of 262 SLE, 44 DM, 33 PM, 38 SSc and 89 RA subjects and compared expression to 24 healthy subjects using Affymetrix U133 Plus 2.0 Genechip®. Activation of the type I IFN pathway in WB was evaluated individually for each subject using the five gene type I IFN signature (IFI27, IFI44, IFI44L, IFI6, and RSAD2), as well as in lesional skin from 16 SLE, and 22 SSc subjects, muscle biopsies from 37 DM and 36 PM subjects, and synovium tissue from 20 RA subjects. Other cytokine gene signatures such as TNF-α, IL1β, IL-10, IL-13, IL-17, and GM-CSF were also assessed for pathway activation in the WB of these subjects. Additionally, a molecular classification of disease and healthy subjects was conducted with a clustering method using expression profiles of both type I IFN-inducible and non-type I IFN-inducible transcripts.

This 5 gene panel used to assess the activation of the type I IFN pathway in subjects was identified from the set of 21 type I IFN-inducible genes used to measure the pharmacodynamics of an anti-IFN-α mAb in subjects with DM, PM, and SLE, as described in Yao et al., 2009. The 5 genes were found to be the most over-expressed among these 21 genes in subjects with SLE, DM, PM, SSc, RA, and Sjogren's disease compared to normal healthy donors (Table 3). The genes in Table 3 are sorted in descending for each block of 21 genes (within each individual disease), to illustrate the ranking of the 5 genes among the 21 genes.

**Table 3 Gene expression in SLE, DM, PM, SSc, RA**

| Probe | Gene | Symbol | p-value | fold change | prevalence | disease |
|---|---|---|---|---|---|---|
| 202411_at | interferon, alpha-inducible protein 27 | IFI27 | 0 | 30.065 | 0.767 | SLE |
| 204415_at | interferon, alpha-inducible protein 6 | IFI6 | 0 | 16.465 | 0.882 | SLE |
| 214059_at | Interferon-induced protein 44 | IFI44 | 0 | 13.855 | 0.779 | SLE |

(continued)

| Probe | Gene | Symbol | p-value | fold change | prevalence | disease |
|---|---|---|---|---|---|---|
| 213797_at | radical S-adenosyl methionine domain containing 2 | RSAD2 | 0 | 13.232 | 0.763 | SLE |
| 204439_at | interferon-induced protein 44-like | IFI44L | 0 | 10.532 | 0.748 | SLE |
| 219211_at | ubiquitin specific peptidase 18 /// similar to ubiquitin specific peptidase 18 /// similar to ubiquitin specific peptidase 18 /// similar to ubiquitin specific peptidase 18 | USP18 | 0 | 7.271 | 0.679 | SLE |
| 44673_at | sialic acid binding Ig-like lectin 1, sialoadhesin | SIGLEC1 | 0 | 6.348 | 0.656 | SLE |
| 202145_at | lymphocyte antigen 6 complex, locus E | LY6E | 0 | 6.186 | 0.683 | SLE |
| 202869_at | 2',5'-oligoadenylate synthetase 1, 40/46kDa | OAS1 | 0 | 6.181 | 0.729 | SLE |
| 205569_at | lysosomal-associated membrane protein 3 | LAMP3 | 0 | 5.189 | 0.725 | SLE |
| 218400_at | 3'-5'-oligoadenylate synthetase 3, 100kDa | OAS3 | 0 | 5.053 | 0.706 | SLE |
| 219863_at | hect domain and RLD 5 | HERC5 | 0 | 4.841 | 0.729 | SLE |
| 203153_at | interferon-induced protein with tetratricopeptide repeats 1 /// interferon-induced protein with tetratricopeptide repeats 1 | IFIT1 | 0 | 4.840 | 0.729 | SLE |
| 205483_s_at | ISG15 ubiquitin-like modifier | ISG15 | 0 | 4.633 | 0.702 | SLE |
| 227609_at | epithelial stromal interaction 1 (breast) | EPSTI1 | 0 | 4.519 | 0.706 | SLE |

(continued)

| Probe | Gene | Symbol | p-value | fold change | prevalence | disease |
|---|---|---|---|---|---|---|
| 202086_at | myxovirus (influenza virus) resistance 1, interferon-inducible protein p78 (mouse) /// myxovirus (influenza virus) resistance 1, interferon-inducible protein p78 (mouse) | MX1 | 0 | 4.142 | 0.725 | SLE |
| 204972_at | 2'-5'-oligoadenylate synthetase 2.69/71kDa | OAS2 | 0 | 4.094 | 0.683 | SLE |
| 204747_at | interferon-induced protein with tetratricopeptide repeats 3 | IFIT3 | 0 | 3.992 | 0.702 | SLE |
| 219684_at | receptor (chemosensory) transporter protein 4 | RTP4 | 0 | 3.928 | 0.649 | SLE |
| 241916_at | Phospholipid scramblase 1 | PLSCR1 | 0 | 3.297 | 0.687 | SLE |
| 241812_at | DNA polymerase-transactivated protein 6 | DNAPTP6 | 0 | 3.217 | 0.561 | SLE |
| 204415_at1 | interferon, alpha-inducible protein 6 | IFI6 | 0 | 12.253 | 0.932 | DM |
| 214059_at1 | Interferon-induced protein 44 | IFI44 | 2.21E-11 | 9.736 | 0.750 | DM |
| 213797_at1 | radical S-adenosyl methionine domain containing 2 | RSAD2 | 5.63E-10 | 9.423 | 0.750 | DM |
| 202411_at1 | interferon, alpha-inducible protein 27 | IFI27 | 6.86E-06 | 8.796 | 0.682 | DM |
| 204439_at1 | interferon-induced protein 44-like | IFI44L | 3.13E-08 | 5.800 | 0.659 | DM |
| 202869_at1 | 2',5'-oligoadenylate synthetase 1, 40/46kDa | OAS1 | 2.91E-08 | 4.149 | 0.727 | DM |
| 203153_at1 | interferon-induced protein with tetratricopeptide repeats 1 /// interferon-induced protein with tetratricopeptide repeats 1 | IFIT1 | 3.68E-06 | 3.883 | 0.682 | DM |

(continued)

| Probe | Gene | Symbol | p-value | fold change | prevalence | disease |
|---|---|---|---|---|---|---|
| 219863_at1 | hect domain and RLD 5 | HERC5 | 3.03E-08 | 3.753 | 0.614 | DM |
| 205569_at1 | lysosomal-associated membrane protein 3 | LAMP3 | 2.11E-06 | 3.366 | 0.591 | DM |
| 204747_at1 | interferon-induced protein with tetratricopeptide repeats 3 | IFIT3 | 3.66E-09 | 3.263 | 0.682 | DM |
| 218400_at1 | 2'-5'-oligoadenylate synthetase 3, 100kDa | OAS3 | 4.34E-06 | 3.191 | 0.591 | DM |
| 227609_at1 | epithelial stromal interaction 1 (breast) | EPSTI1 | 1.86E-06 | 3.096 | 0.568 | DM |
| 202086_at1 | myxovirus (influenza virus) resistance 1, interferon-inducible protein p78 (mouse) /// myxovirus (influenza virus) resistance 1, interferon-inducible protein p78 (mouse) | MX1 | 1.60E-06 | 3.058 | 0.636 | DM |
| 219211_at1 | ubiquitin specific peptidase 18 /// similar to ubiquitin specific peptidase 18 /// similar to ubiquitin specific peptidase 18 /// similar to ubiquitin specific peptidase 18 | USP18 | 5.25E-05 | 3.044 | 0.568 | DM |
| 241916_at1 | Phospholipid scramblase 1 | PLSCR1 | 3.81E-08 | 2.939 | 0.705 | DM |
| 44673_at1 | sialic acid binding Ig-like lectin 1, sialoadliesin | SIGLEC1 | 2.87E-07 | 2.841 | 0.432 | DM |
| 205483_s_at 1 | ISG15 ubiquitin-like modifier | ISG15 | 6.13E-06 | 2.722 | 0.568 | DM |
| 202145_at1 | Lymphocyte antigen 6 complex, locus E | LY6E | 3.07E-05 | 2.685 | 0.500 | DM |
| 204972-at1 | 2'-5'-oligoadenylate synthetase 2, 69/71kDa | OAS2 | 1.59E-06 | 2.545 | 0.500 | DM |
| 219684_at1 | receptor (chemosensory) transporter protein 4 | RTP4 | 3.84E-05 | 2.528 | 0.568 | DM |

(continued)

| Probe | Gene | Symbol | p-value | fold change | prevalence | disease |
|---|---|---|---|---|---|---|
| 241812_at1 | DNA polymerase-transactivated protein 6 | DNAPTP6 | 0.001397 833 | 1.624 | 0.318 | DM |
| 204415_at2 | interferon, alpha-inducible protein 6 | IFI6 | 0 | 11.500 | 1.000 | PM |
| 213797_at2 | radical S-adenosvl methionine domain containing 2 | RSAD2 | 8.53E-08 | 6.775 | 0.727 | PM |
| 214059_at2 | Interferon-induced protein 44 | IFI44 | 4.66E-07 | 6.283 | 0.727 | PM |
| 202411_at2 | interferon, alpha-inducible protein 27 | IFI27 | 0.000334 1 | 5.652 | 0.667 | PM |
| 204439_at2 | interferon-induced protein 44-like | IFI44L | 2.87E-06 | 4.470 | 0.667 | PM |
| 202869_at2 | 2',5'-oligoadenylate synthetase 1, 40/46kDa | OAS1 | 3.08E-08 | 4.431 | 0.697 | PM |
| 219863_at2 | hect domain and RLD 5 | HERC5 | 1.65E-06 | 3.102 | 0.576 | PM |
| 203153_at2 | interferon-induced protein with tetratricopeptide repeats 1 /// interferon-induced protein with tetratricopeptide repeats 1 | IFIT 1 | 0.000223 959 | 2.922 | 0.576 | PM |
| 227609_at2 | epithelial stromal interaction 1 (breast) | EPSTI1 | 2.60E-06 | 2.846 | 0.606 | PM |
| 205569_at2 | lysosomal-associated membrane protein 3 | LAMP3 | 6.22E-05 | 2.831 | 0.576 | PM |
| 241916_at2 | Phospholipid scramblase 1 | PLSCR1 | 3.24E-07 | 2.753 | 0.697 | PM |
| 204747_at2 | interferon-induced protein with tetratricopeptide repeats 3 | IFIT3 | 2.25E-06 | 2.684 | 0.606 | PM |
| 218400_at2 | 2'-5'-oligoadenylate synthetase 3, 100kDa | OAS3 | 0.000152 771 | 2.644 | 0.424 | PM |
| 44673_at2 | sialic acid binding Ig-like lectin 1, sialoadhesin | SIGLEC1 | 5.34E-05 | 2.620 | 0.424 | PM |
| 202145_at2 | lymphocyte antigen 6 complex, locus E | LY6E | 0.000183 714 | 2.406 | 0.394 | PM |

(continued)

| Probe | Gene | Symbol | p-value | fold change | prevalence | disease |
|---|---|---|---|---|---|---|
| 205483_s_at 2 | ISG15 ubiquitin-like modifier | ISG15 | 9.97E-05 | 2.369 | 0.485 | PM |
| 202086_at2 | myxovirus (influenza virus) resistance 1, interferon-inducible protein p78 (mouse) /// myxovirus (influenza virus)resistance 1, interferon-inducible protein p78 (mouse) | MX1 | 0.000540 479 | 2.342 | 0.515 | PM |
| 219684_at2 | receptor (chemosensory) transporter protein 4 | RTP4 | 8.87E-05 | 2.218 | 0.455 | PM |
| 219211_at2 | ubiquitin specific peptidase 18 /// similar to ubiquitin specific peptidase 18 /// similar to ubiquitin specific peptidase 18 /// similar to ubiquitin specific peptidase 18 | USP18 | 0.003664 023 | 2.195 | 0.364 | PM |
| 204972_at2 | 2'-5'-oligoadenylate synthetase 2. 69/71kDa | OAS2 | 0.000106 864 | 2.082 | 0.364 | PM |
| 241812_at2 | DNA polymerase-transactivated protein 6 | DNAPTP6 | 0.016913 8 | 1.533 | 0.242 | PM |
| 204415_at3 | interferon, alpha-inducible protein 6 | IFI6 | 0 | 6.235 | 0.865 | Rheumatoid Arthritis |
| 214059_at3 | Interferon-induced protein 44 | IFI44 | 1.60E-07 | 3.393 | 0.596 | Rheumatoid Arthritis |
| 202411_at3 | interferon, alpha-inducible protein 27 | IFI27 | 0.000442781 | 2.866 | 0.539 | Rheumatoid Arthritis |
| 213797_at3 | radical S-adenosyl methionine domain containing 2 | RSAD2 | 9.28E-06 | 2.783 | 0.494 | Rheumatoid Arthritis |
| 202869_at3 | 2',5'-oligoadenylate synthetase 1, 40/46kDa | OAS1 | 9.98E-08 | 2.391 | 0.528 | Rheumatoid Arthritis |
| 204439_at3 | interferon-induced protein 44-like | IFI44L | 5.15E-05 | 2.235 | 0.461 | Rheumatoid Arthritis |
| 202145_at3 | lymphocyte antigen 6 complex, locus E | LY6E | 5.88E-12 | 2.139 | 0.438 | Rheumatoid Arthritis |
| 241916_at3 | Phospholipid scramblase 1 | PLSCR1 | 1.66E-06 | 2.041 | 0.438 | Rheumatoid Arthritis |

(continued)

| Probe | Gene | Symbol | p-value | fold change | prevalence | disease |
|---|---|---|---|---|---|---|
| 44673_at3 | sialic acid binding Ig-like lectin 1, sialoadhesin | SIGLEC1 | 6.83E-08 | 1.908 | 0.326 | Rheumatoid Arthritis |
| 227609_at3 | epithelial stromal interaction 1 (breast) | EPSTI1 | 6.66E-06 | 1.825 | 0.360 | Rheumatoid Arthritis |
| 204972_at3 | 2'-5'-oligoadenylate synthetase 2. 69/71kDa | OAS2 | 1.23E-09 | 1.761 | 0.315 | Rheumatoid Arthritis |
| 202086_at3 | myxovirus (influenza virus) resistance 1, interferon-inducible protein p78 (mouse) /// myxovirus (influenza virus) resistance 1, interferon-inducible protein p78 (mouse) | MX1 | 0.000342238 | 1.739 | 0.371 | Rheumatoid Arthritis |
| 218400_at3 | 2'-5'-oligoadenylate synthetase 3, 100kDa | OAS3 | 0.000277383 | 1.739 | 0.371 | Rheumatoid Arthritis |
| 219863_at3 | hect domain and RLD 5 | HERC5 | 0.000175256 | 1.692 | 0.303 | Rheumatoid Arthritis |
| 205569_at3 | lysosomal-associated membrane protein 3 | LAMP3 | 0.0029278 | 1.630 | 0.303 | Rheumatoid Arthritis |
| 219684_at3 | receptor (chemosensory) transporter protein 4 | RTP4 | 0.00252807 | 1.544 | 0.281 | Rheumatoid Arthritis |
| 204747_at3 | interferon-induced protein with tetratricopeptide repeats 3 | IFIT3 | 0.005459384 | 1.445 | 0.348 | Rheumatoid Arthritis |
| 205483_s_at 3 | ISG15 ubiquitin-like modifier | ISG15 | 0.009827494 | 1.393 | 0.292 | Rheumatoid Arthritis |
| 219211_at3 | ubiquitin specific peptidase 18 /// similar to ubiquitin specific peptidase 18 /// similar to ubiquitin specific peptidase 18 /// similar to ubiquitin specific peptidase 18 | USP18 | 0.079726231 | 1.274 | 0.236 | Rheumatoid Arthritis |
| 241812_at3 | DNA polymerase-transactivated protein 6 | DNAPTP6 | 0.121840941 | 1.137 | 0.124 | Rheumatoid Arthritis |

(continued)

| Probe | Gene | Symbol | p-value | fold change | prevalence | disease |
|---|---|---|---|---|---|---|
| 203153_at3 | interferon-induced protein with tetratricopeptide repeats 1 /// interferon-induced protein with tetratricopeptide repeats 1 | IFIT1 | 0.654268059 | 1.093 | 0.270 | Rheumatoid Arthritis |
| 204415_at4 | interferon, alpha-inducible protein 6 | IFI6 | 0 | 15.488 | 1.000 | Scleroderma |
| 213797_at4 | radical S-adenosyl methionine domain containing 2 | RSAD2 | 1.92E-08 | 5.758 | 0.711 | Scleroderma |
| 214059_at4 | Interferon-induced protein 44 | IFI44 | 2.52E-07 | 4.494 | 0.658 | Scleroderma |
| 202411_at4 | interferon, alpha-inducible protein 27 | IFI27 | 0.001868066 | 4.096 | 0.579 | Scleroderma |
| 202869_at4 | 2',5'-oligoadenylate synthetase 1, 40/46kDa | OAS1 | 1.43E-08 | 3.581 | 0.711 | Scleroderma |
| 204439_at4 | interferon-induced protein 44-like | IFI44L | 1.98E-05 | 3.105 | 0.553 | Scleroderma |
| 203153_at4 | interferon-induced protein with tetratricopeptide repeats 1 /// interferon-induced protein with tetratricopeptide repeats 1 | IFIT1 | 2.36E-05 | 2.919 | 0.632 | Scleroderma |
| 219863_at4 | hect domain and RLD 5 | HERC5 | 2.37E-08 | 2.860 | 0.632 | Scleroderma |
| 202086_at4 | myxovirus (influenza virus) resistance 1, interferon-inducible protein p78 (mouse) /// myxovirus (influenza virus) resistance 1, interferon-inducible protein p78 (mouse) | MX1 | 9.86E-06 | 2.308 | 0.474 | Scleroderma |
| 218400_at4 | 2'-5'-oligoadenylate synthetase 3, 100kDa | OAS3 | 3.26E-05 | 2.300 | 0.474 | Scleroderma |
| 204747_at4 | interferon-induced protein with tetratricopeptide repeats 3 | IFIT3 | 8.63E-07 | 2.280 | 0.526 | Scleroderma |

(continued)

| Probe | Gene | Symbol | p-value | fold change | prevalence | disease |
|---|---|---|---|---|---|---|
| 202145_at4 | lymphocyte antigen 6 complex, locus E | LY6E | 2.38E-05 | 2.229 | 0.421 | Scleroderma |
| 205569_at4 | lysosomal-associated membrane protein 3 | LAMP3 | 0.0001192 | 2.206 | 0.500 | Scleroderma |
| 205483_s_at4 | ISG15 ubiquitin-like modifier | ISG15 | 1.45E-05 | 2.177 | 0.447 | Scleroderma |
| 227609_at4 | epithelial stromal interaction 1 (breast) | EPSTI1 | 2.54E-05 | 2.177 | 0.553 | Scleroderma |
| 219684_at4 | receptor (chemosensory) transporter protein 4 | RTP4 | 0.000226923 | 1.943 | 0.395 | Scleroderma |
| 204972_at4 | 2'-5'-oligoadenylate synthetase 2, 69/71kDa | OAS2 | 3.34E-06 | 1.909 | 0.368 | Scleroderma |
| 44673_at4 | sialic acid binding Ig-like lectin 1, sialoadhesin | SIGLEC1 | 0.000242727 | 1.831 | 0.342 | Scleroderma |
| 219211_at4 | ubiquitin specific peptidase 18 /// similar to ubiquitin specific peptidase 18 /// similar to ubiquitin specific peptidase 18 /// similar to ubiquitin specific peptidase 18 | USP18 | 0.002152959 | 1.815 | 0.316 | Scleroderma |
| 241916_at4 | Phospholipid scramblase 1 | PLSCR1 | 0.000483597 | 1.793 | 0.395 | Scleroderma |
| 241812_at4 | DNA polymerase-transactivated protein 6 | DNAPTP6 | 0.303084481 | 0.913 | 0.079 | Scleroderma |
| | | | | | | |
| 202411_at | interferon, alpha-inducible protein 27 | IFI27 | 0.00094654 | 25.635 | 1.000 | Sjogren Syndrome |
| 204439_at | interferon-induced protein 44-like | IFI44L | 0.008982765 | 5.742 | 1.000 | Sjogren Syndrome |
| 219211_at | ubiquitin specific peptidase 18 /// similar to ubiquitin specific peptidase 18 /// similar to ubiquitin specific peptidase 18 /// similar to ubiquitin specific peptidase 18 | USP18 | 0.002566614 | 5.079 | 1.000 | Sjogren Syndrome |
| 213797_at | radical S-adenosyl methionine domain containing ? 2 | RSAD2 | 0.003104168 | 4.705 | 1.000 | Sjogren Syndrome |

(continued)

| Probe | Gene | Symbol | p-value | fold change | prevalence | disease |
|---|---|---|---|---|---|---|
| 203153_at | interferon-induced protein with tetratricopeptide repeats 1 /// interferon-induced protein with tetratricopeptide repeats 1 | IFIT1 | 0.0104006 | 4.142 | 1.000 | Sjogren Syndrome |
| 204747_at | interferon-induced protein with tetratricopeptide repeats 3 | IFIT3 | 0.003638823 | 3.857 | 1.000 | Sjogren Syndrome |
| 44673_at | sialic acid binding Ig-like lectin 1, sialoadhesin | SIGLEC1 | 0.007125541 | 3.755 | 1.000 | Sjogren Syndrome |
| 205483_s_at | ISG15 ubiquitin-like modifier | ISG15 | 0.000690576 | 3.557 | 1.000 | Sjogren Syndrome |
| 219863_at | hect domain and RLD 5 | HERC5 | 0.007639108 | 3.499 | 1.000 | Sjogren Syndrome |
| 227609_at | epithelial stromal interaction 1 (breast) | EPSTI1 | 0.001244694 | 3.060 | 1.000 | Sjogren Syndrome |
| 204415_at | interferon, alpha-inducible protein 6 | IFI6 | 0.000905658 | 2.912 | 1.000 | Sjogren Syndrome |
| 218400_at | 2'-5'-oligoadenylate syntlietase 3, 100kDa | OAS3 | 0.009517756 | 2.764 | 1.000 | Sjogren Syndrome |
| 202086_at | myxovirus (influenza virus) resistance 1, interferon,- inducible protein p78 (mouse) /// myxovirus (influenza virus) resistance 1, interferon-inducible protein p78 (mouse) | MX1 | 0.003908144 | 2.722 | 1.000 | Sjogren Syndrome |
| 204972_at | 2'-5'-oligoadenylate synthetase 2, 69/71kDa | OAS2 | 0.00753208 | 2.590 | 1.000 | Sjogren Syndrome |
| 214059_at | Interferon-induced protein 44 | IFI44 | 0.039282229 | 2.477 | 0.750 | Sjogren Syndrome |
| 202145_at | lymphocyte antigen 6 complex, locus E | LY6E | 0.01319652 | 2.418 | 0.750 | Sjogren Syndrome |
| 202869_at | 2',5'-oligoadenylate synthetase 1, 40/46kDa | OAS1 | 0.022562072 | 1.796 | 0.250 | Sjogren Syndrome |
| 219684_at | receptor (chemosensory) transporter protein 4 | RTP4 | 0.231709806 | 1.749 | 0.250 | Sjogren Syndrome |

(continued)

| Probe | Gene | Symbol | p-value | fold change | prevalence | disease |
|---|---|---|---|---|---|---|
| 241916_at | Phospholipid scramblase 1 | PLSCR1 | 0.472321498 | 1.284 | 0.000 | Sjogren Syndrome |
| 241812_at | DNA polymerase-transactivated protein 6 | DNAPTP6 | 0.529363363 | 0.917 | 0.000 | Sjogren Syndrome |
| 205569_at | lysosomal-associated membrane protein 3 | LAMP3 | 0.318182147 | 0.905 | 0.000 | Sjogren Syndrome |

We found that the proportion of SLE, DM, PM, SSc, and RA subjects that were positive for activation of the type I IFN pathway in WB was 72% for SLE, 66% for DM, 61% for PM, 50% for SSc, and 33% for RA. The concordant overexpression of type I IFN-inducible transcripts was observed in both involved tissue and WB.

From these studies, consistently observed activation of the type I IFN pathway in a population of SLE, DM, PM, SSc, and RA subjects. Subgroups of PM and RA subjects also showed activation of the TNF-$\alpha$ pathway and a subgroup of SSc subjects showed strong activation of the IL-17 pathway. A common set of 36 type I IFN-inducible transcripts were among the most over-expressed transcripts in the WB of subjects. Thus, these studies show that it is possible to classify all subjects with different disease manifestations by molecular features, rather than classic SLE, DM, PM, SSc, and RA nomenclature is presented.

**B.** Results

The scores for the type I IFN-inducible gene signatures as calculated using the five type I IFN-inducible genes in the WB of individual SLE, DM, PM, SSc, and RA subjects and healthy subjects are shown in Figure 11A. Composite scores of the relative expression of multiple genes can provide a robust measurement of pathway activity. Patients with the composite scores of more than 4 were considered type I IFN-inducible gene signature positive. The threshold for type I IFN gene signature positive/negative status using the 5 gene panel was determined using the distribution of signature values from the healthy normal donors. Since the sample size for these 24 normal healthy donors is modest, we built in a conservative estimate for a signature status threshold. The maximum of the type I IFN gene signature values in the normal healthy donors is 3. A cut off of 4 allows for additional variance that is likely more aligned with the general population. The scores for the overexpression of type I IFN-inducible gene signatures in the WB of these subjects were significant when compared to that of healthy subjects (median scores for healthy normal, SLE, DM, PM, RA and SSc subjects are 1.1, 34.5, 12.4, 5.3, 2.3, and 4.3, respectively; p-values are $7.8\times10^{-47}$, $7.5\times10^{-7}$, $6.6\times10^{-4}$, $4.7\times10^{-5}$, and $5.2\times10^{-4}$, for SLE, DM, PM, RA, and SSc subjects, respectively).

For each autoimmune disease, the percentage of subjects that were scored as "signature positive" for activation of type I IFN pathways was using the 5 gene type I IFN signature score (Table 4). Patients that are signature positive for activation of the type 1 IFN pathway within each autoimmune disease are: 73% SLE, 66% DM, 61% PM, and 50% SSc, 33% RA. This method for identifying similar subjects with a positive signature (or negative signature) suggests a robust presence of type I IFN gene overexpression in a specific subgroup of these autoimmune diseases.

**Table 4. Percentages of subjects with a positive type I IFN-inducible gene signature in normal healthy controls and SLE, DM, PM, RA, and SSc diseases in the WB**

| Signature | Normal donors | SLE | DM | PM | RA | SSc |
|---|---|---|---|---|---|---|
| Type I IFN signature | 0.0% | 72.5% | 65.9% | 60.6% | 32.6% | 50.0% |
| Sample size | 24 | 262 | 44 | 33 | 89 | 38 |

We also evaluated the overexpression of type I IFN-inducible genes in the disease tissues (lesional skin of SLE and SSc subjects, muscle specimen of DM and PM subjects, and synovium tissue of RA subjects). We used *in vitro* stimulation of a primary human keratinocyte/fibroblast model and myoblast muscle cell line (SkMC) with IFN-$\alpha$ or

IFN-β to identify potential type I IFN-inducible transcripts in cells characteristic of the skin and muscle. Most of the transcripts induced by type I IFN in the resident skin cells or muscle cell lines were also inducible in the WB (skin data was described in Yao Y, Jallal J, et al. Type I IFN as a potential therapeutic target for psoriasis. PLoS ONE 2008; 3(7): e2737. doi:10.1371/journal.pone.0002737.) In the following sample sizes, 16 SLE, 37 DM, 36 PM, 22 SSc, and 20 RA subjects, a high overexpression of type I IFN-inducible genes was observed at the disease sites in a large subset of SLE, SSc, DM, PM, and RA subjects that were evaluated (p-values <0.01 for SLE, SSc, DM, and PM subjects, respectively; median 5 gene type I IFN signature scores are 0.8, 13.6, 4.5 in skin of normal healthy donors, SLE and SSc subjects; 0.9, 15.3, and 5 in muscle specimen of normal health donors, DM and PM subjects; 1.0 and 7.1 in synovial tissues and normal healthy donor and RA subjects (the RA comparison had too small of a normal control sample size to evaluate); Figure 11 B).

In an analysis between those patients that have positive type I IFN-inducible gene signatures (using the 5 gene signature) in matched WB and disease tissue specimens, 13 of 16 SLE subjects showed comparable type I IFN-inducible gene signature scores in WB and lesional skin (P<0.05 using Fisher's exact test). For the 10 DM and 9 PM subjects with paired WB and muscle specimens, 2 DM muscle specimens, 1 PM muscle and 1 PM WB specimen (from the same patient) were negative for a type I IFN-inducible gene signature. For the 10 SSc subjects with paired WB and skin specimens, 7 subjects demonstrated comparable type I IFN-inducible gene signature scores. These observations show a strong trend of concordant expression of type I IFN-inducible genes in the WB and disease tissues of SLE, DM, PM, and SSc subjects.

In a further analysis, we studied the four gene signature (IFI27, IFI44, IFI44L, and RSAD2) and determined it also shows a strong trend of concordant expression of the four gene type I IFN-inducible genes in the WB and disease tissues of SLE, DM, PM, and SSc subjects. *See* Figures 12A and B.

## Claims

1. A method of identifying a subject suitable for treatment of lupus with an anti-interferon alpha antibody or an anti-interferon alpha receptor antibody that modulates type 1 interferon activity, comprising detecting increased mRNA of a group of genes consisting of IFI27, IFI44, IFI44L, and RSAD2 in a sample of the subject, wherein a mean fold change in the mRNA of the group of genes of at least four fold indicates a subject suitable for treatment of lupus with the antibody, wherein the increase is relative to

   a) that of a sample from a tissue or person not having the disease, or
   b) the expression of one or more control genes.

2. The method of claim 1, wherein the anti-interferon antibody is sifalimumab.

3. The method of claim 1, wherein the anti-interferon antibody is not sifalimumab.

4. The method of claim 1, wherein detecting mRNA of IFI27, IFI44, IFI44L, and RSAD2 comprises

   a) isolating RNA from a sample obtained from the subject;
   b) synthesizing cDNA from the RNA;
   c) hybridizing the cDNA with oligonucleotides that hybridize to nucleic acid sequences of SEQ ID NOs: 25 to 28 and 32; and

d) amplifying the cDNA and detecting the amplified products.

**5.** The method of claim 4, wherein the oligonucleotides are chosen from oligonucleotides having the sequences of SEQ ID NOs: 13 to 24.

**6.** An anti-interferon alpha antibody or an anti-interferon alpha receptor antibody that modulates type 1 interferon activity for use in the treatment of lupus in a suitable subject, wherein the subject suitable for treatment is identified by detecting increased mRNA of a group of genes consisting of IFI27, IFI44, IFI44L, and RSAD2 in a sample of the subject, wherein a mean fold change in the mRNA of the group of genes of at least 4 fold indicates a subject suitable for treatment of lupus, wherein the increase is relative to

a) that of a sample from a tissue or person not having the disease, or
b) the expression of one or more control genes.

**7.** The antibody for use of claim 6, wherein the anti-interferon antibody is sifalimumab.

**8.** The antibody for use of claim 6, wherein the anti-interferon antibody is not sifalimumab.

**9.** The antibody for use of claim 6 wherein detecting the mRNA of IFI27, IFI44, IFI44L, and RSAD2 comprises

a) isolating RNA from a sample obtained from the subject;
b) synthesizing cDNA from the RNA;
c) hybridizing the cDNA with oligonucleotides that hybridize to nucleic acid sequences of SEQ ID NOs: 25 to 28 and 32; and
d) amplifying the cDNA and detecting the amplified products.

**10.** The antibody for use of claim 9, wherein the oligonucleotides are chosen from oligonucleotides having the sequences of SEQ ID NOs: 13 to 24.

**11.** The method of any one of claims 1 to 5 or the antibody for use of any one of claims 6 to 10, wherein the increased mRNA is relative to pooled samples from healthy patients.

**12.** The method of any one of claims 1 b) or 2 to 5 or 11, to the extent claims 2 to 5 and 11 refer back to claim 1 b), or the antibody for use of any one of claims 6b) or 7 to 11, to the extent claims 7 to 11 refer back to claim 6b), wherein the control genes are selected from ACTB, GAPDH, and 18S rRNA.

**13.** The method of any one of claims 1 to 5, 11 or 12 or the antibody for use of any one of claims 6 to 12, wherein the sample is whole blood or serum.

**14.** The method of any one of claims 1 to 5, or 11 to 13 or the antibody for use of any one of claims 6 to 13, wherein the subject is in need of treatment of systemic lupus erythematosus.

## Patentansprüche

**1.** Verfahren zum Identifizieren eines Subjekts, das geeignet ist für die Behandlung von Lupus mit einem anti-Interferon-alpha-Antikörper oder einem anti-Interferon-alpha-Rezeptor-Antikörper, der die Typ 1 Interferonaktivität moduliert, wobei das Verfahren das Nachweisen einer Zunahme von mRNA einer Gruppe von Genen in einer Probe des Subjekts umfasst, wobei die Gruppe von Genen aus IFI27, IFI44, IFI44L und RSAD2 besteht, wobei eine Änderung um einen mittleren Faktor von mindestens vier in der mRNA der Gruppe von Genen auf ein Subjekt hinweist, das für die Behandlung von Lupus mit dem Antikörper geeignet ist, wobei die Zunahme im Vergleich zu

a) der einer Probe von einem Gewebe oder einer Person, die nicht die Krankheit hat, oder
b) der Expression eines oder mehrerer Kontrollgene

ist.

**2.** Verfahren nach Anspruch 1, wobei der anti-Interferon-Antikörper Sifalimumab ist.

**3.** Verfahren nach Anspruch 1, wobei der anti-Interferon-Antikörper nicht Sifalimumab ist.

**4.** Verfahren nach Anspruch 1, wobei das Nachweisen von mRNA von IFI27, IFI44, IFI44L und RSAD2 umfasst:

a) Isolieren von RNA aus einer dem Subjekt entnommenen Probe;
b) Synthetisieren von cDNA ausgehend von der RNA;
c) Hybridisieren der cDNA mit Oligonukleotiden, die mit Nukleinsäuresequenzen der SEQ ID NRn: 25 bis 28 und 32 hybridisieren; und
d) Amplifizieren der cDNA und Nachweisen der amplifizierten Produkte.

**5.** Verfahren nach Anspruch 4, wobei die Oligonukleotide aus Oligonukleotiden ausgewählt sind, die die Sequenzen der SEQ ID NRn: 13 bis 24 haben.

**6.** Anti-Interferon-alpha-Antikörper oder anti-Interferon-alpha-Rezeptor-Antikörper, der Typ 1 Interferonaktivität moduliert, für die Verwendung in der Behandlung von Lupus in einem geeigneten Subjekt, wobei das für die Behandlung geeignete Subjekt durch Nachweisen erhöhter mRNA einer Gruppe von Genen in einer Probe des Subjekts identifiziert wird, wobei die Gruppe von Genen aus IFI27, IFI44, IFI44L und RSAD2 besteht, wobei eine Änderung um einen mittleren Faktor von mindestens vier in der mRNA der Gruppe von Genen auf ein Subjekt hinweist, das für die Behandlung von Lupus geeignet ist, wobei die Zunahme im Vergleich zu

a) der einer Probe von einem Gewebe oder einer Person, die nicht die Krankheit hat, oder
b) der Expression eines oder mehrerer Kontrollgene

ist.

**7.** Antikörper für die Verwendung nach Anspruch 6, wobei der anti-Interferon-Antikörper Sifalimumab ist.

**8.** Antikörper für die Verwendung nach Anspruch 6, wobei der anti-Interferon-Antikörper nicht Sifalimumab ist.

**9.** Antikörper für die Verwendung von Anspruch 6, wobei das Nachweisen von mRNA von IFI27, IFI44, IFI44L und RSAD2 umfasst:

a) Isolieren von RNA aus einer dem Subjekt entnommenen Probe;
b) Synthetisieren von cDNA ausgehend von der RNA;
c) Hybridisieren der cDNA mit Oligonukleotiden, die mit Nukleinsäuresequenzen der SEQ ID NRn: 25 bis 28 und 32 hybridisieren; und
d) Amplifizieren der cDNA und Nachweisen der amplifizierten Produkte.

**10.** Antikörper für die Verwendung nach Anspruch 9, wobei die Oligonukleotide aus Oligonukleotiden ausgewählt sind, die die Sequenzen der SEQ ID NRn: 13 bis 24 haben.

**11.** Verfahren nach einem der Ansprüche 1 bis 5 oder Antikörper für die Verwendung nach einem der Ansprüche 6 bis 10, wobei die erhöhte mRNA im Vergleich zu vereinigten Proben gesunder Patienten ist.

**12.** Verfahren nach einem der Ansprüche 1 b) oder 2 bis 5 oder 11, insofern sich die Ansprüche 2 bis 5 und 11 auf den Anspruch 1b) zurückbeziehen, oder Antikörper für die Verwendung nach einem der Ansprüche 6b) oder 7 bis 11, insofern sich die Ansprüche 7 bis 11 auf den Anspruch 6b) zurückbeziehen, wobei die Kontrollgene ausgewählt sind aus ACTB, GAPDH und 18S rRNA.

**13.** Verfahren nach einem der Ansprüche 1 bis 5, 11 oder 12 oder Antikörper für die Verwendung nach einem der Ansprüche 6 bis 12, wobei die Probe Vollblut oder Serum ist.

**14.** Verfahren nach einem der Ansprüche 1 bis 5 oder 11 bis 13 oder Antikörper für die Verwendung nach einem der Ansprüche 6 bis 13, wobei das Subjekt einer Behandlung von systemischem Lupus erythematosus bedarf.

**Revendications**

1. Méthode d'identification d'un sujet approprié pour le traitement du lupus avec un anticorps anti-interféron alpha ou un anticorps anti-récepteur de l'interféron alpha qui module l'activité des interférons de type 1, comprenant la détection d'une augmentation de l'ARNm d'un groupe de gènes constitué de IFI27, IFI44, IFI44L, et RSAD2 dans un échantillon du sujet, dans laquelle un facteur de variation moyen de l'ARNm du groupe de gènes d'au moins quatre fois indique un sujet approprié pour le traitement du lupus avec l'anticorps, dans laquelle l'augmentation est relative à

    a) celle d'un échantillon provenant d'un tissu ou d'une personne ne souffrant pas de la maladie, ou
    b) l'expression d'un ou de plusieurs gènes témoins.

2. Méthode selon la revendication 1, dans laquelle l'anticorps anti-interféron est le sifalimumab.

3. Méthode selon la revendication 1, dans laquelle l'anticorps anti-interféron n'est pas le sifalimumab.

4. Méthode selon la revendication 1, dans laquelle la détection de l'ARNm de IFI27, IFI44, IFI44L, et RSAD2 comprend

    a) l'isolement de l'ARN à partir d'un échantillon obtenu du sujet ;
    b) la synthèse de l'ADNc à partir de l'ARN ;
    c) l'hybridation de l'ADNc avec des oligonucléotides qui s'hybrident aux séquences d'acide nucléique de SEQ ID NO : 25 à 28 et 32 ; et
    d) l'amplification de l'ADNc et la détection des produits amplifiés.

5. Méthode selon la revendication 4, dans laquelle les oligonucléotides sont choisis parmi des oligonucléotides ayant les séquences de SEQ ID NO : 13 à 24.

6. Anticorps anti-interféron alpha ou anticorps anti-récepteur de l'interféron alpha qui module l'activité des interférons de type 1 pour une utilisation dans le traitement du lupus chez un sujet approprié, le sujet approprié pour le traitement étant identifié par la détection d'une augmentation de l'ARNm d'un groupe de gènes constitué de IFI27, IFI44, IFI44L, et RSAD2 dans un échantillon du sujet, dans laquelle un facteur de variation moyen de l'ARNm du groupe de gènes d'au moins 4 fois indique un sujet approprié pour le traitement du lupus, dans laquelle l'augmentation est relative à

    a) celle d'un échantillon provenant d'un tissu ou d'une personne ne souffrant pas de la maladie, ou
    b) l'expression d'un ou de plusieurs gènes témoins.

7. Anticorps pour une utilisation selon la revendication 6, l'anticorps anti-interféron étant le sifalimumab.

8. Anticorps pour une utilisation selon la revendication 6, l'anticorps anti-interféron n'étant pas le sifalimumab.

9. Anticorps pour une utilisation selon la revendication 6, où la détection de l'ARNm de IFI27, IFI44, IFI44L, et RSAD2 comprend

    a) l'isolement de l'ARN à partir d'un échantillon obtenu du sujet ;
    b) la synthèse de l'ADNc à partir de l'ARN ;
    c) l'hybridation de l'ADNc avec des oligonucléotides qui s'hybrident aux séquences d'acide nucléique de SEQ ID NO : 25 à 28 et 32 ; et
    d) l'amplification de l'ADNc et la détection des produits amplifiés.

10. Anticorps pour une utilisation selon la revendication 9, où les oligonucléotides sont choisis parmi des oligonucléotides ayant les séquences de SEQ ID NO : 13 à 24.

11. Méthode selon l'une quelconque des revendications 1 à 5 ou anticorps pour une utilisation selon l'une quelconque des revendications 6 à 10, où l'augmentation de l'ARNm est relative à des échantillons groupés provenant de patients en bonne santé.

12. Méthode selon l'une quelconque des revendications 1b) ou 2 à 5 ou 11, dans la mesure où les revendications 2 à 5 et 11 se rapportent à la revendication 1b, ou anticorps pour une utilisation selon l'une quelconque des revendications 6b) ou 7 à 11, dans la mesure où les revendications 7 à 11 se rapportent à la revendication 6b), dans laquelle les gènes témoins sont choisis parmi ACTB, GAPDH, et l'ARNr 18S.

**13.** Méthode selon l'une quelconque des revendications 1 à 5, 11 ou 12 ou anticorps pour une utilisation selon l'une quelconque des revendications 6 à 12, où l'échantillon est du sang total ou du sérum.

**14.** Méthode selon l'une quelconque des revendications 1 à 5, ou 11 à 13 ou anticorps pour une utilisation selon l'une quelconque des revendications 6 à 13, où le sujet a besoin d'un traitement du lupus érythémateux systémique.

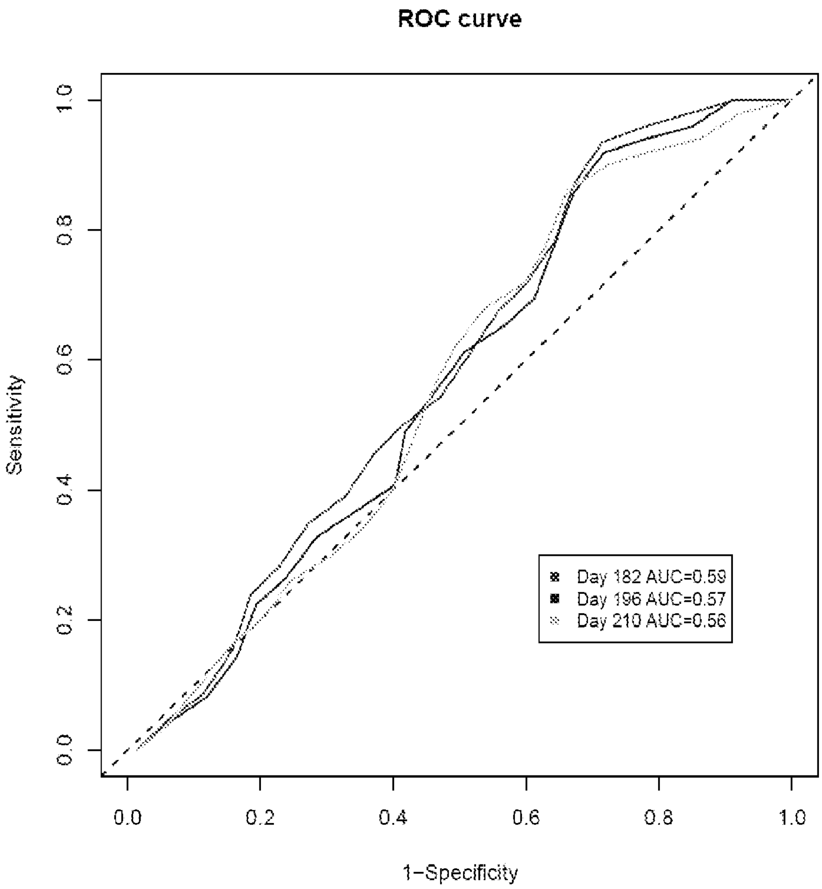


Sensitivity

Day 182=87.0%

Day 196=85.7%

Day 210=86.0%

Specificity

Day 182=32.9%

Day 196=32.8%

Day 210=33.8%

Figure 1

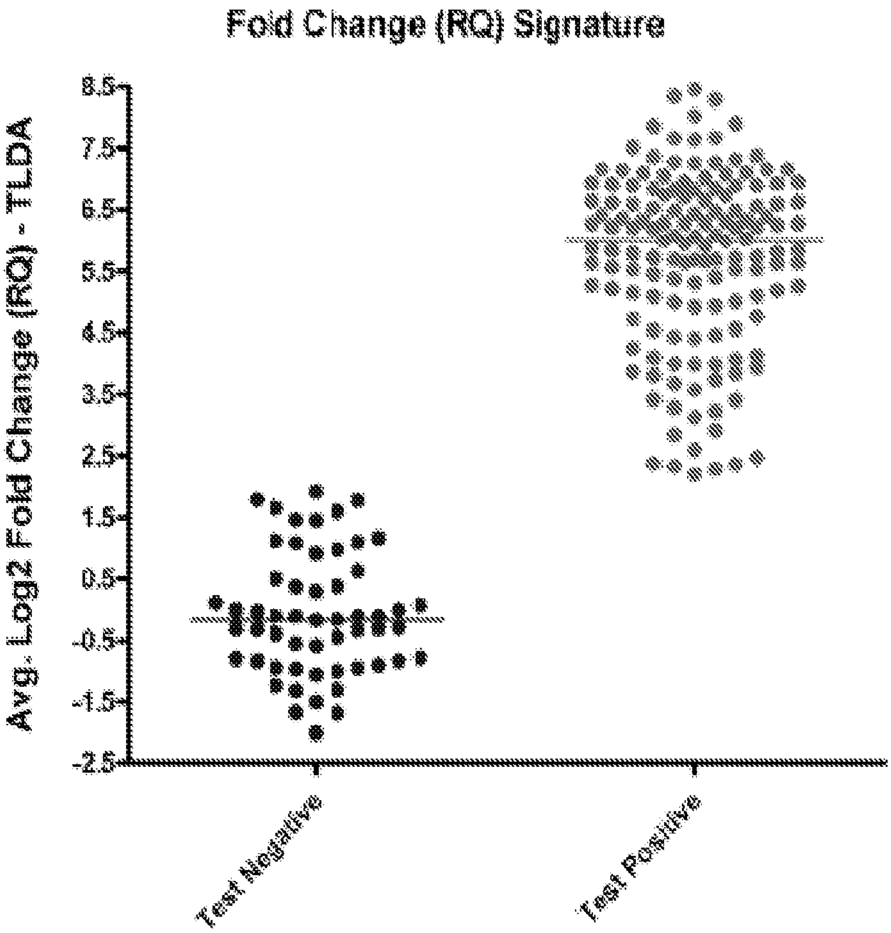
Fold Change (RQ) Signature
Avg. Log2 Fold Change (RQ) - TLDA
8.5
7.5
6.5
5.5
4.5
3.5
2.5
1.5
0.5
-0.5
-1.5
-2.5
Test Negative
Test Positive

Figure 2 A

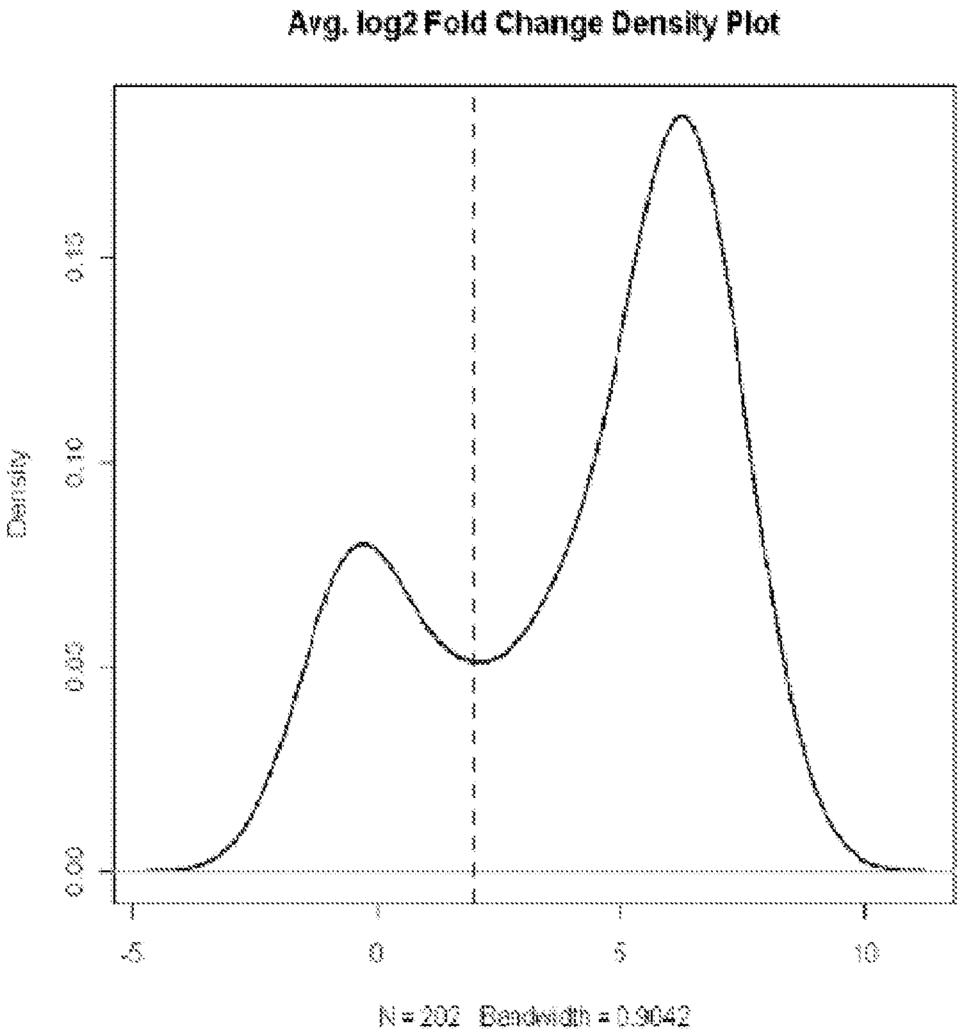
Avg. log2 Fold Change Density Plot
Density
0.00
0.05
0.10
0.15
-5
0
5
10
N = 202   Bandwidth = 0.9042

Figure 2 B

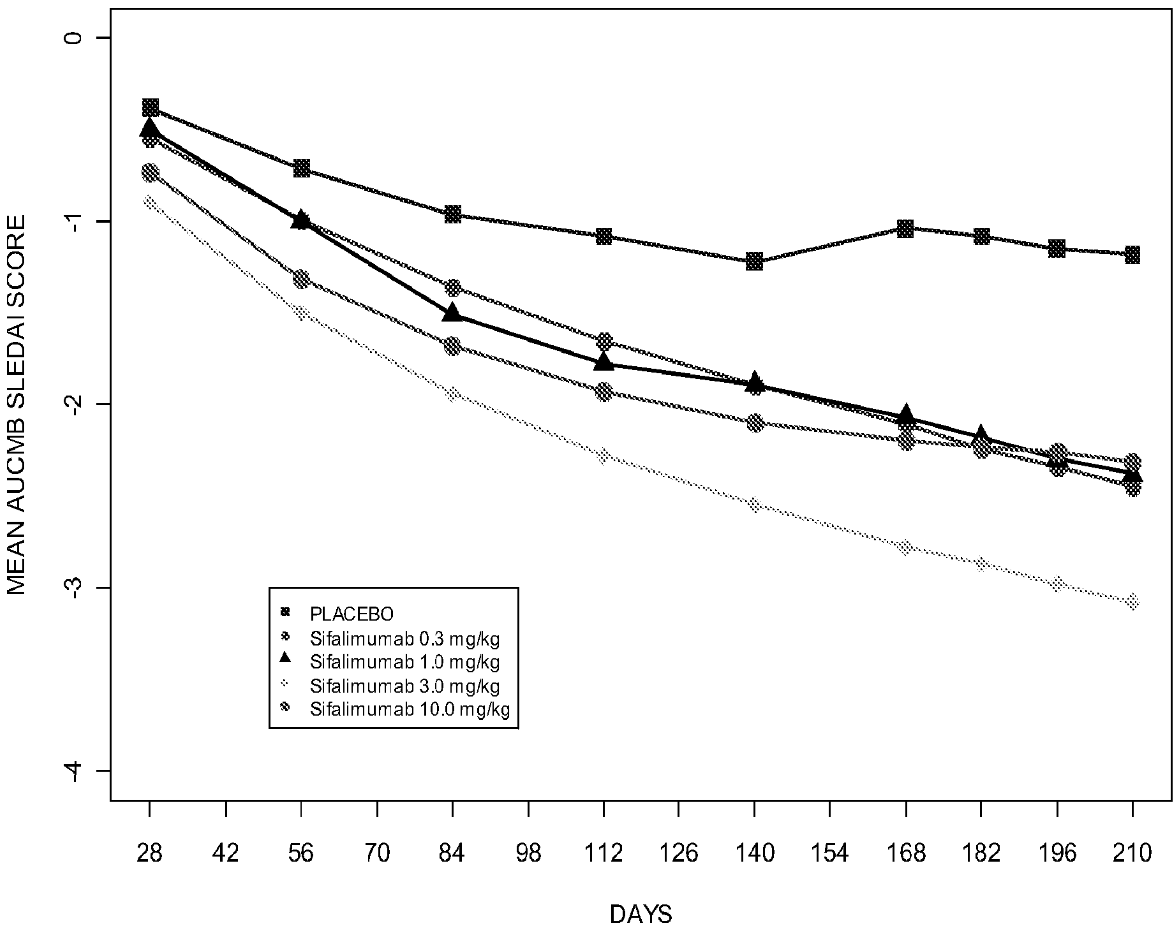
0
-1
-2
-3
-4
MEAN AUCMB SLEDAI SCORE
PLACEBO
Sifalimumab 0.3 mg/kg
Sifalimumab 1.0 mg/kg
Sifalimumab 3.0 mg/kg
Sifalimumab 10.0 mg/kg
28
42
56
70
84
98
112
126
140
154
168
182
196
210
DAYS

Figure 3 A

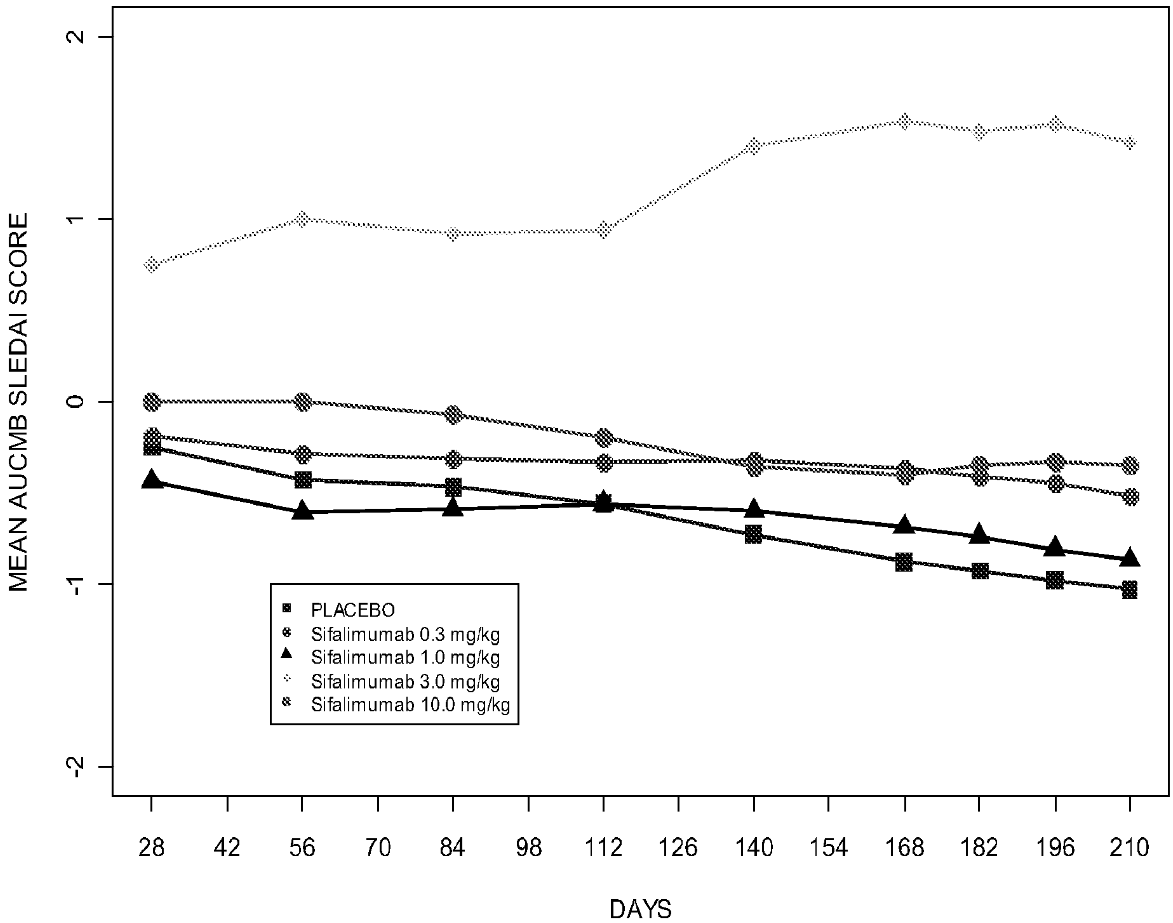
MEAN AUCMB SLEDAI SCORE
2
1
0
-1
-2
28
42
56
70
84
98
112
126
140
154
168
182
196
210
DAYS
PLACEBO
Sifalimumab 0.3 mg/kg
Sifalimumab 1.0 mg/kg
Sifalimumab 3.0 mg/kg
Sifalimumab 10.0 mg/kg

Figure 3 B

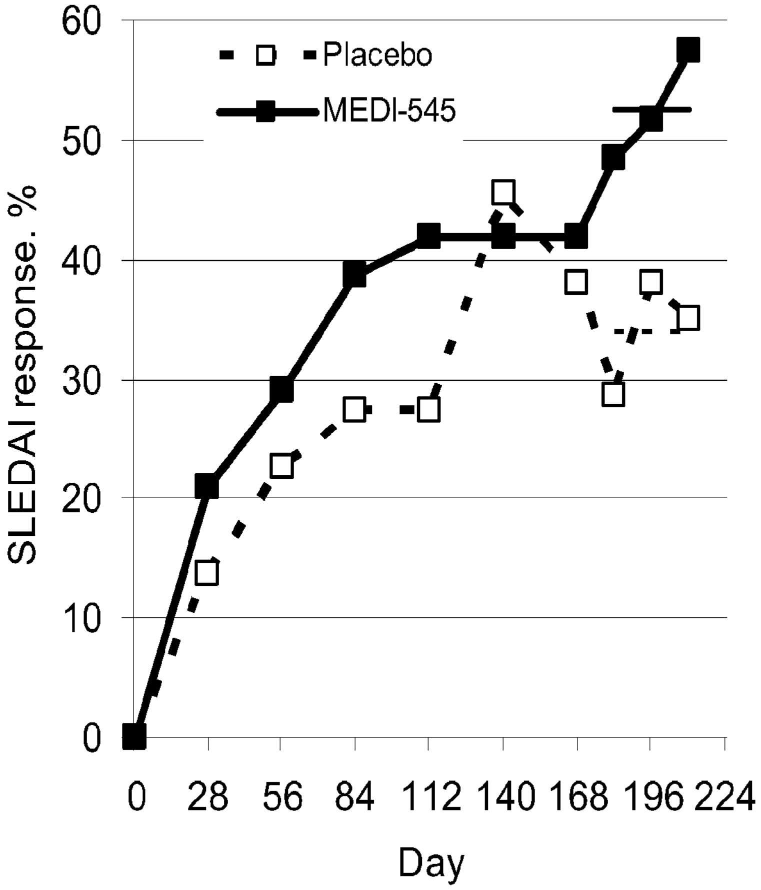
60
50
40
30
20
10
0
SLEDAI response. %
Placebo
MEDI-545
0
28
56
84
112
140
168
196
224
Day

Figure 4 A

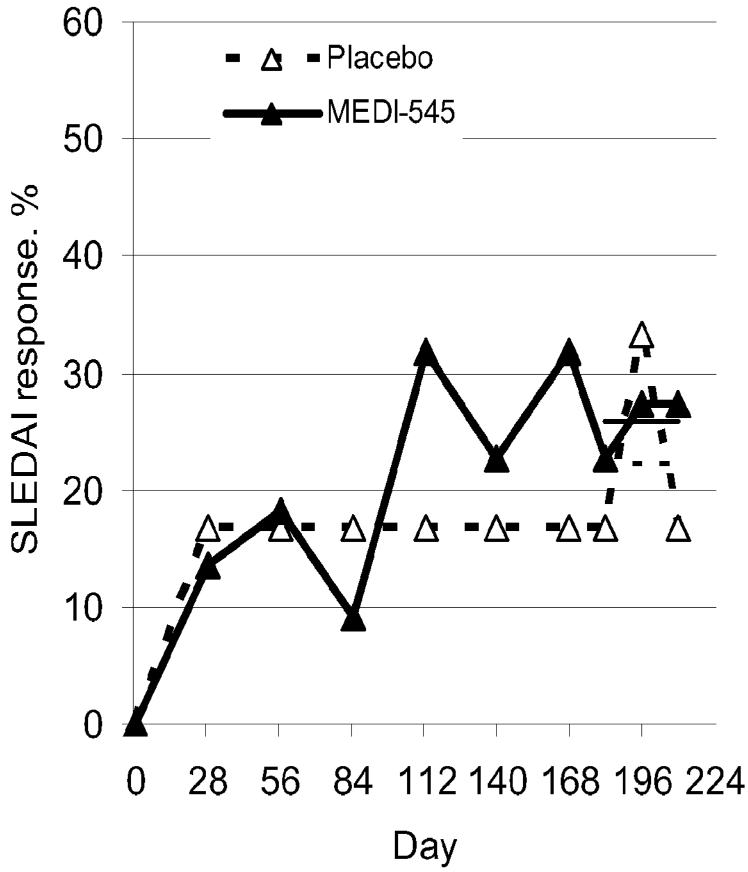
60
50
40
30
20
10
0
Placebo
MEDI-545
SLEDAI response. %
0
28
56
84
112
140
168
196
224
Day

Figure 4 B

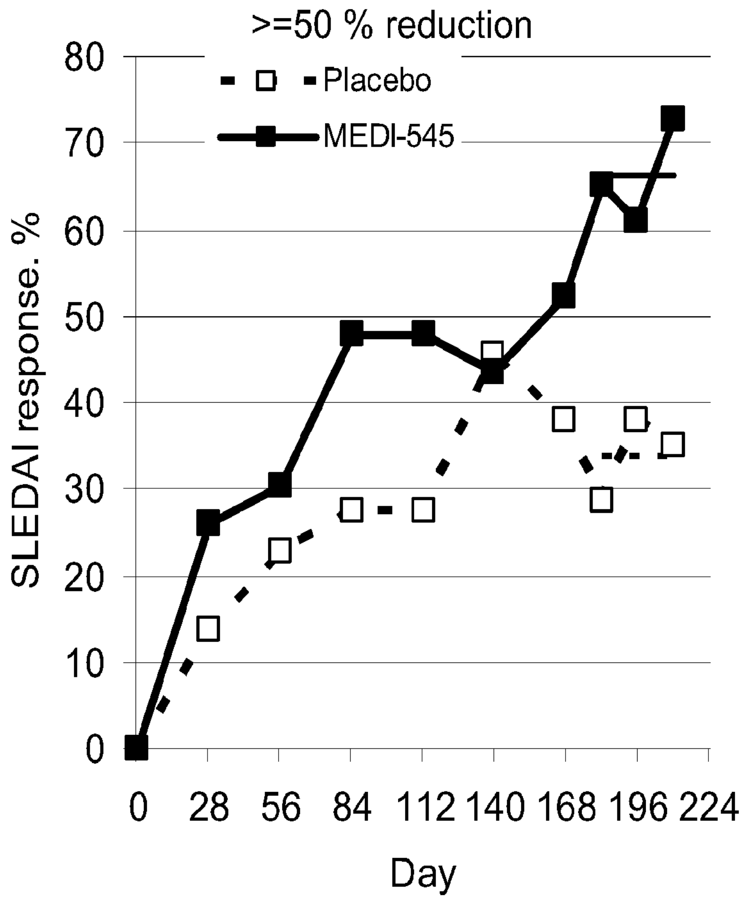

>=50 % reduction
Placebo
MEDI-545
SLEDAI response. %
80
70
60
50
40
30
20
10
0
0
28
56
84
112
140
168
196
224
Day


Figure 5 A

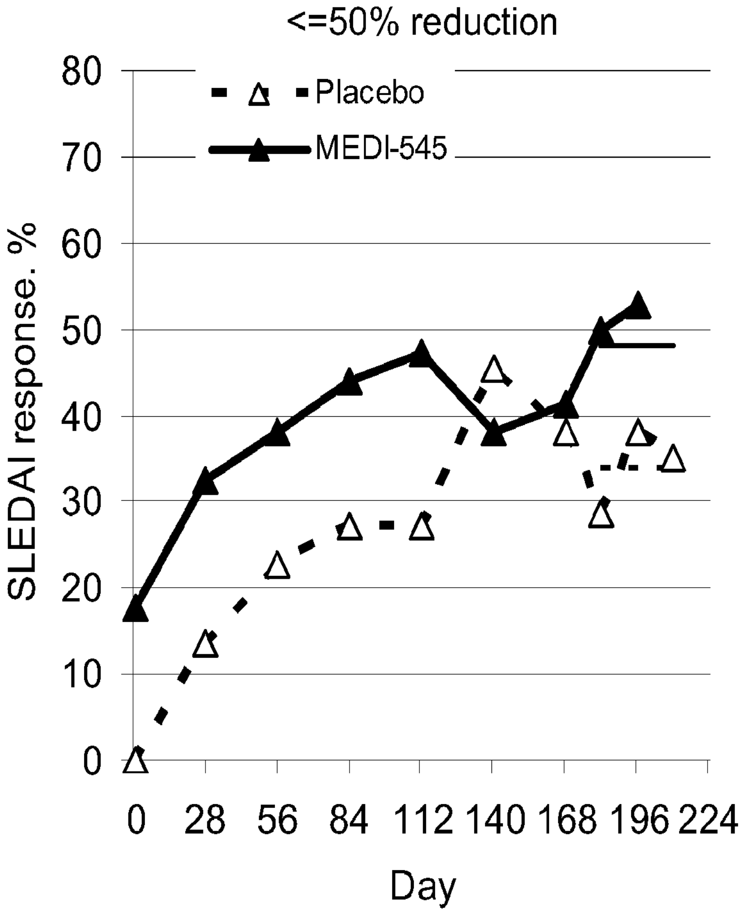
<=50% reduction
Placebo
MEDI-545
SLEDAI response. %
0
10
20
30
40
50
60
70
80
0
28
56
84
112
140
168
196
224
Day

Figure 5 B

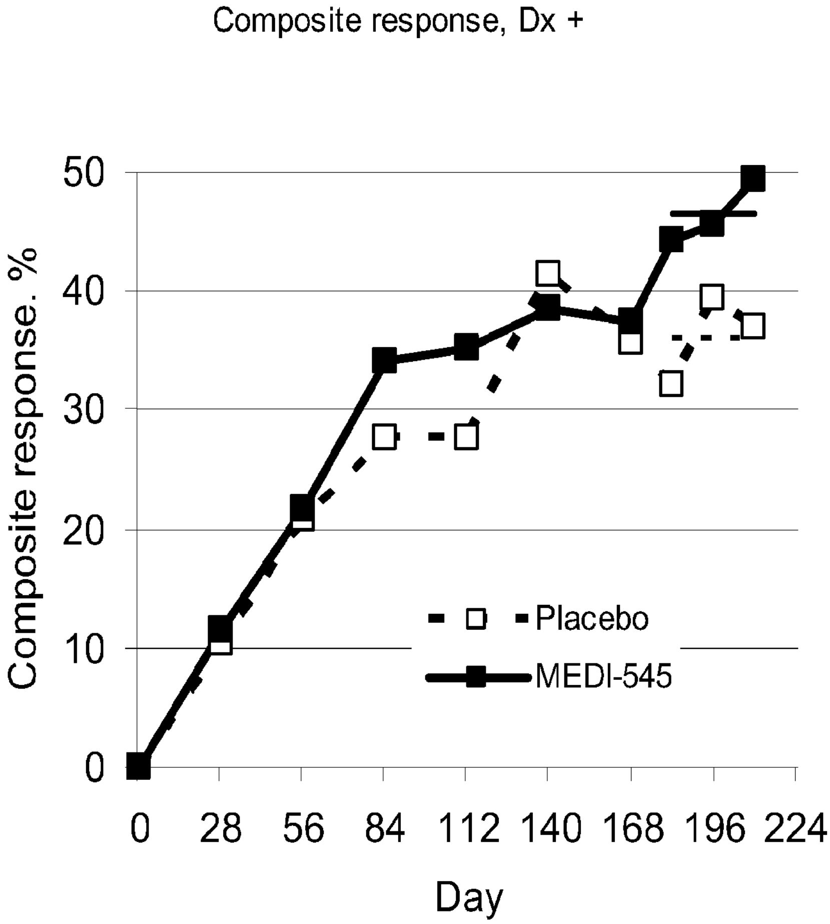
Composite response, Dx +
Composite response. %
50
40
30
20
10
0
0
28
56
84
112
140
168
196
224
Day
Placebo
MEDI-545

Figure 6 A

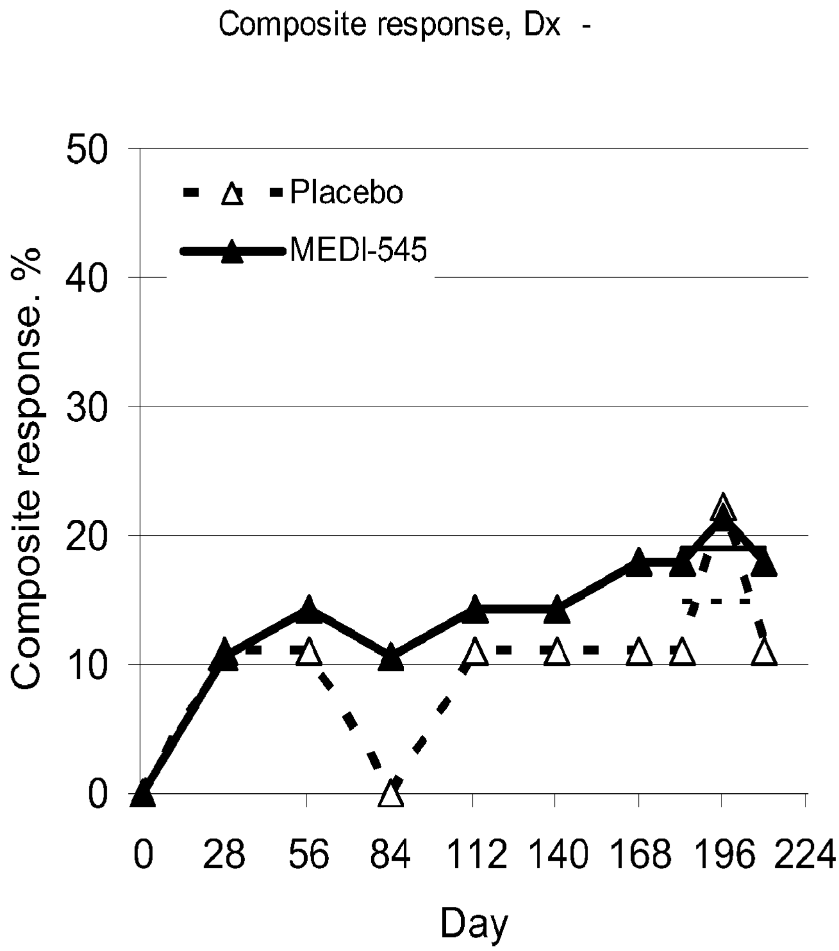
Composite response, Dx -
Placebo
MEDI-545
Composite response. %
50
40
30
20
10
0
0
28
56
84
112
140
168
196
224
Day

Figure 6 B

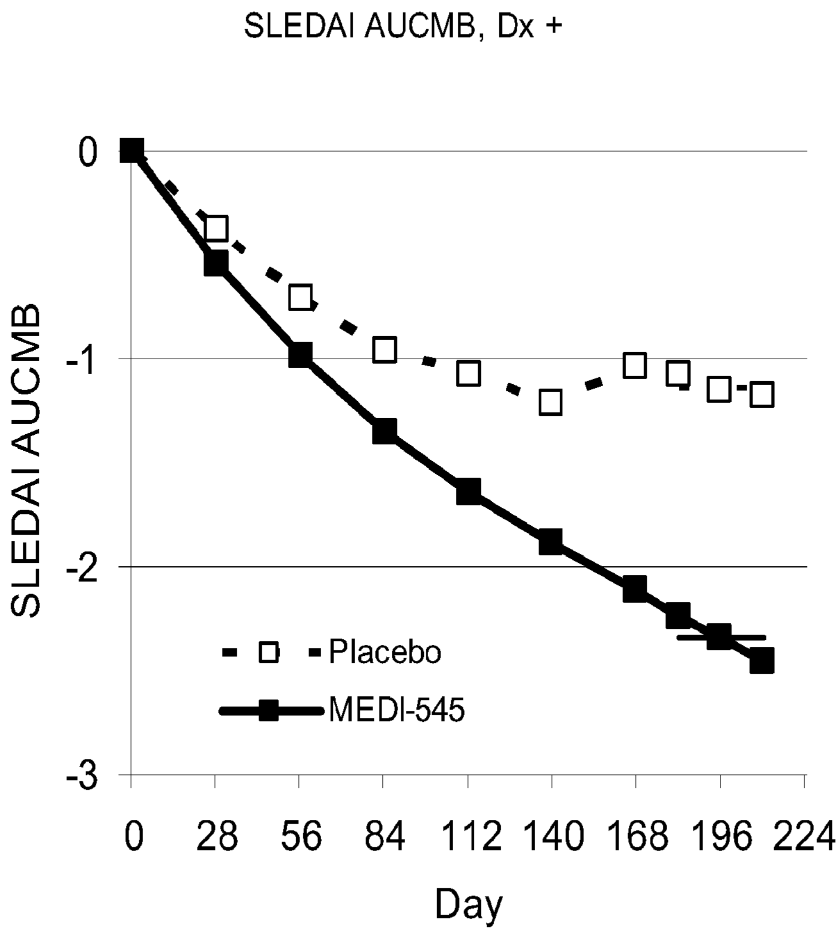
SLEDAI AUCMB, Dx +
SLEDAI AUCMB
0
-1
-2
-3
Placebo
MEDI-545
0
28
56
84
112
140
168
196
224
Day

Figure 7 A

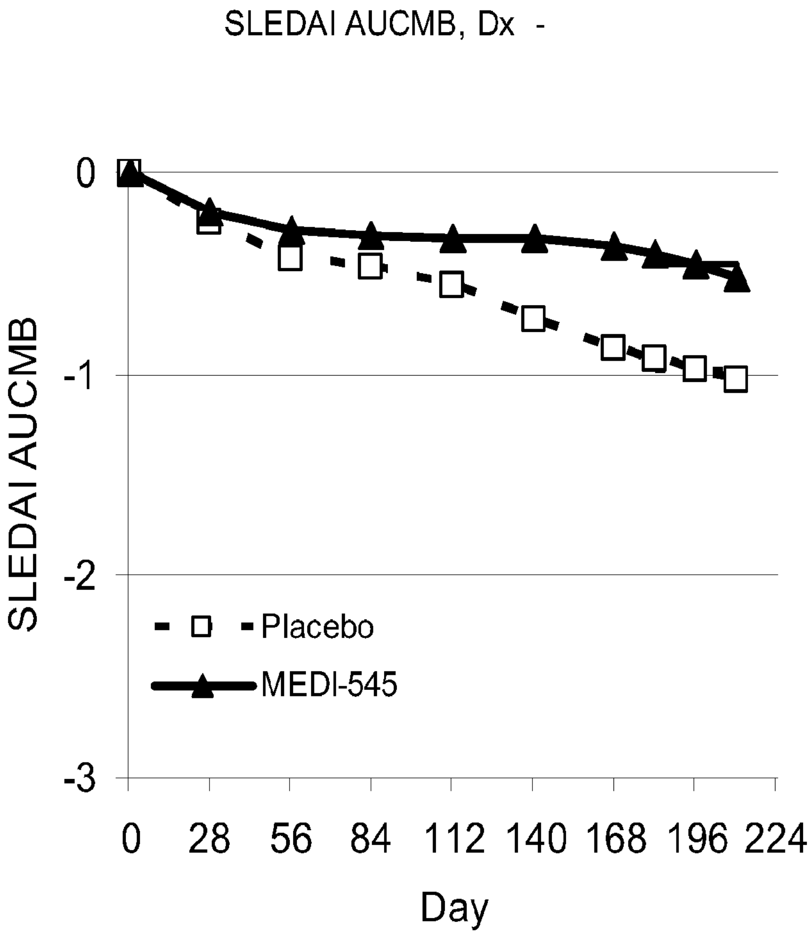
SLEDAI AUCMB, Dx -
0
-1
-2
-3
SLEDAI AUCMB
Placebo
MEDI-545
0 28 56 84 112 140 168 196 224
Day

Figure 7 B

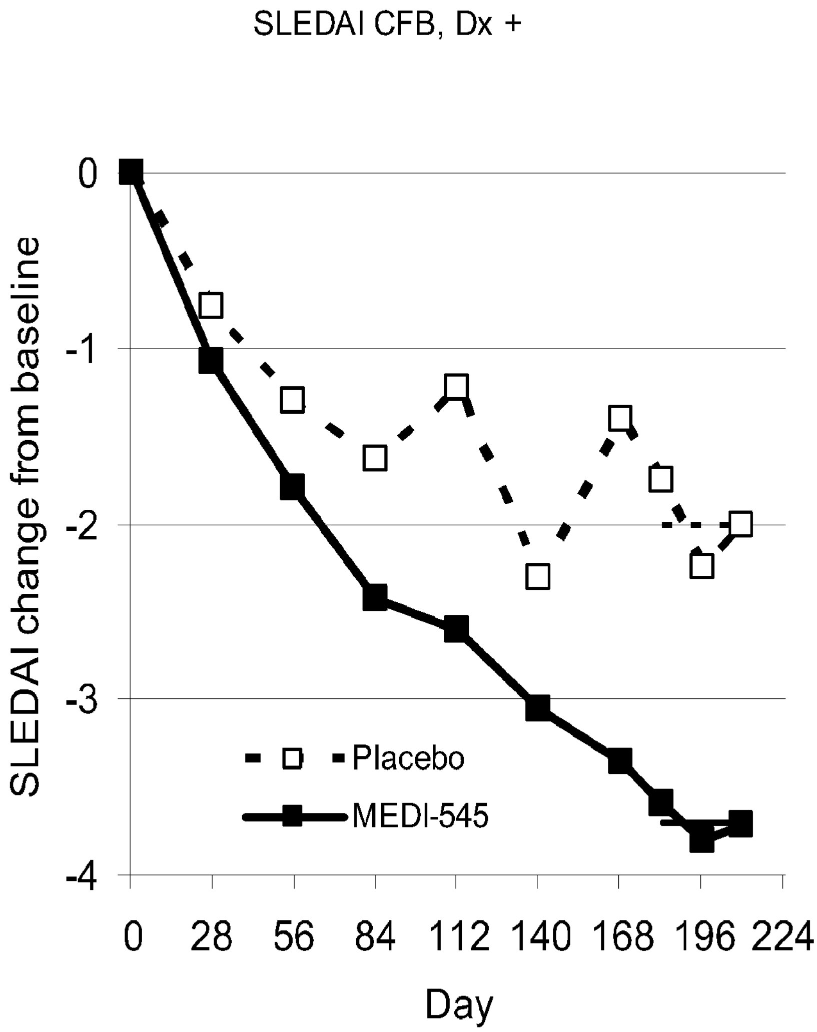
SLEDAI CFB, Dx +
SLEDAI change from baseline
0
-1
-2
-3
-4
Placebo
MEDI-545
0
28
56
84
112
140
168
196
224
Day

Figure 8 A

SLEDAI CFB, Dx -

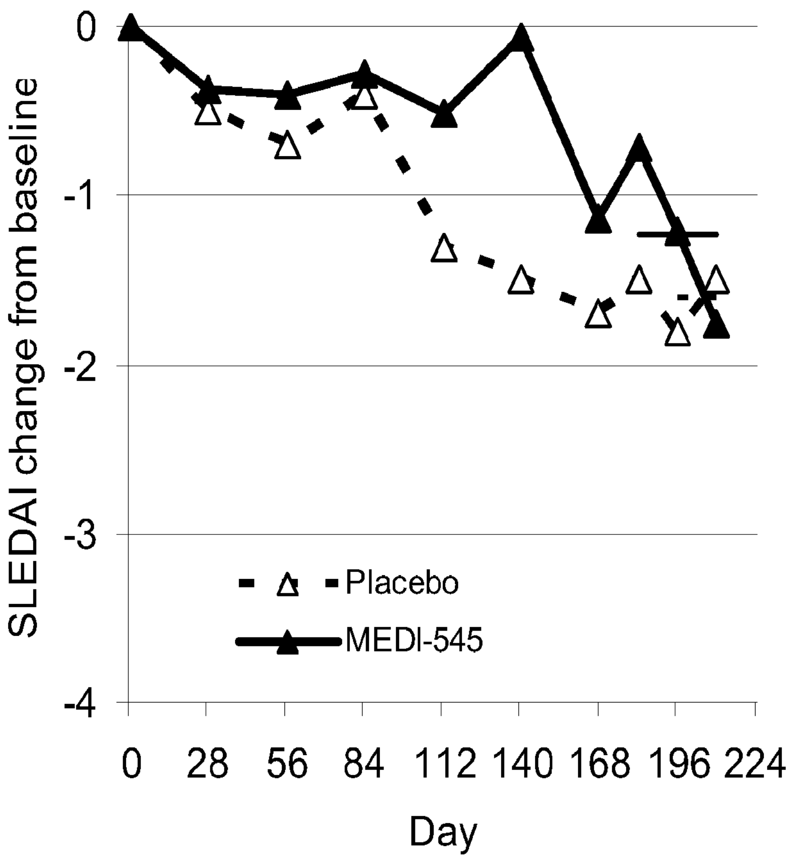


Figure 8 B

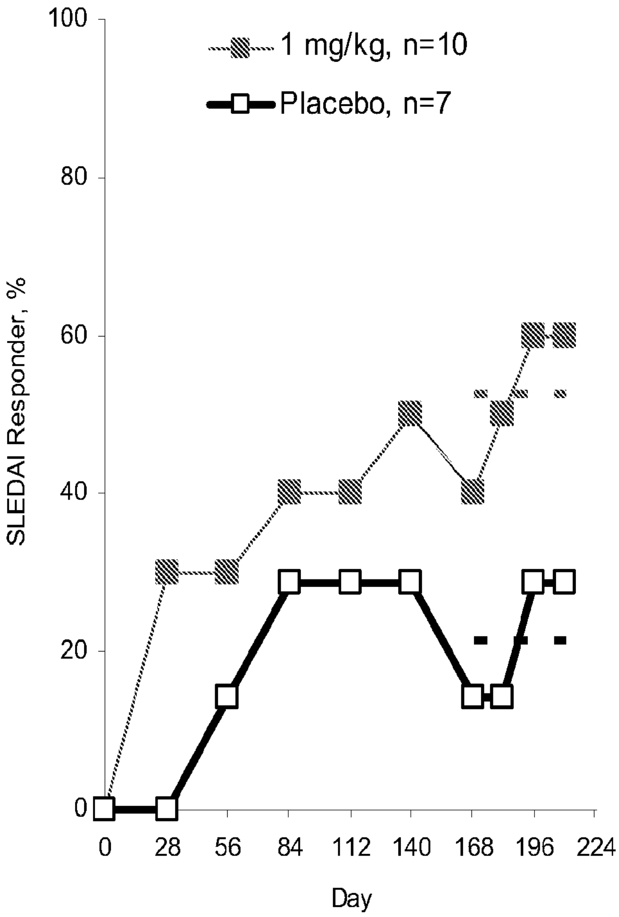
100
80
60
40
20
0
1 mg/kg, n=10
Placebo, n=7
SLEDAI Responder, %
0
28
56
84
112
140
168
196
224
Day

Figure 9 A

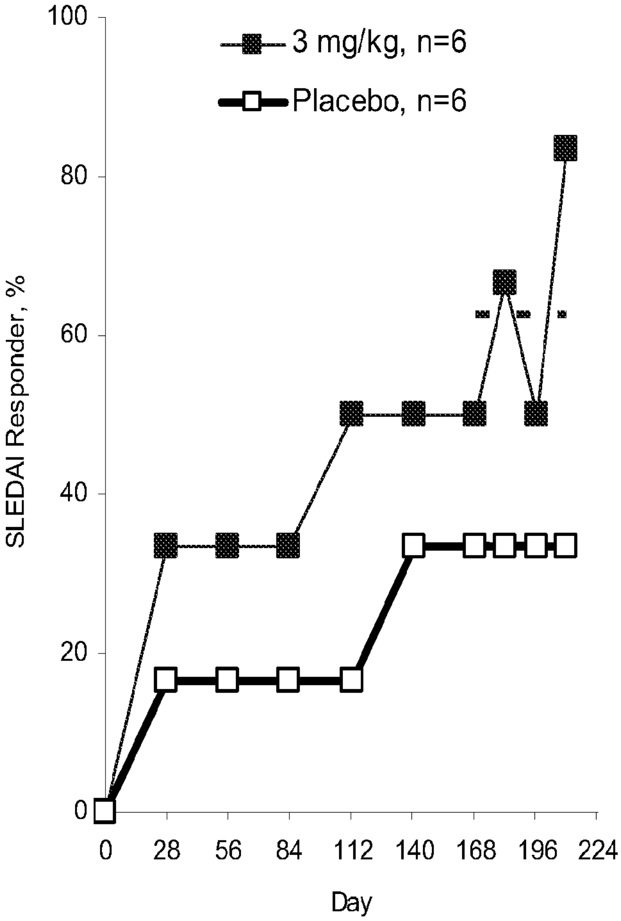
100
80
60
40
20
0
SLEDAI Responder, %
3 mg/kg, n=6
Placebo, n=6
0
28
56
84
112
140
168
196
224
Day

Figure 9 B

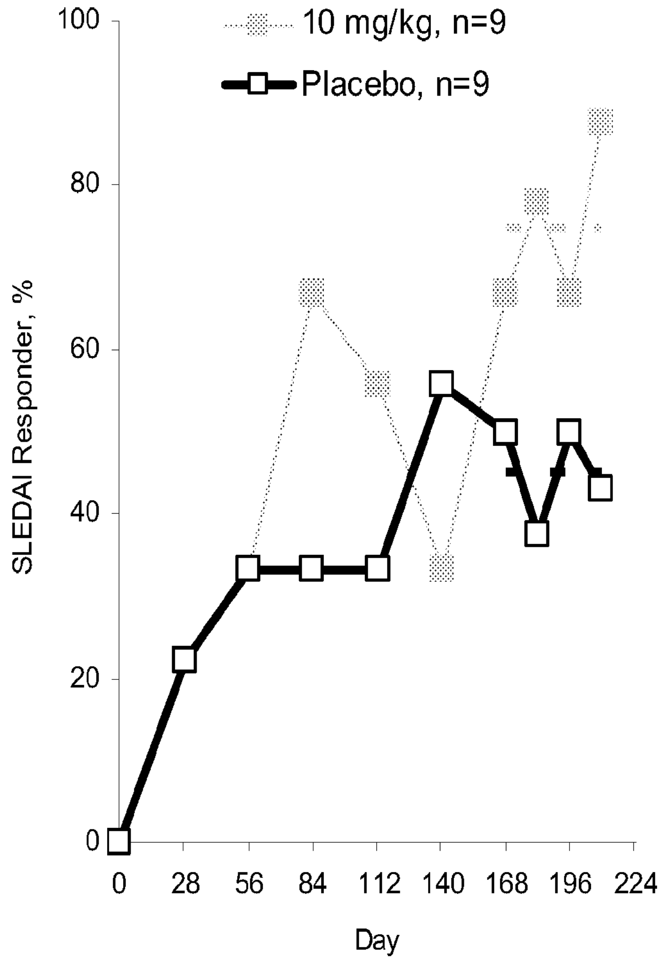
10 mg/kg, n=9
Placebo, n=9
SLEDAI Responder, %
100
80
60
40
20
0
0
28
56
84
112
140
168
196
224
Day

Figure 9 C

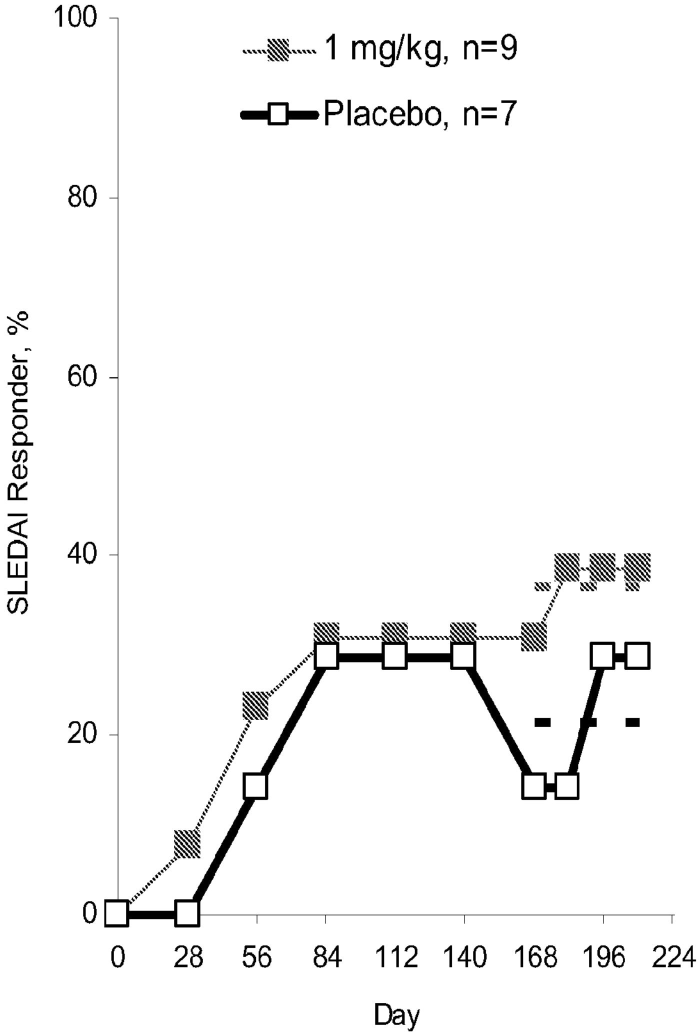
100
80
60
40
20
0
SLEDAI Responder, %
1 mg/kg, n=9
Placebo, n=7
0
28
56
84
112
140
168
196
224
Day

Figure 10 A

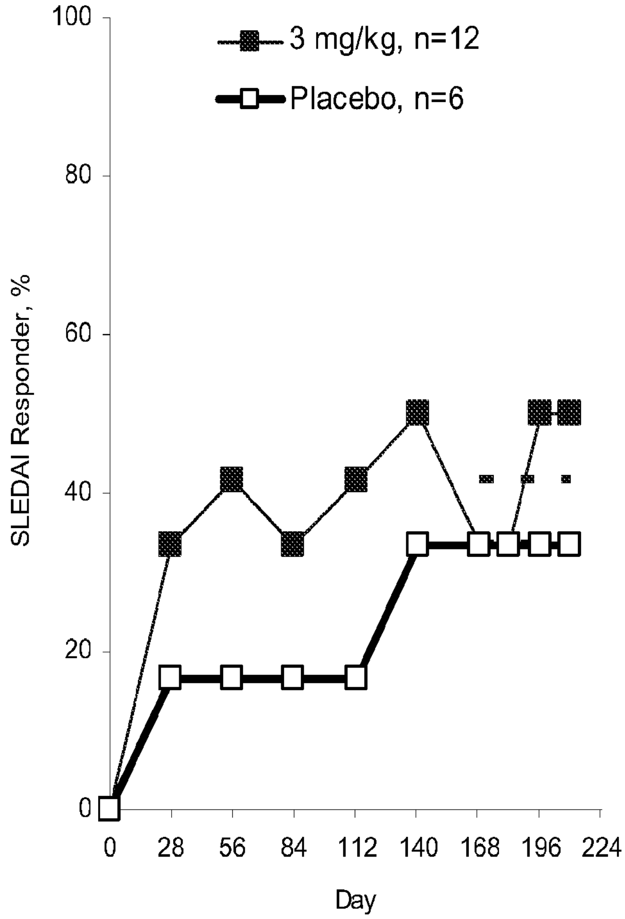
100
80
60
40
20
0
SLEDAI Responder, %
3 mg/kg, n=12
Placebo, n=6
0 28 56 84 112 140 168 196 224
Day

Figure 10 B

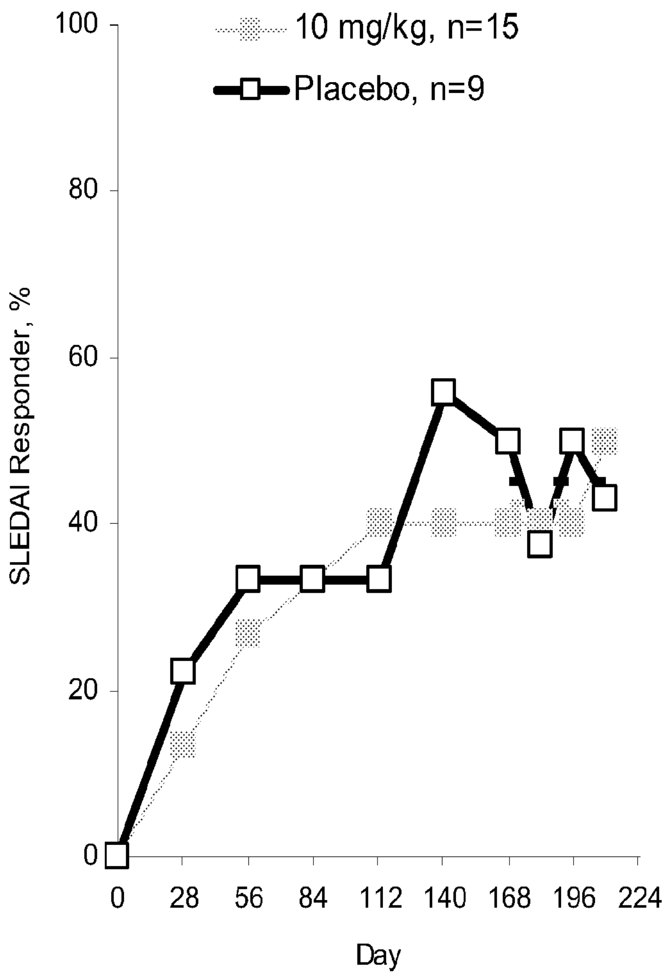
10 mg/kg, n=15
Placebo, n=9
100
80
60
40
20
0
SLEDAI Responder, %
0
28
56
84
112
140
168
196
224
Day

Figure 10 C

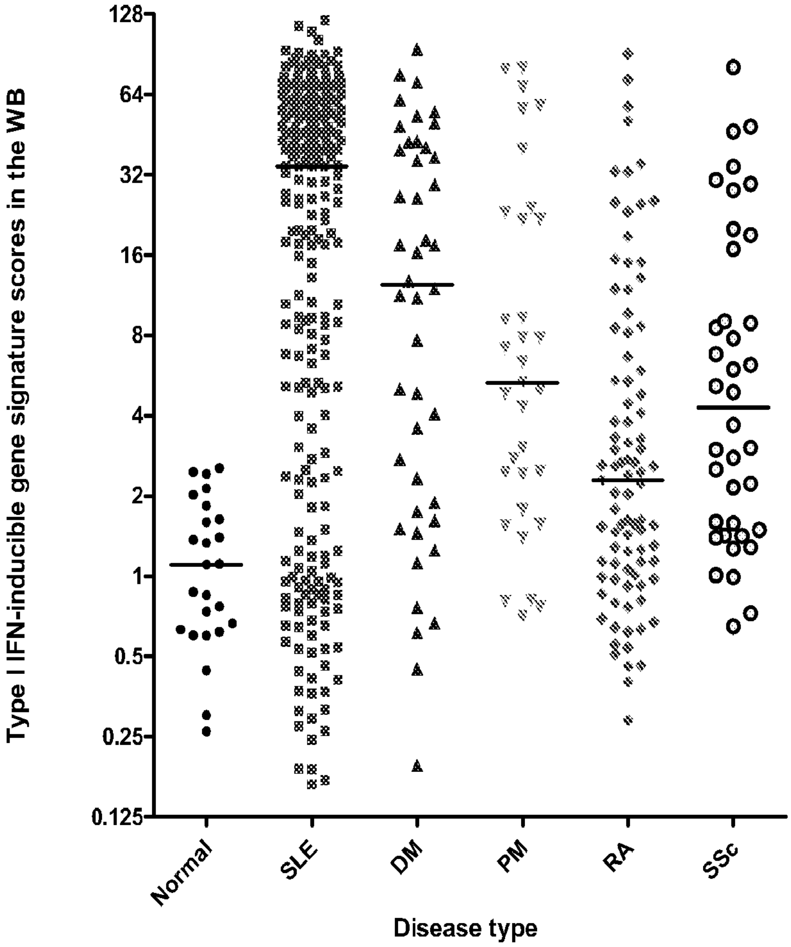
Type I IFN-inducible gene signature scores in the WB
128
64
32
16
8
4
2
1
0.5
0.25
0.125
Normal
SLE
DM
PM
RA
SSc
Disease type

Figure 11 A

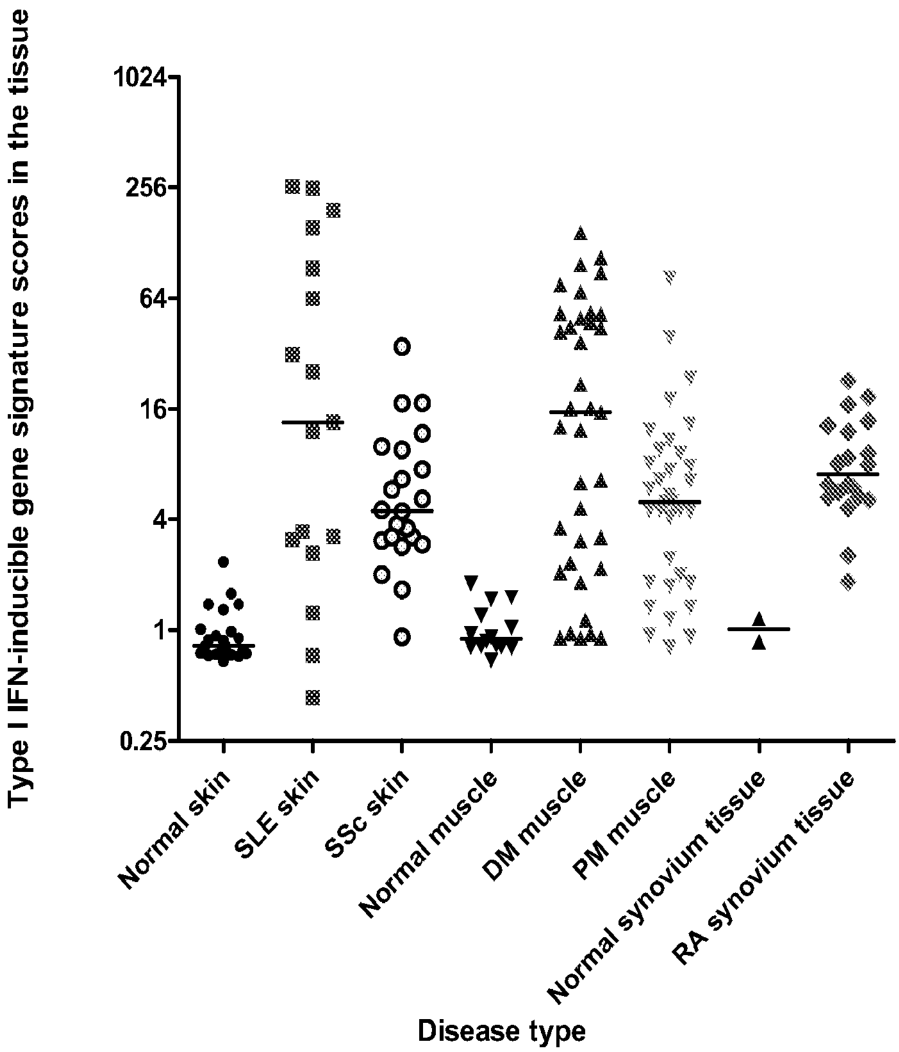
Type I IFN-inducible gene signature scores in the tissue
1024
256
64
16
4
1
0.25
Normal skin
SLE skin
SSc skin
Normal muscle
DM muscle
PM muscle
Normal synovium tissue
RA synovium tissue
Disease type

Figure 11 B

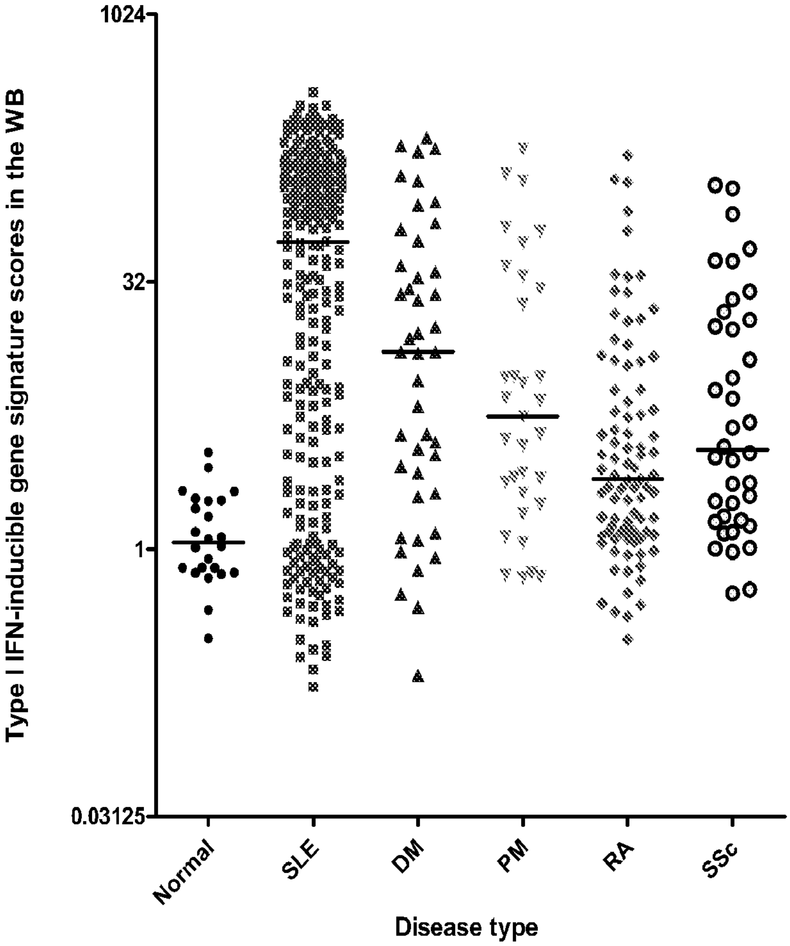
1024
32
1
0.03125
Type I IFN-inducible gene signature scores in the WB
Normal
SLE
DM
PM
RA
SSc
Disease type

Figure 12 A

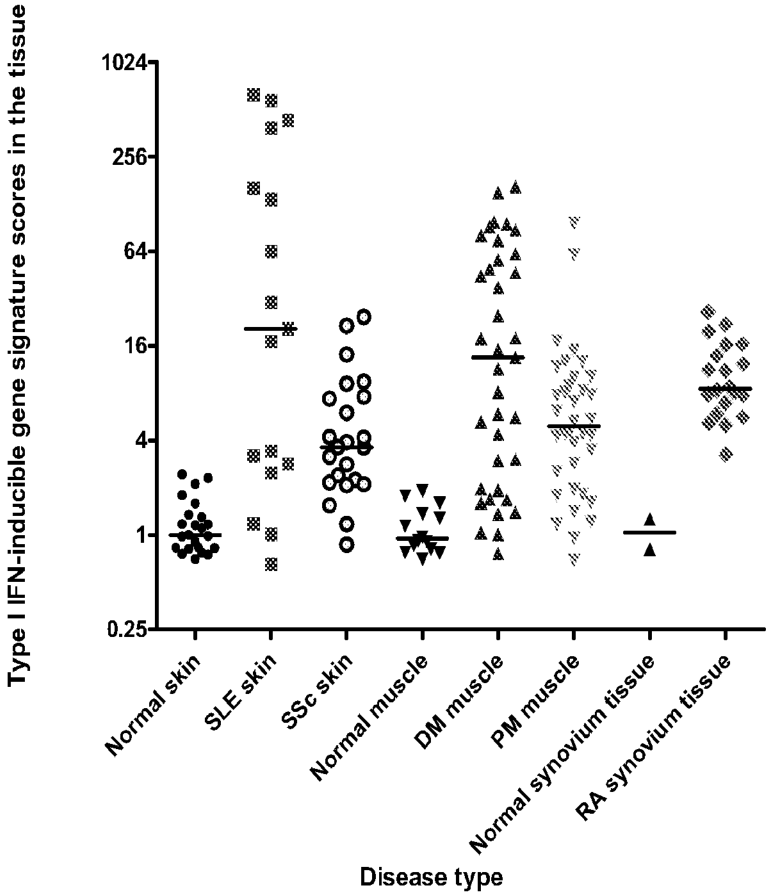

Type I IFN-inducible gene signature scores in the tissue
1024
256
64
16
4
1
0.25
Normal skin
SLE skin
SSc skin
Normal muscle
DM muscle
PM muscle
Normal synovium tissue
RA synovium tissue
Disease type


Figure 12 B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008070137 A
- US 2007024947 W
- US 5744305 A
- US 5143854 A
- US 2007024941 W
- US 00941004 A
- US 11157494 B
- US 7087726 B
- WO 8403105 A
- US 4902618 A
- US 4885166 A
- EP 119476 A
- US 20070014724 A
- US 2008058133 W
- US 2008058132 W
- US 7619070 B
- US 7662381 B
- US 2009033358 W
- US 5972901 A
- US 6008336 A
- US 6077835 A
- US 5718905 A
- US 6207195 B
- US 2008062646 W
- US 2009033407 W
- US 2009048028 W
- WO 61239630 A


### Non-patent literature cited in the description


- **HROVAT, A ; ZAVEC, AB ; POGACNIK, A ; FRANGEZ, R ; VRECL, M.** *Cellular & Molecular Biology Letters,* 2010, vol. 1, 55-69
- **SVEC, J ; ERGANG, P ; MANDYS, V ; KMENT, M ; PACHA, J.** *International Journal of Experimental Pathology,* 2010, vol. 1, 44-53
- **KUROKAWA, T ; HE, GA ; SIDDIK, ZH.** *Cancer Chemotherapy and Pharmacology,* 2010, vol. 3, 427-436
- **MANIATIS et al.** Molecular Cloning. 1989
- **BOMBARDIER C ; GLADMAN D D ; UROWITZ M B ; CARON D ; CHANG C H.** SLE: Derivation of the SLEDAI for Lupus Patients. *Arthritis Rheum,* 1992, vol. 35, 630-640
- **GRIFFITHS et al.** *Assessment of Patients with Systemic Lupus Erythematosus and the use of Lupus Disease Activity Indices*
- **LOEFFLER ; BEHR.** *Meth. Enzymol.,* 1993, vol. 217, 599-618
- **COHEN et al.** *Meth. Enzymol.,* 1993, vol. 217, 618-644
- *Clin. Pharma. Ther.,* 1985, vol. 29, 69-92
- **SAMBROOK et al.** Molecular Cloning: A Laboratory Manual. Cold Spring Harbor Laboratory Press, 1989
- Current Protocols in Molecular Biology. John Wiley & Sons, Inc, 1987
- **ALCANTARA et al.** *Nuc. Acid. Res.,* 2002, vol. 30, 2068-2075
- **KIRCHHOFF et al.** *Oncogene,* 1999, vol. 18, 3725-3736
- **YAO Y ; JALLAL J et al.** Type I IFN as a potential therapeutic target for psoriasis. *PLoS ONE,* 2008, vol. 3 (7), e2737